# EUROPEAN PATENT SPECIFICATION

(11) **EP 2 212 696 B1**
(45) Date of publication and mention of the grant of the patent: **04.12.2013**
(21) Application number: 08841442.0
(22) Date of filing: 24.10.2008
(51) Int. Cl.: G01N 33/50, A61K 48/00, C12N 7/00, C12Q 1/70

(54) **SYSTEMS AND METHODS FOR VIRAL THERAPY**
SYSTEME UND VERFAHREN FÜR DIE VIRUSTHERAPIE
SYSTÈMES ET PROCÉDÉS POUR UNE VIROTHÉRAPIE

(30) Priority: 25.10.2007 US 602 P; 14.11.2007 US 3275 P; 29.05.2008 US 57191 P
(43) Date of publication of application: 04.08.2010
(73) Proprietor: Genelux Corporation, San Diego, California 92109 (US)
(72) Inventor: YU, Yong, A., San Diego, CA 92130 (US); CHEN, Nanhai, San Diego, CA 92129 (US); ZHANG, Qian, San Diego, CA 92130 (US); FRENTZEN, Alexa, San Diego, CA 92109 (US); SZALAY, Aladar A., Highland, CA 92346 (US)
(74) Representative: Baldock, Sharon Claire
(86) International application number: PCT/US2008/012061
(87) International publication number: WO 2009/054996

(56) References cited:
- HIRAOKA KEI ET AL: "Therapeutic efficacy of replication-competent retrovirus vector-mediated suicide gene therapy in a multifocal colorectal cancer metastasis model" CANCER RESEARCH, vol. 67, no. 11, 1 June 2007 (2007-06-01), pages 5345-5353, XP008101254 ISSN: 0008-5472
- ZHANG Q ET AL: "Eradication of solid human breast tumors in nude mice with an intravenously injected light-emitting oncolytic vaccinia virus" CANCER RESEARCH,, vol. 67, no. 20, 1 October 2007 (2007-10-01), pages 10038-10046, XP002505303
- ZHAN JINGHUI ET AL: "Tumor-specific intravenous gene delivery using oncolytic adenoviruses" CANCER GENE THERAPY, NORWALK, CT, US, vol. 12, no. 1, 1 January 2005 (2005-01-01), pages 19-25, XP002461312 ISSN: 0929-1903
- LIN ET AL: "Treatment of anaplastic thyroid carcinoma in vitro with a mutant vaccinia virus" SURGERY, C.V. MOSBY CO., ST. LOUIS, US, vol. 142, no. 6, 5 November 2007 (2007-11-05), pages 976-983, XP022421340 ISSN: 0039-6060
- KELLY KAITLYN J ET AL: "Novel oncolytic agent GLV-1h68 is effective against malignant pleural mesothelioma" HUMAN GENE THERAPY, MARY ANN LIEBERT, NEW YORK ,NY, US, vol. 19, no. 8, 1 August 2008 (2008-08-01), pages 774-782, XP008101255 ISSN: 1043-0342

## Description

### FIELD OF INVENTION

Diagnostic methods for assaying the efficacy of therapeutic viruses *in vitro* for the treatment of cancer and methods for identifying therapeutic viruses are provided. Combinations and kits for use in the practicing the methods are also described.

### BACKGROUND

Current standard cancer therapies include surgery, chemotherapy, radiation, and autologous cell transplantation. Surgery is generally effective in the early treatment of cancer; however, metastatic growth of tumors can prevent any complete cure. Chemotherapy, which involves administration of compounds having antitumor activity, while effective in the treatment of some cancers, is often accompanied by severe side effects, including nausea and vomiting, bone marrow depression, renal damage, and central nervous system depression. Radiation therapy has also been used to target cancer cells, as cancer cells are less able to repair themselves after treatment with radiation. However, radiation cannot be used to treat many cancers because of the sensitivity of normal cells which surround cancerous tissue.

Viral therapy provides an additional tool to treat cancer. Approaches to viral therapy are at least twofold. A first approach includes the use of non-destructive viruses to introduce genes into cells. In this approach, genes can express an enzyme such as thymidine kinase that the cells do not otherwise express. The rationale of this type of therapy is to selectively provide tumor cells with an enzymatic activity that is lacking or is much lower in the normal cells and which renders the tumor cells sensitive to certain drugs. Another approach to viral therapy to treat cancerous cells involves direct inoculation of tumor with attenuated viruses. Attenuated viruses can exhibit a reduced virulence yet are able to actively multiply and may ultimately cause the destruction of infected cells.

Hiraoka et al., (Cancer Research 67(11): 5345-5353, 2007), reports on the use of a retroviral vector encoding cytosine deaminase for tumor therapy in a mouse model. The replication kinetics of this virus in a colorectal cell line were also assessed to confirm that the virus does indeed replicate in these cells.

There remains a need to assess whether viral therapy will be successful in treating a given subject and to develop additional effective vectors for use in viral therapy.

### SUMMARY

Provided herein is a method for predicting efficacy of viral therapy for a tumor in a subject, comprising:
introducing a therapeutic virus into a tumor cell obtained from a biopsy or body fluid of a subject or into a cell culture that comprises the tumor cells;
determining a replication indicator indicative of the level or amount of viral replication within a predetermined period of time or as a function of time after introduction of the therapeutic virus into tumor cells to thereby determine whether replication is delayed compared to a control or standard; and if replication is not delayed, selecting the virus as a candidate therapeutic virus for treatment of the tumor in the subject.

Provided are methods for predicting the efficacy of a particular therapeutic virus for treatment of a particular tumor. As described herein, therapeutic viruses, such as oncolytic viruses, often are effective against one type of tumor (a responder), but not against another (a non-responder). A responder is a tumor cell that is susceptible to treatment with the virus and a non-responder is a tumor cell that is resistant to treatment with the virus.

Methods are provided herein for predicting for which viruses a tumor will be a responder. This permits, for example, selection of an appropriate viral therapy. As shown herein, while many viruses replicate in the tumor, those that will be not be effective for a particular tumor type, exhibit a delay in replication. Hence, the level of replication early after introduction or administration of a virus to a tumor, is an indicator of the viruses efficacy for a particular tumor. Also provided herein, are tumor cell markers, such as housekeeping genes, whose expression decreases upon viral infection and are indicative of non-delayed replication. In addition, as shown herein, the presence or absence of certain host cell makers also can indicate efficacy of therapeutic virus for a particular tumor. To assess such markers or measure viral replication, levels can be compared to suitable controls or to standards or to predetermined values.

For example, delayed replication can be assessed by:
infecting a cell culture with a therapeutic virus, wherein the cell culture contains cells from a tumor;
after a predetermined time, determining a replication indicator of replication of the virus in the culture; and
based on the value of the replication indicator, predicting a therapeutic efficacy of the virus against the tumor.

Viral replication can be assessed in appropriate tumor cells, including, but not limited to, tissue cultured tumor cells or cells from a tumor biopsy or body fluid containing tumor cells.

Therapeutic viruses include any therapeutic viruses known to those of skill in the art, including viruses, such as adenoviruses, herpesviruses and pox viruses, such as vaccinia viruses. Often the virus is an oncolytic virus, which optionally expresses a therapeutic product and/or detectable markers or other appropriate product. Exemplified herein are vaccinia viruses of the strain LIVP, such as the virus that contains inactivated, such as by insertion of heterologous nucleic acid, in the HA, F3 and F14.5 genes/loci. Exemplary of such viruses is the strain designated GLV-1h68, which optionally can be modified to express additional heterologous nucleic acid molecules (in place of or in addition to the inserted heterologous nucleic acid in GLV-1h68.

The replication indicator that is measured is any parameter from which the level or amount or relative amount of viral replication, typically within a day of administration to the tumor cells, can be assessed or inferred. For example, replication can be determined by infecting or introducing the test virus into a tumor cell and assessing viral titer at a particular time or as a function of time. This can be compared to a predetermined standard or compared to other test candidates. Those that replicate relatively early, typically within about or zero to 10 days, about or zero to 5 days, about or zero to 3 days about or zero to 2 days, about or zero to 1 day, such as within two days or one day or 10 to 24 hours or 5 to 10 or 20 hours, are candidate therapeutic viruses. The particular time value to select can be empirically determined if necessary. Thus, the replication indicator can be determined and, for example, can be compared to a standard indicative of delayed replication or non-delayed replication. The standard can be pre-determined, such as a database of values of the indicator that represent non-delayed replication. Thus, for example, the replication indicator can be compared to a database of predetermined values for tumor cell types to determine whether the replication indicator has a value indicative of non-delayed replication.

Replication indicators, include but are not limited to one or more of:
(i) an increase in expression of a viral gene or a heterologous gene encoded by the virus, wherein an increase in expression is indicative that the tumor cells are responsive to virus therapy;
(ii) a decrease in expression of a housekeeping gene expressed in the tumor upon viral expression, wherein a decrease in expression is indicative that the tumor cells are responsive to virus therapy; or
(iii) a change in expression of a gene expressed by the tumor cells, wherein a change in expression is indicative that the tumor cells are responsive to virus therapy

Typically, when gene expression in the tumor cells is assessed, expression or a plurality of such genes, such as housekeeping genes whose expression decreases in tumor cells that are responders, are assessed. Hence panels of genes can be assessed, such as by reacting a nucleic acid sample, with an array (or high density microarray) containing nucleic acid encoding sufficient portions of a plurality of genes to detect expression. In some embodiment, patterns of expression can be detected and correlated with a responder/non-responder phenotype. Genes that can be assed include expression of one or more genes encoding a protein selected from among IL-18 (Interleukin-18), MCP-5 (Monocyte Chemoattractant Protein-5; CCL12), IL-11 (Interleukin-11), MCP-1 (Monocyte Chemoattractant Protein-1), MPO (Myeloperoxidase), Apo A1 (Apolipoprotein A1), TIMP-1 (Tissue Inhibitor of Metalloproteinase Type-1), CRP (C Reactive Protein), Fibrinogen, MMP-9 (Matrix Metalloproteinase-9), Eotaxin (CCL11), GCP-2 (Granulocyte Chemotactic Protein-2; CXCL6), IL-6 (Interleukin-6), Tissue Factor (TF), SAP (Serum Amyloid P), FGF-basic (Fibroblast Growth Factor-basic), MCP-3 (Monocyte Chemoattractant Protein-3; CCL7), IP-10 (CXCL 10), MIP-2, Thrombopoetin, Cancer antigen 125, CD40, CD40 ligand, ENA-78, Ferritin, IL-12p40, IL-12p70, IL-16, MMP-2, PAI-1, TNF RII, TNF-beta and VCAM-1 indicates that the tumor cells are responsive to virus therapy.

Increases in expression of housekeeping genes or panels, such as arrays or probes, for detecting expression of housekeeping genes in a tumor cell following introduction of a virus can be assess, such as a function of time, indicate that a tumor is a non-responder. Housekeeping genes, include genes encoding proteins, such as actin, various ribosomal proteins are well known (see, e.g., the article "Human Housekeeping genes are compact" (2003) Trends in Genetics 19:362-365). For example, increases in gene expression of one or more genes encoding a protein selected from among MIP-1beta (Macrophage Inflammatory Protein-1 beta), MDC (Macrophage-Derived Chemokine; CCL22), MIP-1alpha (Macrophage Inflammatory Protein-1 alpha; CCL3), KC/GROalpha (Melanoma Growth Stimulatory Activity Protein), VEGF (Vascular Endothelial Cell Growth Factor), Endothelin-1, MIP-3 beta (Macrophage Inflammatory Protein-3 beta; Exodus-3 or ELC), Beta-2 microglobulin, IL-5 (Interleukin-5), IL-1 alpha (Interleukin-1 alpha), EGF (Epidermal Growth Factor), Lymphotactin (XCL1), GM-CSF (Granulocyte Macrophage-Colony Stimulating Factor), MIP-1gamma (Macrophage Inflammatory Protein-1gamma; CCL4), IL-1beta (Interteukin-1 beta), Brain-derived neutrophic factor, Cancer antigen 19-9, Carcinoembryonic antigen, C reactive protein, EGF, Fatty acid binding protein, Factor VII, Growth hormone, IL-1 alpha, 1L-1 beta, IL-1 rat, IL-7, IL-8, MDC, Prostatic acid phosphatase, Prostate specific antigen, free, Stem cell factor, Tissue factor, TNF-alpha, VEGF and Von Willebrand factor, indicates that the tumor cells are not responsive to virus therapy.

In all of the methods, the virus can be modified to include a gene that encodes a protein whose expression is increased in responders compared to non-responders or encodes a gene product that reduces expression of a protein whose level of expression is increased in non-responders compared to responders. Those genes whose expression improves response to the virus can be included in the therapeutic virus to improve therapeutic efficacy.

As noted, a responder is a tumor cell that is susceptible to treatment with the virus and a non-responder is a tumor cell the is resistant to treatment with the virus.

### DETAILED DESCRIPTION

**A. Definitions**
**B. Methods for Assessing Viral Therapy**
   **1. Methods of Assessing Whether a Subject is Likely to Respond Favorably or Poorly to Viral Therapy by Assessing a Replication Indicator**
      **a. Virus titer**
      **b. Expression of virus genes**
      **c. Decreased expression of housekeeping genes**
      **d. Expression of tumor proteins**
   **2. Methods of Assessing Whether a Subject is Likely to Respond favorably or Poorly to Viral Therapy by Marker Expression Profiling**
**C. Therapeutic Viruses**
   **1. Modifications of Therapeutic Viruses**
   **2. Viruses Encoding a Marker Protein that is Increased in Cells that Respond Favorable to Tumor Therapy**
      **a. IP-10 encoding viruses**
      **b. MCP-1 encoding viruses**
      **c. TIMP-1, 2, 3 encoding viruses**
   **3. Viruses an Agent which Reduces the Level of Expression of a Marker Protein**
**D. Host Cells**
   **1. Harvesting tumor cells from patient**
**E. Pharmaceutical Compositions**
**F. Methods of administering viral therapy**
   **1. Monitoring the progress of viral therapy**
**G. Identifying markers associated with a response to viral therapy**
**H. Identifying a virus for viral therapy**
**I. Articles of Manufacture and Kits**
**J. Examples**

### A. DEFINITIONS

Unless defined otherwise, all technical and scientific terms used herein have the same meaning as is commonly understood by one of skill in the art to which the invention(s) belong.

In the event that there is a plurality of definitions for terms herein, those in this section prevail. Where reference is made to a URL or other such identifier or address, it is understood that such identifiers can change and particular information on the internet can come and go, but equivalent information is known and can be readily accessed, such as by searching the internet and/or appropriate databases. Reference thereto evidences the availability and public dissemination of such information. To the extent, if any, that publications and patents or patent applications contradict the disclosure contained in the specification, the specification is intended to supersede and/or take precedence over any such contradictory material.

As used herein, "virus" refers to any of a large group of entities referred to as viruses. Viruses typically contain a protein coat surrounding an RNA or DNA core of genetic material, and are capable of growth and multiplication only in living cells. Viruses for use in the methods provided herein include, but are not limited, to a poxvirus, including a vaccinia virus. Other exemplary viruses include, but are not limited to, adenovirus, adeno-associated virus, herpes simplex virus, Newcastle disease virus, vesicular stomatitis virus, mumps virus, influenza virus, measles virus, reovirus, human immunodeficiency virus (HIV), hanta virus, myxoma virus, cytomegalovirus (CMV), lentivirus, Sindbis virus, and any plant or insect virus.

As used herein, the term "viral vector" is used according to its art-recognized meaning. It refers to a nucleic acid vector construct that includes at least one element of viral origin and can be packaged into a viral vector particle. The viral vector particles can be used for the purpose of transferring DNA, RNA or other nucleic acids into cells either *in vitro* or *in vivo.* Viral vectors include, but are not limited to, retroviral vectors, vaccinia vectors, lentiviral vectors, herpes virus vectors (e.g., HSV), baculoviral vectors, cytomegalovirus (CMV) vectors, papillomavirus vectors, simian virus (SV40) vectors, semliki forest virus vectors, phage vectors, adenoviral vectors, and adeno-associated viral (AAV) vectors.

As used herein, the term "modified" with reference to a gene refers to a deleted gene, a gene encoding a gene product having one or more truncations, mutations, insertions or deletions, or a gene that is inserted (into the chromosome or on a plasmid, phagemid, cosmid, and phage) encoding a gene product, typically accompanied by at least a change in function of the modified gene product or virus.

As used herein, the term "modified virus" refers to a virus that is altered with respect to a parental strain of the virus. Typically modified viruses have one or more truncations, mutations, insertions or deletions in the genome of virus. A modified virus can have one or more endogenous viral genes modified and/or one or more intergenic regions modified. Exemplary modified viruses can have one or more heterologous nucleic acid sequences inserted into the genome of the virus. Modified viruses can contain one more heterologous nucleic acid sequences in the form of a gene expression cassette for the expression of a heterologous gene. As used herein, modification of a heterologous nucleic acid molecule with respect to a virus containing a heterologous nucleic acid molecule refers to any alteration of the heterologous nucleic acid molecule including truncations, mutations, insertions, or deletions of the nucleic acid molecule. Modification of a heterologous nucleic acid molecule can also include alteration of the viral genome, which can be, for example, a deletion of all or a potion heterologous nucleic from the viral genome or insertion of an additional heterologous nucleic acid molecule into the viral genome.

As used herein, the term "therapeutic virus" refers to a virus that is administered for the treatment of a disease or disorder. A therapeutic virus is typically a modified virus. Such modifications include one or more insertions, deletions, or mutations in the genome of the virus. Therapeutic viruses typically possess modifications in one or more endogenous viral genes or one or more intergenic regions, which attenuate the toxicity of the virus, and can optionally express a heterologous therapeutic gene product and/or detectable protein. Therapeutic viruses can contain heterologous nucleic acid molecules, including one or more gene expression cassettes for the expression of the therapeutic gene product and/or detectable protein. Therapeutic viruses can be replication competent viruses (e.g., oncolytic viruses) including conditional replicating viruses, or replication-defective viruses. As used herein, the term, "therapeutic gene product" refers to any heterologous protein expressed by the therapeutic virus that ameliorates the symptoms of a disease or disorder or ameliorates the disease or disorder.

As used herein, a responder is a tumor cell for which a therapeutic virus is effective against *in vivo.* The methods provided herein provide in vitro assays for predicted whether a particular tumor is a responder or a non-responder. If a tumor is a predicted responder for a therapeutic virus, the tumor is likely to respond favorably to tumor treatment. As used herein, a tumor that respond favorably to a treatment with a therapeutic virus means that treatment of a tumor with the virus will cause the tumor to slow or stop tumor growth, or cause the tumor to shrink or regress.

As used herein, a nonresponder is a tumor for which a therapeutic virus is not effective against *in vivo.*

As used herein, a marker is any gene product for which level of gene expression is assayed. A marker can be a gene product that is increased, decreased, or unchanged in a tumor or that is increased, decreased, or unchanged in a tumor that is treated with a virus. A marker can also be a gene product that is increased, decreased or unchanged in a subject that bears a tumor or that is increased, decreased, or unchanged in a subject that bears a tumor and that is treated with a virus. The characteristic levels of expression of one or more marker proteins in a tumor or in a host that bears a tumor can be used to generate a marker profile, or expression profile for the particular tumor. Marker profiles can be generated for untreated tumors and tumors that have been treated with a virus.

As use herein, delayed replication refers to the inability of a therapeutic virus to efficiently replicate in a tumor in the *in vitro* replication assay methods provided herein. Viruses that exhibit delayed replication in a tumor following infection of the tumor are predicted to not be effective for therapy of

As used herein, a replication indicator is any parameter indicative of viral replication. For example, such indicators include, but are not limited to, virus titer, expression of viral proteins, expression of reporter proteins, expression of host housekeeping genes or other host proteins.

As used herein, a housekeeping gene is a gene involved in basic functions needed for the sustenance of the cell. Housekeeping genes are constitutively expressed. Exemplary housekeeping genes can be found in the Examples and elsewhere herein.

As used herein, attenuation of a virus means to a reduction or elimination of deleterious or toxic effects to a host upon administration of the virus compared to an un-attenuated virus. As used herein, a virus with low toxicity means that upon administration a virus does not accumulate in organs and tissues in the host to an extent that results in damage or harm to organs, or that impacts survival of the host to a greater extent than the disease being treated does. For the purposes herein, attenuation of toxicity is used interchangeably with attenuation of virulence and attenuation of pathogenicity.

As used herein, the term "viral load" is the amount of virus present in the blood of a patient. Viral load also is referred to as viral titer or viremia. Viral load can be measured in variety of standard ways, including immunochemistry methods or by plaque assay.

As used herein, the term "toxicity" with reference to a virus refers to the ability of the virus to cause harm to the subject to which the virus has been administered.

As used herein virulence and pathogenicity with reference to a virus refers to the ability of the virus to cause disease or harm in the subject to which the virus has been administered. Hence, for the purposes herein the terms toxicity, virulence, and pathogenicity with reference to a virus are used interchangeably.

As used herein, a delivery vehicle for administration refers to a lipid-based or other polymer-based composition, such as liposome, micelle, or reverse micelle, which associates with an agent, such as a virus provided herein, for delivery into a host animal.

As used herein, a disease or disorder refers to a pathological condition in an organism resulting from, for example, infection or genetic defect, and characterized by identifiable symptoms.

As used herein, treatment means any manner in which the symptoms of a condition, disorder or disease are ameliorated or otherwise beneficially altered. Treatment also encompasses any pharmaceutical use of the viruses described and provided herein.

As used herein, amelioration or alleviation of symptoms associated with a disease refers to any lessening, whether permanent or temporary, lasting or transient of symptoms that can be attributed to or associated with a disease. Similarly, amelioration or alleviation of symptoms associated with administration of a virus refers to any lessening, whether permanent or temporary, lasting or transient of symptoms that can be attributed to or associated with an administration of the virus for treatment of a disease.

As used herein, an effective amount of a virus or compound for treating a particular disease is an amount that is sufficient to ameliorate, or in some manner reduce the symptoms associated with the disease. Such an amount can be administered as a single dosage or can be administered according to a regimen, whereby it is effective. The amount can cure the disease but, typically, is administered in order to ameliorate the symptoms of the disease. Repeated administration can be required to achieve the desired amelioration of symptoms.

As used herein, an effective amount of a therapeutic agent for control of viral unit numbers or viral titer in a patient is an amount that is sufficient to prevent a virus introduced to a patient for treatment of a disease from overwhelming the patient's immune system such that the patient suffers adverse side effects due to virus toxicity or pathogenicity. Such side effects can include, but are not limited to fever, abdominal pain, aches or pains in muscles, cough, diarrhea, or general feeling of discomfort or illness that are associated with virus toxicity and are related to the subject's immune and inflammatory responses to the virus. Side effects or symptoms can also include escalation of symptoms due to a systemic inflammatory response to the virus, such as, but not limited to, jaundice, blood-clotting disorders and multiple-organ system failure. Such an amount can be administered as a single dosage or can be administered according to a regimen, whereby it is effective. The amount can prevent the appearance of side effects but, typically, is administered in order to ameliorate the symptoms of the side effects associated with the virus and virus toxicity. Repeated administration can be required to achieve the desired amelioration of symptoms.

As used herein, an *in vivo* method refers to a method performed within the living body of a subject.

As used herein, a subject includes any animal for whom diagnosis, screening, monitoring or treatment is contemplated. Animals include mammals such as primates and domesticated animals. An exemplary primate is human. A patient refers to a subject such as a mammal, primate, human, or livestock subject afflicted with a disease condition or for which a disease condition is to be determined or risk of a disease condition is to be determined.

As used herein, the term "neoplasm" or "neoplasia" refers to abnormal new cell growth, and thus means the same as tumor, which can be benign or malignant. Unlike hyperplasia, neoplastic proliferation persists even in the absence of the original stimulus.

As used herein, neoplastic disease refers to any disorder involving cancer, including tumor development, growth, metastasis and progression.

As used herein, cancer is a term for diseases caused by or characterized by any type of malignant tumor, including metastatic cancers, lymphatic tumors, and blood cancers. Exemplary cancers include, but are not limited to: leukemia, lymphoma, pancreatic cancer, lung cancer, ovarian cancer, breast cancer, cervical cancer, bladder cancer, prostate cancer, glioma tumors, adenocarcinomas, liver cancer and skin cancer. Exemplary cancers in humans include a bladder tumor, breast tumor, prostate tumor, basal cell carcinoma, biliary tract cancer, bladder cancer, bone cancer, brain and CNS cancer (e.g., glioma tumor), cervical cancer, choriocarcinoma, colon and rectum cancer, connective tissue cancer, cancer of the digestive system; endometrial cancer, esophageal cancer; eye cancer; cancer of the head and neck; gastric cancer; intra-epithelial neoplasm; kidney cancer; larynx cancer; leukemia; liver cancer; lung cancer (e.g. small cell and non-small cell); lymphoma including Hodgkin's and Non-Hodgkin's lymphoma; melanoma; myeloma, neuroblastoma, oral cavity cancer (e.g., lip, tongue, mouth, and pharynx); ovarian cancer; pancreatic cancer, retinoblastoma; rhabdomyosarcoma; rectal cancer, renal cancer, cancer of the respiratory system; sarcoma, skin cancer; stomach cancer, testicular cancer, thyroid cancer; uterine cancer, cancer of the urinary system, as well as other carcinomas and sarcomas. Malignant disorders commonly diagnosed in dogs, cats, and other pets include, but are not limited to, lymphosarcoma, osteosarcoma, mammary tumors, mastocytoma, brain tumor, melanoma, adenosquamous carcinoma, carcinoid lung tumor, bronchial gland tumor, bronchiolar adenocarcinoma, fibroma, myxochondroma, pulmonary sarcoma, neurosarcoma, osteoma, papilloma, retinoblastoma, Ewing's sarcoma, Wilm's tumor, Burkitt's lymphoma, microglioma, neuroblastoma, osteoclastoma, oral neoplasia, fibrosarcoma, osteosarcoma and rhabdomyosarcoma, genital squamous cell carcinoma, transmissible venereal tumor, testicular tumor, seminoma, Sertoli cell tumor, hemangiopericytoma, histiocytoma, chloroma (e.g., granulocytic sarcoma), corneal papilloma, corneal squamous cell carcinoma, hemangiosarcoma, pleural mesothelioma, basal cell tumor, thymoma, stomach tumor, adrenal gland carcinoma, oral papillomatosis, hemangioendothelioma and cystadenoma, follicular lymphoma, intestinal lymphosarcoma, fibrosarcoma and pulmonary squamous cell carcinoma. In rodents, such as a ferret, exemplary cancers include insulinoma, lymphoma, sarcoma, neuroma, pancreatic islet cell tumor, gastric MALT lymphoma and gastric adenocarcinoma. Neoplasias affecting agricultural livestock include leukemia, hemangiopericytoma and bovine ocular neoplasia (in cattle); preputial fibrosarcoma, ulcerative squamous cell carcinoma, preputial carcinoma, connective tissue neoplasia and mastocytoma (in horses); hepatocellular carcinoma (in swine); lymphoma and pulmonary adenomatosis (in sheep); pulmonary sarcoma, lymphoma, Rous sarcoma, reticulo-endotheliosis, fibrosarcoma, nephroblastoma, B-cell lymphoma and lymphoid leukosis (in avian species); retinoblastoma, hepatic neoplasia, lymphosarcoma (lymphoblastic lymphoma), plasmacytoid leukemia and swimbladder sarcoma (in fish), caseous lumphadenitis (CLA): chronic, infectious, contagious disease of sheep and goats caused by the bacterium Corynebacterium pseudotuberculosis, and contagious lung tumor of sheep caused by jaagsiekte.

As used herein, the term "malignant," as it applies to tumors, refers to primary tumors that have the capacity of metastasis with loss of growth control and positional control.

As used herein, metastasis refers to a growth of abnormal or neoplastic cells distant from the site primarily involved by the morbid process.

As used herein, proliferative disorders include any disorders involving abnormal proliferation of cells, such as, but not limited to, neoplastic diseases.

As used herein, a method for treating or preventing neoplastic disease means that any of the symptoms, such as the tumor, metastasis thereof, the vascularization of the tumors or other parameters by which the disease is characterized are reduced, ameliorated, prevented, placed in a state of remission, or maintained in a state of remission. It also means that the indications of neoplastic disease and metastasis can be eliminated, reduced or prevented by the treatment. Non-limiting examples of the indications include uncontrolled degradation of the basement membrane and proximal extracellular matrix, migration, division, and organization of the endothelial cells into new functioning capillaries, and the persistence of such functioning capillaries.

As used herein, an anti-cancer agent or compound (used interchangeably with "anti-tumor or anti-neoplastic agent") refers to any agents, or compounds, used in anti-cancer treatment. These include any agents, when used alone or in combination with other compounds, that can alleviate, reduce, ameliorate, prevent, or place or maintain in a state of remission of clinical symptoms or diagnostic markers associated with neoplastic disease, tumors and cancer, and can be used in methods, combinations and compositions provided herein. Exemplary anti-cancer agent agents include, but are not limited to, the viruses provided herein used singly or in combination and/or in combination with other anti-cancer agents, such as cytokines, growth factors, hormones, photosensitizing agents, radionuclides, toxins, prodrug converting enzymes, anti-metabolites, signaling modulators, anti-cancer antibiotics, anti-cancer antibodies, anti-cancer oligopeptides, angiogenesis inhibitors, radiation therapy, hypothermia therapy, hyperthermia therapy, laser therapy, chemotherapeutic compounds, or a combination thereof.

As used herein, a prodrug is a compound that, upon *in vivo* administration, is metabolized or otherwise converted to the biologically, pharmaceutically or therapeutically active form of the compound. To produce a prodrug, the pharmaceutically active compound is modified such that the active compound is regenerated by metabolic processes. The prodrug can be designed to alter the metabolic stability or the transport characteristics of a drug, to mask side effects or toxicity, to improve the flavor of a drug or to alter other characteristics or properties of a drug. By virtue of knowledge of pharmacodynamic processes and drug metabolism *in vivo,* those of skill in this art, once a pharmaceutically active compound is known, can design prodrugs of the compound (see, e.g., Nogrady (1985) Medicinal Chemistry A Biochemical Approach, Oxford University Press, New York, pages 388-392).

As used herein the term assessing or determining is intended to include quantitative and qualitative determination in the sense of obtaining an absolute value for the activity of a product, and also of obtaining an index, ratio, percentage, visual or other value indicative of the level of the activity. Assessment can be direct or indirect.

An oncolytic virus is a virus that preferentially replicates in, and kills, neoplastic or cancer cells. The virus can be a naturally-occurring virus or an engineered virus. Preferably, the virus is a modified vaccinia virus.

As used herein, the phrase "immunoprivileged cells and tissues" refers to cells and tissues, such as solid tumors and wounded tissues, which are sequestered from the immune system.

As used herein, an array refers to a collection of elements, such as proteins, nucleic acids, cells or viruses, containing three or more members. An addressable array is one in which the members of the array are identifiable, typically by position on a solid phase support or by virtue of an identifiable or detectable label, such as by color, fluorescence, electronic signal (i.e. RF, microwave or other frequency that does not substantially alter the interaction of the molecules of interest), bar code or other symbology, chemical or other such label. Hence, in general the members of the array are immobilized to discrete identifiable loci on the surface of a solid phase or directly or indirectly linked to or otherwise associated with the identifiable label, such as affixed to a microsphere or other particulate support (herein referred to as beads) and suspended in solution or spread out on a surface.

The term "array" is to be construed broadly, and includes any arrangement wherein a plurality of different polypeptides are held, presented, positioned, situated, or supported. Arrays can include microtiter plates, such as 48-well, 96-well, 144-well, 192-well, 240-well, 288-well, 336-well, 384-well, 432-well, 480-well, 576-well, 672-well, 768-well, 864-swell, 960-well, 1056-well, 1152-well, 1248-well, 1344-well, 1440-well, or 1536-well plates, tubes, slides, chips, flasks, or any other suitable laboratory apparatus. Furthermore, arrays can also include a plurality of sub-arrays. A plurality of sub-arrays encompasses an array where more than one arrangement is used to position the polypeptides. For example, multiple 96-well plates could constitute a plurality of sub-arrays and a single array.

As used herein, an address refers to a unique identifier whereby an addressed entity can be identified. An addressed moiety is one that can be identified by virtue of its address. Addressing can be effected by position on a surface or by other identifiers, such as a tag encoded with a bar code or other symbology, a chemical tag, an electronic, such RF tag, a color-coded tag or other such identifier.

As used herein, "a combination" refers to any association between two or among more items. Such combinations can be packaged as kits.

As used herein, a composition refers to any mixture. It can be a solution, a suspension, an emulsion, liquid, powder, a paste, aqueous, non-aqueous or any combination of such ingredients.

As used herein, fluid refers to any composition that can flow. Fluids thus encompass compositions that are in the form of semi-solids, pastes, solutions, aqueous mixtures, gels, lotions, creams and other such compositions.

As used herein, a kit is a packaged combination, optionally, including instructions for use of the combination and/or other reactions and components for such use.

The section headings used herein are for organizational purposes only and are not to be construed as limiting the described subject matter in any way.

It is to be understood that the foregoing general description and the following detailed description are exemplary and explanatory only and are not restrictive. The articles "a" and "an" are used herein to refer to one or to more than one (i.e., to at least one) of the grammatical object of the article. By way of example, "an element" means one element or more than one element. Further, unless otherwise required by context, singular terms shall include pluralities and plural terms shall include the singular.

As used herein, "comprising" as used herein is synonymous with "including," "containing," or "characterized by," and is inclusive or open-ended and does not exclude additional, unrecited elements or method steps.

All numbers expressing quantities are to be understood as being modified in all instances by the term "about." The word "about" carries the understanding that the number referred to can vary by up to ±10%, unless indicated otherwise in the text or as understood in the art, and still remain within the meaning of the disclosure. At the very least, and not as an attempt to limit the application of the doctrine of equivalents to the scope of the claims, each numerical parameter should be construed in light of the number of significant digits and ordinary rounding approaches.

Generally, nomenclature used in connection with, and techniques of, cell and tissue culture, molecular biology, and protein and oligo- or polynucleotide chemistry and hybridization described herein are those well known and commonly used in the art. The techniques and procedures described herein are generally performed according to conventional methods well known in the art and as described in various general and more specific references that are cited and discussed throughout the instant specification, for example, Sambrook et al., Molecular Cloning: A Laboratory Manual (Third ed., Cold Spring Harbor Laboratory Press, Cold Spring Harbor, N.Y. 2000). The nomenclatures utilized in connection with, and the laboratory procedures and techniques described herein are those well know and commonly used in the art.

### B. METHODS FOR ASSESSING VIRAL THERAPY

Provided herein are methods and compositions for viral therapy. Methods to predict or assess or determine a subject's, such as a subject with a tumor or cancer, response to viral therapy are provided. The methods include assessing whether a subject bearing a tumor is likely to respond to treatment of the tumor with a therapeutic virus. In some examples, the methods include measuring a replication indicator that is predictive for tumor response to viral therapy. In some examples, markers for use in assessing responses to viral therapy are provided.. Therapeutic viruses designed for viral therapy also are described elsewhere herein.

Also described herein are methods to identify markers associated with a subject's response to viral therapy. In some examples, methods to assess a candidate therapeutic virus are provided. Methods for administering viral therapy to a subject also are described. In further examples, methods to monitor the progress of viral therapy in a subject are described. Also described are pharmaceutical compositions containing a therapeutic virus. In some examples, kits to measure markers are described.

### 1. Methods of Assessing Whether a Subject is Likely to Respond Favorably or Poorly to Viral Therapy by Assessing a Replication Indicator

Provided herein are methods of assessing the likely response of a tumor to treatment with a therapeutic virus by assessing a replication indicator. As described herein, virus replication patterns in tissue cultures can be used to predict in vivo viral therapeutic effects. For example, a tissue or cell sample can be obtained (e.g., biopsy) from a subject (e.g., human or non-human animal subject), and the sample can be infected with one or more types of viruses. Based on the replication patterns of the one or more viruses, therapeutic effects of those viruses on the tissue or cells (from which the sample was obtained) can be predicted. In some examples, such prediction can be a binary prediction, indicating whether the tissue or cells of interest will be a responder type or a non-responder type.

As described herein by examples, known tumor cell lines can be characterized, for examples, as responders or non-responders for various types of viruses. Such information can be stored in a database in known manners. During a viral therapy planning phase, such database can be utilized to customize a treatment if information about the to-be-treated tumor cell line is available. In some example the database can be used as a standard by to compare a replication indicator determined from a tumor sample obtained from a subject.

If information about the particular tumor type is uncertain or not known, prediction of therapeutic effects can be made by obtaining replication patterns of one or more viruses in a culture of a sample obtained from the subject (*e.g*., tumor biopsy). For example, a sample can be obtained from a patient, and be infected by one or more viral candidates. After a certain period, replication indicator, such as changes in viral titer or relative gene expression, can be obtained; and based on such indicator values, prediction as to therapeutic effect of each of the viral candidates can be made.

In some examples, determination of the replication indicator in the tissue or cell sample can be achieved via an array having different types of viruses, or an array capable of receiving such viruses for infection. In some examples, a panel of one or more candidate viruses in an array is assayed. In some examples, one or more cell types or tumor samples are assayed. The tumor samples can include known responders, known non-responders or unknown responders or non-responders, or a combination thereof. In some examples, such an array can be a microarray. In some example, replication indicators from the array can be at least partially automated or controlled by a computing device.

In some embodiments, such period for determining viral replication patterns can be in a range of approximately zero to 10 days, zero to 5 days, zero to 2 days, zero to 1 day, zero to 2 days or more. Other ranges of such period also are possible. Viral titers can also be assayed at any time point during the experiment. In some examples, a replication indicator such as viral titer can be obtained at approximately 24 hours after infection. In other examples the virus titer is assayed before 24 hours, such as for example 6 hours or more or 12 hours of more.

In some viruses, period associated with lagging of viral replication in non-responders may vary. Accordingly, such variations can be accounted for in forming predictions of therapeutic effects.

In some applications, replication indicators can include assaying viral titer performed using a standard method, such as by plaque assy. In some examples, viruses can encode other gene products whose expression can be used as an indicator of viral replication, such as, for example, but not limited to, proteins that emit light. In some examples, the virus can encode a protein such as a fluorescent protein or a luciferase or a combination thereof. In one example, the virus is vaccinia virus, such as GLV-1h68. Other replication indictors include but are not limited to detection of changes in viral gene expression or host gene expression.

As described herein, obtaining the viral replication patterns relatively early after infection (around 24 hours post infection) can provide a replication index indicative of therapeutic effect of the virus against a particular tumor of tumor type. Such indices can be assigned for a given combination of virus and cell line. In some examples, such a therapeutic index can be within a finite range of values.

The therapeutic index can be formed, for example, by plotting a curve of the relative change in tumor volume against time after virus injection and a curve of the change in tumor volume during the same time period in the absence of virus. The untreated control curve represents the relative change in tumor volume without virus treatment; and the virus treatment curve represents the relative change in tumor volume with virus treatment. Such studies can be performed in well-recognized tumor xenograft models as described herein.

One example way of forming a therapeutic index is to determine or estimate a quantity given by $therapeutic index = \left(A-B\right) / A$
where A is the area under the untreated control curve, and B is the area under the virus treatment curve, with both as from time T0 to T. The time T0 can be the time when virus is introduced to the tumor-bearing subject, and can be measured in appropriate time scale such as days.

The exemplary therapeutic index ((A-B)/A) can generally provide a range of values between approximately zero and one. For example, if the value ofB is relatively low (where tumor volume increase is relatively low followed by a decrease in volume), the therapeutic index is closer to one. On the other hand, if the value of B is relatively high (where tumor volume increase is relatively high), the therapeutic index is closer to zero. In some examples, the use of integrated values (*e.g*., areas under curves) associated with curves can average out fluctuations that may be present in the curves, which thereby yields a more accurate representation of the therapeutic index. Also, in some examples, normalizing the therapeutic index, such as in the examples provided herein, can facilitate a more meaningful comparison of various combinations of viruses and cell lines.

In some embodiments, various concepts of the present disclosure can be utilized for applications such as cancer treatment planning and/or patient screening. For example, a biopsy can be performed on a patient, and the sample tissue can be prepared for infection. A biopsy sample may contain cancerous and healthy cells. Thus, such preparation can include a step where, for example, healthy cells are allowed to die off in a known manner (*e.g*., over a period such as one week). Various known techniques can be utilized to increase the relative number of cancerous cells in the biopsy sample prior to viral infection.

A replication indicator can be any parameter that correlates with the ability of a therapeutic virus to replicate efficiently in a tumor. As described herein and in the Examples, delayed replication of a virus *in vitro* in a tumor cell is indicative of the inability of the virus to cause tumor regression *in vivo.* Likewise, efficient and early replication of the virus following infection of a tumor cell *in vitro* is indicative of a favorable response to tumor therapy by the virus *in vivo.*

### a. Virus titer

Determination of virus titer can be used a replication indicator. In some examples, cells obtained from a biopsy can be infected (e.g., 0.01 MOI) with a therapeutic virus (e.g., GLV-1h68). Such infected cells can be assayed for viral titer after a selected time (e.g., 24 hours), and such viral titer can be used to predict the therapeutic index of the therapeutic virus on the tumor or cancerous cells associated with the biopsied cells.

In some examples, a threshold value can be assigned to allow prediction of responsiveness or non-responsiveness of a particular tumor. A threshold value can be assigned at, for example, a viral titer value between 4.00 and 4.50 log pfu/10⁶ above which a particular tumor is classified a responder.

As shown in the examples provided, where the therapeutic index values are constrained in a finite range, the slopes of lines increase as the viral titer time (post infection) increases. It is therefore advantageous to have as large a range of viral titer values as possible so as to allow better separation of such values. In the examples provided, the 24 time point following infection of the tumor cells provided the largest range of viral titer values among the three example times assayed (24, 48, and 72 hours), thereby allowing more effective separation and classifying of cell lines.

In the example data described herein, the 24-hour viral titer provides a relatively convenient and fast method for obtaining sufficient replication statistics for the purpose of classifying tumor cells as better responders or poorer responders to the candidate virus (*e.g*. GLV-1h68). It will be understood, however, that other viral titer times can be selected for other viruses and/or cell lines. For example, earlier or later time points such as for example around 12 hour to around 36 hours can be assayed in order to classify the candidate viruses as responder or non responders.

In the examples provide herein, the multiplicity of infection employed MOI was 0.01. It is understood, however, that other MOI can be selected to allow better separation of, viral titer values, such as for example in the range of around 0.001 MOI - 1 MOI.

In some examples, the viral titer of the biopsy sample can be accompanied by one or more controls. In some examples, one or more known cell lines can be infected and assayed along with the cells from the biopsy. For example, known cell lines that are responders such as, for example, PANC-1 and GI-101A, and/or non responders, such as for example, PC-3 and MB-231 can be used as control(s). Viral titer from the biopsied cells, normalized in an appropriate manner, can be compared with titers from the known cell line(s). If the biopsied cells' viral titer is less than or equal to that of PC-3, for example, such cells can be considered to be potential poorer responders; and if the biopsied cells' viral titer is greater than or equal to that of PANC-1, for example, such cells can be considered to be potential better responders. If the biopsied cells' viral titer is between the values of PC-3 and PANC-1, a classification can be made which may include consideration of other factors.

### b. Expression of virus genes

Another exemplary replication indicator can be a change in the expression of overall expression of vaccinia viral genes. As described in the examples provided herein, the overall expression of vaccinia viral genes was upregulated in the responder tumor cells compared to the non-responder cells at 24 hours post viral infection. Thus, an assay for one or more viral genes or a panel of viral genes can be performed in order to determine whether the virus efficiently replicates in the tumor cell type. If the infected tumor cell exhibits an increase in viral gene expression, the tumor cell can be classified as a predicted responder to viral therapy. In the methods provided herein, one or more viral genes can be assessed to determine the level of viral gene expression in the infected tumor cells. For example, 1, 2, 3, 4, 5 or more, 10 or more, 50 or more 100 or more housekeeping genes can be assessed. Exemplary viral genes that can be assayed for oncolytic viruses, such as vaccinia viruses, are provided elsewhere herein. Such viral genes can be arrayed on microarrays and micro array analysis can be performed to determine the level of viral gene expression using standard techniques well known in the art and described elsewhere herein.

### c. Decreased expression of housekeeping genes

An additional exemplary replication indicator provided herein is the downregulation housekeeping genes in the tumor cell. Previous studies have shown that housekeeping genes are dysregulated in tumors infected with viruses indicative of the infected host shutdown of cellular functions due to the increased energy demands of the infecting virus (Guerra, S (2007) J Virol. 81:8707-8721). As described herein, the nearly all of the housekeeping genes in a responder tumor tested had decreased expression within 24 hours of viral infection. Housekeeping gene expression was not altered in the non-responder tumor during the same period (see Examples).

In the methods provided herein, one or more housekeeping genes can be assessed to determine whether the level of expression decreases in the presence of the virus. For example, 1, 2, 3, 4, 5 or more, 10 or more, 50 or more 100 or more housekeeping genes can be assessed. Exemplary housekeeping genes include, but are not limited to ACTB, ALDOA, GAPD, PGK1, LDHA, RPS27A, RPL19, RPL11, NONO, GDI, ARHGDIA, RPL32, RPS 18, HSPCB, ILF2, USP11, ATP6V1G1, A1S9T, UBE1, CSNK2B, CPNE1, TNFRSF5, CTNNB1, EIF3S7, NDUFA1, ARAF1, SAFB, ATP6IP1, H2BFL, COX7A2L, ENSA, BTF3, ETR, ATP5J2, SFRS9, G10, CSTB, SLC9A3R2, TETRAN, VEGFB, STK24, RAD9, EFNA3, ARHGAP1, TAPBP, BAT1, TKT, HLA-C, RAB1A, UBE2D2, UBE2M, GNAS, PTBP1, RPL36AL, C21orf33, GPI, COX7C, EIF4A2, COX6B, FBR-MuSV, FAU, GRIK5, COX5B, COX5A, CDC10, VAMP3, GPAA1, PABPN1, HSBP1, YARS, UBE2I, PABPC1, GCNSL1, COX4I1, SPAG7, PSMD8, ZFP36L1, ODC1, RPL18, RPL13, RPS11, CCND3, RPL14, PSMD11, TPMT, RPL8, MTA1, COL6A1, AP2M1, ATP5D, STK19, RPS25, RPS19, MAPKAPK2, AIF1, C14orf2, MAP4, RPS9, B4GALT3, CCBP2, RPS5, TPR-containingSGT, H6PD, MMPL1, E2F4, ADAM15, ADD1, ADAR, PAX8, ANXA6, CHIT1, TAGLN, FOLR1, ACTN4, RING1, ACVRL1, CDA, PTTG1IP, BCRP1, JAG1, ID3, ARHA, SULT1A3, CANX, ARF5, ARF4, ARF1, TSTA3, GDI2, SSR2, ADRBK1, ELAVL3, CAPZB, SNRPA, SDHA, PPP2CB, PITPNM, ILK, HDGF, GGTLA1, NEDD5, DAP, CSK, COX8, ANXA2, SSTR5, CTBP1, CHD4, ZNF91, ZNF91, TTC1, TEGT, SRM, SGSH, PSME2, PRKAG1, PGD, PRDX1, NM23B, MTX1, MSN, MC2R, LTBP4, LMO1, IMPDH2, IFITM 1, GRM4, GNAI2, GDI1, GAS1, FTH1, EIF4G2, DAXX, CNTN1, BSG, ARL2, ARF3, DNCL1, HLA-G, HGS, C11orf13, ATPSA1, CSNK1E, SNX3, CTSD, PSMA7, PSMB7, LDHB, SREBF1, PSMB4, PSMB2, PSMB1, MYH9, CENPB, PFDN5, SYNGR2, AP1B1, H3F3A, ARHGEF7, YWHAZ, MAP3K11, AES, VIL2, PHF1, PFDN1, CKB, YWHAH, RNH, SLC25A11, CYC1, PTMA, SNRPG, TUFM, YWHAB, RPA2, CD81, CALM2, ATP6V1F, H2AFY, NDUFB7, HMGB1, CD23A, FCER2, GUK1, G22P1, BECN1, MCM3AP, CSF1, HPCAL1, ATP6V0C, ATP6V0B, ATP6V1E1, COX7A2, COX6A1, FKBP1A, RPL29, RPL27, RPLP2, RPLP1, GM2A, RPL3, ENO1, RPL38, RPL37, RPL34, RPL15, RPS2, RPS24, RPS16, RPS 15, RPS 13, RPL5, RPL17, POLR2A, RPS12, HNRPK, HNRPD, HNRPAB, RPS10, MAZ, MYC, FBL, AP2S1, ACTG1, M6PR, SNRPD2, LGALS9, DIA1, COMT, MGAT1, EIF3S8, DDT, FUS, ALTE, RRBP1, NDUFS5, ERH, B2M, LYZ, NM23A, MVK, ENTPD6, UQCRC1, TXN, TUBB, TCOF1, SRP14, SRF, SOD1, SNRPB, SNRP70, SFRS2, RPS6KB2, RPN1, PRKCSH, PNMT, PKM2, PIM1, SLC25A3, NDUFC1, NDUFA2, MPG, MIF, JAK1, HRMT1L2, GPX4, GP2, GOT2, EXTL3, EIF3S5, EIF3S4, EIF3S2, DHCR7, DAD1, CYB5, CLTB, CLTA, CKAP1, CAPNS1, ATP5O, ATP5G3, ATF4, APLP2, ZNF, DNAJB6, HSPA8, PHGDH, RAP1B, SKIP, MBC2, SAP18, COPS6, ARPC4, ARPC3, NSAP1, BMI1, TERF2IP, ARPC2, STARD7, FEZ1, FBXO7, PLSCR3, PDAP1, DXSE, STS, PRPH, ZNF, RAGA, C1D, GABARAPL2, TADA3L, SEC61G, HAX1, DNPEP, CGI-57, DKFZPK, MGC, M9, AF69, PRO, FLJ3, TRAP1, KIAA, COPE, CG1I, UQCRH, NOT56L, H2AV, PLXNB2, DJ-1, REA, UBC, MACMARCKS, COBRA1, RAD23A, UQCR, GPR56, RERE, KIFC3, MCL1, PPP1R11, QP-C, HBOA, TUBB4, KIAA, MGC, HLA-DRB4, RFP, DNAJB1, RNPS1, CGB7, TIMM44, SIAHBP1, BART1, AFG3L2, MFN2, RUVBL2, DIAPH1, MLC-B, MGC, FLJ2, TMSB10, HCDI, PTOV1, KIAA, KIAA, RE2, NEDD8, CCT7, SARS, PFN1, SDC3, RPL35, K-ALPHA-1, NXF1, SLC6A7, AGPAT1, MRPL23, POLR2F, SEC61B, RPS14, HNRPH1, TALDO1, ARMET, DXYSE, PTDSS1, FOXMI, RAC1, VAMP1, KIAA, ABL1, RAN, A2LP, RTN4, ZFPL1, JTB, NUDT3, CPNE6, PGPL, TIP-1, FCGR2A, JUND, NFKBIA, LAMP1, KIAA, MEA, BC-2, CIZ1, ASE-1, CALM1, RBPMS, RBM3, CRF, VARS2, TACC1, PIN1, LASP1, MT3, UQCRFS1, CCT3, TCFL1, SLC6A8, KARS, ISLR, CFL1, NCL, MLF2, PRPF8, TOM7, MRC2, AKR1A1, LQFBS-1, GNB2L1, MLN51, HSGP25L2G, BRMS1, APOBEC3C, UBB, CREB3, RANBP16, MRPL9, PDCD6, MDH1, JAM3, MAP2K2, REQ, YWHAQ, MGC3, FLJ4, SLC25A1, FLJ2, SARI, G2AN, PPP2R1A, SMT3H2, TSFM, HSPA5, TMEM4, RNP24, MAPK8IP1, GNB2, LYPLA2, NDUFV1, BTBD2, ANAPC5, SUI1, DDOST, PKD2, DRAP1, MYL6, WDR1, MEL, TLN1, SCAMP3, CDC2L2, RBM8A, RPL10, SDCCAG33, RPL10A, TRIM28, AATF, P-RHO-GEF, RPL13A, POLR2L, NIFIE14, XBP1, HYOU1, C9orfl6, C12orf8, FLJ7, GSK3A, MRPS12, NDUFV2, CLSTN1, DAZAP2, HSA16, DKK4, PAK4, PRKCABP, ZNF, TCEB2, GABARAP, ATPSI, SMT3H1, IDH3B, KDELR1, KIF1C, TUBGCP2, API5, ANP32B, RABAC1, HIS1, ATP5H, ACAT2, SRRM1, NACA, HINT1, ATP5G1, ALDOC and NDUFA (Eisenberg and Levanon (2003) Trends in Genetics 19, 362-365). Such housekeeping genes can be arrayed on microarrays and microarray analysis to measure gene expression can be performed using standard techniques well known in the art and described elsewhere herein.

Any method known in the art can be used for assessing the expression of housekeeping genes in a tumor. For example, methods for measuring protein expression levels which can be used include, but are not limited to, microarray analysis, ELISA assays, Western blotting, or any other technique for the quantitation of specific proteins. For RNA levels, examples of techniques which can be used include microarray analysis, quantitative PCR, Northern hybridization, or any other technique for the quantitation of specific nucleic acids.

In an exemplary method provided herein includes contacting a tumor sample with the virus for a period of time *in vitro* and measuring the level of expression of one or more housekeeping genes compared to level of the one ore more housekeeping genes in a second tumor sample. Decreased expression of the one or more housekeeping genes is indicative of a favorable response to tumor therapy.

### d. Expression of tumor proteins

As described herein, the level of one or more tumor proteins can be upregulated or down regulated in response to viral infection. Thus, measuring the level of expression of such protein can be a indicator of efficient viral replication in the tumor cell. Examples of such proteins are provided elsewhere herein and in the examples. In an exemplary method, a tumor cell is infected with a virus and the level of gene expression in the tumor is measured compared to the level of gene expression in the tumor in the absence of the virus. An increase or decrease in the level of gene expression in a tumor can be compared to the pattern of gene expression obtained when a responder is infected with a therapeutic virus versus the pattern of gene expression obtained when a non- responder is infected with a virus. Comparison of the patterns of gene expression will allow one classify the tumor as one likely to respond favorably to tumor therapy.

Any method known in the art can be used for assessing the level of gene expression in a tumor. For example, methods for measuring protein expression levels which can be used include, but are not limited to, microarray analysis, ELISA assays, Western blotting, or any other technique for the quantitation of specific proteins. For RNA levels, examples of techniques which can be used include microarray analysis, quantitative PCR, Northern hybridization, or any other technique for the quantitation of specific nucleic acids.

### 2. Methods of Assessing Whether a Subject is Likely to Respond Favorably or Poorly to Viral Therapy by Marker Expression Profiling

Methods of assessing whether a subject is likely to respond favorably or poorly to viral therapy are provided. Such methods can be used to determine whether to administer viral therapy to a subject or whether to utilize a therapeutic approach other than viral therapy. In some examples, such methods can be used to determine the type of therapeutic virus to administer to a subject.

In some examples, a marker profile of a cell contacted with a therapeutic virus can be used to assess whether a subject is likely to respond favorably or poorly to viral therapy. In some examples, a marker profile of a cell not contacted with a therapeutic virus can be used to assess whether a subject is likely to respond favorably or poorly to viral therapy. A marker profile can be obtained by determining whether the level of expression of a plurality of markers indicative of a favorable or poor response to viral therapy is altered when a biological sample, such as a tumor sample, from the subject is contacted with a therapeutic virus. Markers can include markers whose expression is altered in cells that are known to respond favorably to viral therapy; markers whose expression is altered in cells that are known to respond poorly to viral therapy; and markers whose expression is known to remain substantially the same in cells which respond favorably or poorly to viral therapy. In some examples, the cells which respond favorably to viral therapy are cells which permit replication of the therapeutic virus following infection. In some examples, the cells which respond poorly to viral therapy are cells in which the therapeutic virus replicates poorly. In some examples, markers can include markers for which an increased level of expression is indicative of a favorable or poor response to viral therapy, markers for which a decreased level of expression is indicative of a favorable or poor response to viral therapy or markers for which a substantially unchanged level of expression is indicative of a favorable or poor response to viral therapy. For example, some of the markers can be one or more of those listed in Table 1, Table 2, and Table 3.

**Table 1**

| **Markers For Which an Increased Expression Level is Indicative of a Favorable Response to Viral Therapy** | |
|---|---|
| | **SEQ ID No.** |
| IL-18 (Interleukin-18) | 407 |
| MCP-5 (Monocyte Chemoattractant Protein-5; CCL12) | 52 |
| IL-11 (Interleukin-11) | 35 |
| MCP-1 (Monocyte Chemoattractant Protein-1) | 50, 135 |
| MPO (Myeloperoxidase) | 53, 136 |
| Apo A1 (Apolipoprotein A1) | 3, 72 |
| TIMP-1 (Tissue Inhibitor of Metalloproteinase Type-1) | 62, 449 |
| CRP (C Reactive Protein) | 5, 77 |
| Fibrinogen | 13, 97 |
| MMP-9 (Matrix Metalloproteinase-9) | 49, 134 |
| Eotaxin (CCL11) | 10, 88 |
| GCP-2 (Granulocyte Chemotactic Protein-2; CXCL6) | 17 |
| IL-6 (Interleukin-6) | 32, 117 |
| Tissue Factor (TF) | 63, 151 |
| SAP (Serum Amyloid P) | 58, 143 |
| FGF-basic (Fibroblast Growth Factor-basic) | 15, 100 |
| MCP-3 (Monocyte Chemoattractant Protein-3; CCL7) | 51 |
| IP-10 (CXCL 10) | 485 |
| MIP-2 | 47 |
| Thrombopoetin | 61, 147 |
| Cancer antigen 125 | 80 |
| CD40 | 6, 82 |
| CD40 ligand | 7, 83 |
| ENA-78 | 90 |
| Ferritin | 95 |
| IL-12p40 | 121 |
| IL-12p70 | 36, |
| IL-16 | 125 |
| MMP-2 | 132 |
| PAI-1 | 138 |
| TNF RII | 155 |
| TNF-beta | 154 |
| VCAM-1 | 65, 156 |

**Table 2**

| **Cell Markers For Which an Decreased Expression Level is Indicative of a Favorable Response to Viral Therapy** | |
|---|---|
| | **SEQ ID No.** |
| MIP-1 beta (Macrophage Inflammatory Protein-1 beta) | 45, 131 |
| MDC (Macrophage-Derived Chemokine; CCL22) | 43, 128 |
| MIP-1alpha (Macrophage Inflammatory Protein-1alpha; CCL3) | 44, 130 |
| KC/GROalpha (Melanoma Growth Stimulatory Activity Protein) | 39 |
| VEGF (Vascular Endothelial Cell Growth Factor) | 66, 157 |
| Endothelin-1 | 8, 87 |
| MIP-3 beta (Macrophage Inflammatory Protein-3 beta; Exodus-3 or ELC) | 48 |
| Beta-2 microglobulin | 75 |
| IL-5 (Interleukin-5) | 31, 116 |
| IL-1 alpha (Interleukin-1 alpha) | 26, 110 |
| EGF (Epidermal Growth Factor) | 89 |
| Lymphotactin (XCL1) | 41, 128 |
| GM-CSF (Granulocyte Macrophage-Colony Stimulating Factor) | 18, 103 |
| MIP-1gamma (Macrophage Inflammatory Protein-1gamma; CCL4) | 46 |
| IL-1beta (Interleukin-1 beta) | 27, 111 |
| Brain-derived neutrophic factor | 76 |
| Cancer antigen 19=9 | 79 |
| Carcinoembryonic antigen | 81 |
| C reactive protein | 5, 77 |
| EGF | 89 |
| Fatty acid binding protein | 94 |
| Factor VII | 12, 93 |
| Growth hormone | 104 |
| IL-1 alpha | 26, 110 |
| IL-1 beta | 27, 111 |
| IL-1 ra | 112 |
| IL-7 | 33, 118 |
| IL-8 | 119 |
| MDC | 43, 129 |
| Prostatic acid phosphatase | 141 |
| Prostate specific antigen, free | 140 |
| Stem cell factor | 60, 146 |
| Tissue factor | 63, 151 |
| TNF-alpha | 64,153 |
| VEGF | 66, 157 |
| Von Willebrand factor | 67, 158 |

**Table 3**

| **Tumor Cell Markers For Which the Expression Level is Substantially Unchanged in Cells Which Respond Favorably to Viral Therapy** | |
|---|---|
| | **SEQ ID No.** |
| IgA (Immunoglobulin A) | 486 |
| Haptoglobin | 23, 105 |
| MIP-2 (Macrophage Inflammatory Protein-2) | 47, 132 |
| IL-17 (Interleukin-17) | 38 |
| SGOT (Serum Glutamic-Oxaloacetic Transaminase) | 59, 144 |
| IP-10 (Inducible Protein-10) | 485 |
| IL-10 | 34, 120 |
| FGF-9 (Fibroblast Growth Factor-9) | 16 |
| M-CSF (Macrophage-Colony Stimulating Factor) | 42 |
| IL-4 (Interleukin-4) | 30, 115 |
| IL-3 (interleukin-3) | 29, 114 |
| TPO (Thrombopoietin) | 61, 147 |
| SCF (Stem Cell Factor) | 60, 146 |
| LIF (Leukemia Inhibitory Factor) | 40 |
| IL-2 (Interleukin-2) | 28, 113 |
| VCAM-1 (Vascular Cell Adhesion Molecule-1; CD106) | 65, 156 |
| TNF alpha | 64, 153 |
| OSM (Oncostatin M) | 55 |

In some examples, the expression profile for a tumor that is responsive to tumor therapy can be compared to the expression profile for a tumor that is non-responsive to viral therapy in the absence of any viral treatment. As described herein and in the examples, tumors that are non-responsive to tumor therapy have increased expression of markers that are predictive of whether the tumor will respond to viral therapy. Such markers can be employed to assess whether a particular tumor will be responsive to tumor therapy. Listed in Table 4 are marker where increased expression is indicative of a poor response to therapy. Such markers include but are not limited to Beta-2 Microglobulin, Brain-Derived Neurotrophic Factor, Cancer Antigen 19-9, Carcinoembryonic Antigen, C Reactive Protein, EGF, Fatty Acid Binding Protein, Factor VII, Growth Hormone, GM-CSF, IL-1 alpha, IL-1 beta, IL-1 ra, IL-7, IL-8, Prostatic Acid Phosphatase, Prostate Specific Antigen, Stem Cell Factor, TNF-alpha, and VEGF.

**Table 4**

| **Tumor Markers For Which an Increased Expression Level is Indicative of a Poor Response to Viral Therapy** | |
|---|---|
| | **SEQ ID No.** |
| Beta-2 Microqlobulin | 75 |
| Brain-Derived Neurotrophic Factor | 76 |
| Cancer Antigen 19-9 | 79 |
| Carcinoembryonic Antigen | 81 |
| C Reactive Protein | 5, 77 |
| EGF | 89 |
| Fatty Acid Binding Protein | 94 |
| Factor VII | 12, 93 |
| Growth Hormone | 104 |
| GM-CSF | 18, 103 |
| IL-1alpha | 26, 110 |
| Il-1beta | 27, 111 |
| IL-1ra | 112 |
| IL-7 | 33, 118 |
| IL-8 | 119 |
| Prostatic Acid Phosphatase | 141 |
| Prostate Specific Antigen | 140 |
| Stem Cell Factor | 60, 146 |
| TNF-alpha | 64, 153 |
| VEGF | 66, 157 |

Methods to determine whether a subject is likely to have a favorable or a poor response to viral therapy can include one or more steps. In some examples, a biological sample, such as a tumor sample, from the subject is contacted with a therapeutic virus. In such examples, the level of expression of at least one marker in the biological sample, such as a tumor sample, contacted with the virus is assessed to determine whether the level of expression is altered in response to the therapeutic virus. If the expression level of the at least one marker is indicative of a favorable response to viral therapy, viral therapy with the therapeutic virus can be performed on the subject. If the expression level of the at least one marker is indicative of a poor response to viral therapy, a therapeutic approach other than viral therapy can be employed for the subject. In some examples, such methods can include obtaining a biological sample from the subject, for example, a biopsy of a tumor; measuring the level of expression of at least one marker in a first biological sample; measuring the level of expression of the same at least one marker in a second biological sample contacted with a therapeutic virus; and determining whether the level of expression of the at least one marker has increased, decreased or remained substantially the same in response to contacting the biological sample with the therapeutic virus. Alternatively, for this method and any of the methods described herein, rather than comparing the level of expression in contacted or non-contacted samples, the level of expression in the contacted biological sample or cells can be compared to a previously determined expression level which has been shown to be an accurate baseline expression level for uncontacted biological samples or cells.

In some examples, the at least one marker can be selected from the markers listed in Table 1, Table 2 and Table 3. In some examples, the at least one marker encompasses a plurality of markers selected from the markers listed in Table 1, Table 2, or Table 3. In some examples the expression level of at least 1, at least 5, at least 10, at least 15, at least 20 markers, or more than 20 markers selected from the markers listed in Table 1, Table 2 and Table 3 can be determined. In some examples, the expression levels of all the markers of Table 1, Table 2, and Table 3 can be determined.

The at least one marker can be a marker identified by methods described herein. In some examples, the marker can be a marker for which the level of expression is indicative of a favorable or poor response to viral therapy. In some examples, the marker can be a marker for which the level of expression is associated with good or poor replication of the virus in a biological sample.

In some examples, a single biological sample, such as a tumor sample, is obtained and divided into two test samples. One test sample is not contacted with the virus, while the other test sample is contacted with the virus. The level of expression of the marker in the two portions is compared. In other examples, the second biological sample, such as a second tumor sample, can be a portion of the first biological sample or from the same source as the first biological sample.

Methods to determine whether a subject is likely to have a favorable or a poor response to viral therapy can include culturing a biological sample, such as a tumor sample, contacted with a therapeutic virus, prior to measuring the level of expression of at least one marker. In some examples, the biological sample, such as a tumor sample, can be cultured *in vitro* according to methods known in the art. Alternatively, the biological sample, such as a tumor sample, can be cultured *in vivo.* In such methods, the biological sample, such as a tumor sample, can be implanted subcutaneously into an organism, such as a nude mouse. Where the biological sample is cultured *in vivo,* the level of expression can be measured for markers of the host organism, and for markers of the biological sample.

The biological sample, such as a tumor sample, contacted with the therapeutic virus can be cultured for a period of time sufficient to detect a response to the virus. In some examples, the period of time can be determined by the sensitivity of the method used to measure the level of expression of at least one marker. For example, the biological sample, such as a tumor sample, contacted with the therapeutic virus can be cultured for about 30 minutes, about 1 hour, about 6 hours, about 12 hours, about 1 day, about 2 days, about 3 days, about 4 days, about 5 days, about 6 days, about 7 days, about 8 days, about 9 days, about 10 days, about 11 days, about 12 days, about 13 days, about 14 days, about 2 weeks, about 3 weeks, about 4 weeks, or about 1 month.

The measurement of the level of expression of at least one marker can be carried out using methods well known in the art. Examples of methods for measuring protein expression levels which can be used include, but are not limited to, microarray analysis, ELISA assays, Western blotting, or any other technique for the quantitation of specific proteins. For RNA levels, examples of techniques which can be used include microarray analysis, quantitative PCR, Northern hybridization, or any other technique for the quantitation of specific nucleic acids.

In some examples of the methods for assessing whether a subject is likely to respond favorably or poorly to viral therapy described herein, the step of determining whether the level expression of the at least one marker in a biological sample, such as a tumor sample, contacted with a therapeutic virus has decreased, increased, or remained substantially the same, as compared to the expression of the same at least one marker in a non-contacted biological sample can be performed by comparing quantitative or semi-quantitative results obtained from the determining step. In some examples, a difference in expression of the same marker between the contacted and non-contacted biological samples of about less than 2-fold, about 2-fold, about 3-fold, about 4-fold, about 5-fold, about 6-fold, about 7-fold, about 8-fold, about 9-fold, about 10-fold, about 20-fold, about 30-fold, about 40-fold, about 50-fold, about 60-fold, about 70-fold, about 80-fold, about 90-fold, about 100-fold or greater than about 100-fold is indicative of a favorable or poor response to viral therapy. For markers for which a substantially unchanged level of expression is indicative of a favorable or poor response to viral therapy, the difference in expression of the same marker between the contacted and non-contacted samples can be less than about 2-fold, less than about 1.5 fold, or less than about 1.

In examples where the at least one marker is selected from Table 1, an increase in expression of the selected marker in a contacted biological sample, such as a tumor sample, as compared to expression of the same marker in a non-contacted biological sample can indicate that the subject is likely to respond favorably to viral therapy. Conversely, no increase can indicate that the subject is likely to have a poor response to viral therapy. In examples where the at least one marker is selected from Table 2, a decrease in expression of the selected marker in a contacted biological sample as compared to expression of the same marker in a non-contacted biological sample can indicate that the subject is likely to respond favorably to viral therapy. Conversely, no decrease can indicate that the subject is likely to have a poor response to viral therapy. In examples where the at least one marker is selected from Table 3, no substantial change in the level of expression of the selected marker in a contacted biological sample as compared to expression of the same marker in a non-contacted biological sample can indicate that the subject is likely to respond favorably to viral therapy. In some examples where the at least one marker is selected from Table 3, evidence of a subject's likely favorable or poor response to viral therapy can be corroborated by measuring the level of expression of markers from Table 1 and Table 2.

Any therapeutic virus described herein can be used to assess whether a subject is likely to respond favorably or poorly to viral therapy. In some examples, a subject can be screened with a plurality of viruses designed for viral therapy to determine which virus can be used in viral therapy. For example, where a subject responds poorly to a first therapeutic virus, at least a second virus can be used to determine whether a subject is likely to respond favorably or poorly to viral therapy containing the at least a second virus. In such examples, a biological sample, such as a tumor sample, from the subject is contacted with the at least a second virus and the level of expression of at least one marker is determined. If the level of expression of the at least one marker is indicative of a favorable response, viral therapy can be performed using the at least a second virus.

In some examples, methods to determine whether a subject is likely to respond to favorably or poorly to viral can include determining the level of expression of at least one marker in a biological sample, such as a tumor sample, not contacted with a therapeutic virus. In such examples, a biological sample, for example, a biopsy (the following is not limited to a biopsy samples only), can be obtained from a subject and the level of expression of at least one marker is determined. In some examples, the at least one marker can be selected from among markers listed in Table 1, Table 2 and Table 3. In some examples, the at least one marker encompasses a plurality of markers selected from among the markers listed in Table 1, Table 2, and Table 3. In some examples, the expression of at least 1, at least 5, at least 10, at least 15, at least 20, or more than 20 markers selected from among the markers listed in Table 1, Table 2 and Table 3 can be determined. In some examples, the expression level of all the markers in Table 1, Table 2, and Table 3 can be determined. In other examples, the at least one marker can be a marker identified by methods described herein.

In some examples, the amount or pattern of expression of the at least one marker or a plurality of markers in a biological sample, such as a tumor sample, which has not been contacted with a therapeutic virus is assessed to determine whether the amount or pattern resembles the amount or pattern characteristic of biological samples which respond favorably to viral therapy or biological samples which respond poorly to viral therapy. For example, if the amount or pattern resembles the amount or pattern characteristic of biological samples which respond favorably to viral therapy then the subject from which the biological sample was obtained is likely to respond favorably to viral therapy and therapy with the therapeutic virus can be initiated. In another example, if the amount or pattern resembles the amount or pattern characteristic of biological samples which respond poorly to viral therapy then the subject from which the biological sample was obtained is likely to respond poorly to viral therapy and a therapeutic approach other than therapy with the therapeutic virus can be employed.

In some examples, the amount or pattern of expression of the at least one marker or plurality of markers in the biological sample, such as a tumor sample, can be compared to the amount or pattern of expression which has been previously determined to be characteristic of biological samples which respond favorably or poorly to viral therapy. For example, in some examples, the amount of expression of the at least one marker or plurality of markers can be determined relative to a standard such as the size of the tumor or other biological tissue in the biological sample, the volume of the biological sample, the weight of the biological sample, the amount of total protein in the biological sample, the total amount of nucleic acid in the biological sample, the amount of DNA in the biological sample, the amount of RNA in the biological sample or any other appropriate standard. For example, if a concentration of 50 ng/ml or more of a particular marker per 150 mg of tumor has been shown to be characteristic of tumors which respond favorably to viral therapy and the tumor biopsy obtained from the subject has an expression level of 75 ng/ml per 150 mg of tumor, it is likely that the subject will respond favorably to viral therapy and viral therapy can be initiated. The same type of analysis can be performed using the concentrations of a plurality of markers or by measuring the concentrations of the markers relative to any of the standards described above.

The pattern of expression of one or more markers in the biological sample, such as a tumor sample, also can be compared to the pattern of expression of one or more markers which has been previously determined to be characteristic of biological samples which respond favorably or poorly to viral therapy. For example, if it has been determined that biological samples which respond favorably to viral therapy exhibit a high level of expression of a first marker, a low level of expression of a second marker, and an intermediate level of expression of a third marker and this same pattern of expression is observed in a biological sample obtained from the subject, the subject is likely to respond favorably to viral therapy and therapy with the therapeutic virus can be initiated. In another example, if it has been determined that biological samples which respond poorly to viral therapy exhibit a low level of expression of a first marker, a low level of expression of a second marker, and a high level of expression of a third marker and this same pattern of expression is observed in a biological sample obtained from the subject, the subject is likely to respond poorly to viral therapy and a therapeutic approach other than therapy with the therapeutic virus can be initiated.

### C. THERAPEUTIC VIRUSES

Described herein are viruses designed for viral therapy (*i.e.* therapeutic viruses). Viruses described in U.S. Patent Publication Nos. 2005/0031643, 2004/0234455 and 2004/0213741, can be used in conjunction with examples. In particular, U.S. Patent Publication Nos. 2005/0031643, 2004/0234455 and 2004/0213741 describe desirable characteristics of viruses designed for viral therapy, such as, attenuated pathogenicity, reduced toxicity, preferential accumulation in certain cells and tissues, such as a tumor, ability to activate an immune response against tumor cells, immunogenicity, replication competence, expression of exogenous proteins, and any combination of the foregoing characteristics.

In some examples, viruses designed for viral therapy can include recombinant vaccinia viruses. A variety of vaccinia virus strains are available, including Western Reserve (WR), Copenhagen, Tashkent, Tian Tan, Lister, Wyeth, IHD-J, and IHD-W, Brighton, Ankara, MVA, Dairen I, L-IPV, LC16M8, LC16MO, LIVP, WR 65-16, Connaught, New York City Board of Health. In further examples, a vaccinia virus can be a member of the Lister strain. In even further examples, the Lister virus can be an attenuated Lister strain, such as the LIVP (Lister virus from the Institute for Research on Virus Preparations, Moscow, Russia) strain.

In particular examples, a therapeutic virus can include the GLV-1h68 virus (Zhang et al. (2007) Cancer Research 67:10038-10046). The GLV-1h68 virus is a replication-competent recombinant vaccinia virus that was constructed by inserting three expression cassettes (encoding *Renilla* luciferase-*Aequorea* green fluorescent protein fusion, β-galactosidase, and β-glucuronidase) into the F14.5L, J2R (encoding thymidine kinase) and *A56R* (encoding hemagglutinin) loci of the viral genome, respectively. GLV-1h68 has an enhanced tumor targeting specificity and much reduced toxicity compared with its parental LIVP strains.

In some examples, a therapeutic virus can be immunogenic where the virus can induce a host immune response against the virus. The immune response can be activated in response to viral antigens or can be activated as a result of viral-infection induced cytokine or chemokine production. In some examples, an immune response against a therapeutic virus can result in killing of a target tissue or target cell. In some examples, the immune response can result in the production of antibodies against tumor antigens. In some examples, an immune response can result in cell killing through a bystander effect, for example, where uninfected cells in close proximity to cells infected by the virus are killed as infected cells are killed.

In some examples, a therapeutic virus can be an attenuated virus which is replication competent. Such viruses can have a decreased capacity to cause disease in a host and accumulate in targeted tissues or cells. Methods to attenuate a virus can include reducing the replication competence of the virus. For example, in the vaccinia virus one or more genes selected from among the thymidine kinase gene, the hemaglutinin gene and the F14.5L gene can be modified so as to attenuate the virus. In some examples, the modification reduces the ability of the virus to replicate. In some examples, the modification inactivates the protein encoded by the gene or results in a lack of expression of the protein encoded by the gene.

In some examples, a therapeutic virus can accumulate in any of a variety of organs, tissues or cells of the host. Accumulation can be evenly distributed over the entire host organism, or can be concentrated in one or a few organs or tissues. In certain examples, viruses can accumulate in targeted tissues, such as tumors, metastases, or cancer cells. In exemplary examples, viruses designed for viral therapy can accumulate in a targeted organ, tissue or cell at least about 2-fold greater, at least about 5-fold greater, at least about 10-fold greater, at least about 100-fold greater, at least about 1,000-fold greater, at least about 10,000-fold greater, at least about 100,000-fold greater, or at least about 1,000,000-fold greater, than the accumulation in a non-targeted organ, tissue or cell.

Methods for the generation of recombinant viruses using recombinant DNA techniques are well known in the art (*e.g*., see U.S. Pat. No. 4,769,330, 4,603,112, 4,722,848, 4,215,051, 5,110,587, 5,174,993, 5,922,576, 6,319,703, 5,719,054, 6,429,001, 6,589,531, 6,573,090, 6,800,288, 7,045,313, He et al. (1998) PNAS 95(5): 2509-2514, Racaniello et al., (1981) Science 214: 916-919, Hruby et al., (1990) Clin Micro Rev. 3:153-170).

Non-limiting examples of attenuated Lister strain LIVP viruses that can be used in any of the method provided herein or that can be modified to encode proteins provided herein include, LISP viruses described in U.S. Patent Publication Nos. 2005/0031643, 2004/0234455 and 2004/0213741 and U.S. Patent Application Serial No. 11/975,088. Exemplary viruses contained therein that can be modified as described here include viruses which have one or more expression cassettes removed from GLV-1h68 and replaced with a heterologous non-coding DNA molecule (*e.g*., GLV-1h70, GLV-1h71, GLV-1h72, GLV-1h73, GLV-1h74, GLV-1h85, and GLV-1h86). GLV-1h70 contains (P_{SEL})*Ruc*-*GFP* inserted into the *F14.5L* gene locus, (P_{SEL})*rTrƒR* and (P_{7.5k})*LacZ* inserted into the *TK* gene locus, and a non-coding DNA molecule inserted into the *HA* gene locus in place of (P₁₁₁ₖ)*gusA.* GLV-1h71 contains a non-coding DNA molecule inserted into the *F14.5L* gene locus in place of (P_{SEL})*Ruc-GFP*, (P_{SEL})*rTrƒR* and (P_{7.5k})*LacZ* inserted into the *TK* gene locus, and (P₁₁ₖ)*gusA* inserted into the *HA* gene locus. GLV-1h72 contains *(P_{SEL})Ruc-GFP* inserted into the *F14.5L* gene locus, a non-coding DNA molecule inserted into the *TK* gene locus in place of (P_{SEL})*rTrƒR* and (P_{7.5k})*LacZ*, and P11k*gusaA* inserted into the *HA* gene locus. GLV-1h73 contains a non-coding DNA molecule inserted into the *F14.5L* gene locus in place of (P_{SEL})*Ruc-GFP,* (P_{SEL})*rTrƒR* and (P_{7.5k})*LacZ* inserted into the *TK* gene locus, and a non-coding DNA molecule inserted into the *HA* gene locus in place of (P₁₁ₖ)*gusA.* GLV-1h74 contains a non-coding DNA molecule inserted into the *F14.5L* gene locus in place of (P_{SEL})*Ruc-GFP,* a non-coding DNA molecule inserted into the *TK* gene locus in place of (P_{SEL)}*rTrƒR* and (P_{7.Sk})*LacZ*, and a non-coding DNA molecule inserted into the *HA* gene locus in place of (P₁₁ₖ)*gusA.* GLV-1h85 contains a non-coding DNA molecule inserted into the *F14.5L* gene locus in place of *(P_{SEL})Ruc-GFP,* a non-coding DNA molecule inserted into the *TK* gene locus in place of (P_{SEL})*rTrƒR* and (P_{7.5k})*LacZ*, and (P₁₁ₖ)*gusA* inserted into the *HA* gene locus. GLV-1h86 contains (P_{SEL})*Ruc-GFP* inserted into the *F14.5L* gene locus, a non-coding DNA molecule inserted into the *TK* gene locus in place of (P_{SEL})*rTrƒR* and (P_{7.5k})*LacZ*, and a non-coding DNA molecule inserted into the HA gene locus in place of (P₁₁ₖ)*gusA .* Other exemplary viruses include, but are not limited to, LIVP viruses that express one or more therapeutic gene products, such as angiogenesis inhibitors (*e.g.*, GLV-1h81, which contains DNA encoding the plasminogen K5 domain under the control of the vaccinia synthetic early-late promoter in place of the *gusA* expression cassette in GLV-1h68; GLV-1h104, GLV-1h105 and GLV-1h106, which contain DNA encoding a truncated human tissue factor fused to the αᵥβ₃-integrin RGD binding motif (tTF-RGD) under the control of a vaccinia synthetic early promoter, vaccinia synthetic early/late promoter or vaccinia synthetic late promoter, respectively, in place of the *LacZ*/*rTFr* expression cassette at the *TK* locus of GLV-1h68; GLV-1h107, GLV-1h108 and GLV-1h109, which contains DNA encoding an anti-VEGF single chain antibody G6 under the control of a vaccinia synthetic early promoter, vaccinia synthetic early/late promoter or vaccinia synthetic late promoter, respectively, in place of the *LacZ*/*rTFr* expression cassette at the *TK* locus of GLV-1h68) and proteins for tumor growth suppression (*e.g*., GLV-1h90, GLV-1h91 and GLV-1h92, which express a fusion protein containing an IL-6 fused to an IL-6 receptor (sIL-6R/IL-6) under the control of a vaccinia synthetic early promoter, vaccinia synthetic early/late promoter or vaccinia synthetic late promoter, respectively, in place of the *gusA* expression cassette in GLV-1h68; and GLV-1h96, GLV-1h97 and GLV-1h98, which express IL-24 (melanoma differentiation gene, mda-7) under the control of a vaccinia synthetic early promoter, vaccinia synthetic early/late promoter or vaccinia synthetic late promoter, respectively, in place of the *Ruc-GFP* fusion gene expression cassette at the *F14.5L* locus of GLV-1h68). Additional therapeutic gene products that can be engineered in the viruses provided herein also are described elsewhere herein.

### 1. Modifications of Therapeutic Viruses

In some examples, therapeutic viruses can contain a genetic modification. Such modifications can include truncations, insertions, deletions and mutations to the viral genome. In some examples, modifications can result in a change of viral characteristics, for example, immunogenicity, pathogenicity, toxicity, ability to lyse cells or cause cell death, and ability to preferentially accumulate in particular cells.

In some examples, a virus can be modified to produce a genetic variant using techniques well known in the art. Such techniques for modifying vaccinia strains by genetic engineering are well established (Moss (1993) Curr. Opin. Genet. Dev. 3:86-90; Broder and Earl (1999) Mol. Biotechnol. 13, 223-245; Timiryasova et al. (2001) Biotechniques 31: 534-540), and described in U.S. Patent Application No. 11/238,025. In some examples, genetic variants can be obtained by general methods such as mutagenesis and passage in cell or tissue culture and selection of desired properties, as exemplified for respiratory syncytial virus in Murphy et al. (1994) Virus Res. 32:13-26. In some examples, genetic variants can be obtained by methods in which nucleic acid residues of the virus are added, removed or modified relative to the wild type. Any of a variety of known mutagenic methods can be used, including recombination-based methods, restriction endonuclease-based methods, and PCR-based methods. Mutagenic methods can be directed against particular nucleotide sequences such as genes, or can be random, where selection methods based on desired characteristics can be used to select mutated viruses. Any of a variety of viral modifications can be made, according to the selected virus and the particular known modifications of the selected virus.

In certain examples, any of a variety of insertions, mutations or deletions of the vaccinia viral genome can be used herein. Such modifications can include insertions, mutations or deletions of: the thymidine kinase (TK) gene, the hemagglutinin (HA) gene, the VGF gene (U.S. Patent Publication No. 20030031681); a hemorrhagic region or an A type inclusion body region (U.S. Patent No. 6,596,279); Hind III F, F13L, or Hind III M (as taught in U.S. Patent No. 6,548,068); A33R, A34R, A36R or B5R genes (Katz et al., J. Virology 77:12266-12275 (2003); SalF7L (Moore et al., EMBO J. 1992 11:1973-1980); N1L (Kotwal et al., Virology 1989 171:579-587); Ml lambda (Child et al., Virology. 1990 174:625-629); HR, HindIII-MK, HindIII-MKF, HindIII-CNM, RR, or BaniF (Lee et al., J. Virol. 1992 66:2617-2630); C21L (Isaacs et al., Proc Natl Acad Sci USA. 1992 89:628-632); or F3 (F14.5L) (U.S. Patent Application No. 11/238,025).

### 2. Viruses Encoding a Marker Protein that is Increased in Cells that Respond Favorable to Tumor Therapy

Some examples relate to viruses designed for gene therapy which encode a marker protein whose level of expression is increased in cells which respond favorably to viral therapy. Other examples relate to viruses designed for gene therapy which encode an agent which reduces the level of expression of a marker protein whose level of expression is decreased in cells which respond favorably to viral therapy. Each of the foregoing viruses can, in some examples, be a modified virus such as those described above.

In some examples, a therapeutic virus can contain a heterologous nucleic acid encoding a protein whose levels are increased in cells that respond favorably to viral therapy. In certain examples, the heterologous nucleic acid is operatively linked to regulatory elements. Such regulatory elements can include promoters, enhancers, or terminator sequences. Promoter sequences can be constitutive or inducible. Methods to increase the level of expression of a particular protein can include providing a therapeutic virus expressing the particular protein to a cell.

The methods can include providing a therapeutic virus expressing a transactivator to a cell, where the transactivator can increase the level of expression of a particular endogenous protein in the cell. In such examples, the endogenous protein can be a protein whose level of expression is increased in cells that respond favorably to viral therapy.

In some examples, the increase in the level of expression of a particular protein in a cell contacted with a therapeutic virus containing a therapeutic agent compared with a cell not contacted with the virus can be about less than 2-fold, about 2-fold, about 3-fold, about 4-fold, about 5-fold, about 6-fold, about 7-fold, about 8-fold, about 9-fold, about 10-fold, about 20-fold, about 30-fold, about 40-fold, about 50-fold, about 60-fold, about 70-fold, about 80-fold, about 90-fold, about 100-fold or greater than about 100-fold.

In some examples, the virus can encode one or more proteins whose level of expression is increased in cells that respond favorably to viral therapy. Such proteins can include, but are not limited to, the proteins listed in Table 1. In some examples, the virus can encode one or more proteins selected from among IL-18 (Interleukin-18), MCP-5 (Monocyte Chemoattractant Protein-5; CCL12), IL-11 (Interleukin-11), MCP-1 (Monocyte Chemoattractant Protein-1), MPO (Myeloperoxidase), Apo A1 (Apolipoprotein A1), TIMP-1 (Tissue Inhibitor of Metalloproteinase Type-1), CRP (C Reactive Protein), Fibrinogen, MMP-9 (Matrix Metalloproteinase-9), Eotaxin (CCL11), GCP-2 (Granulocyte Chemotactic Protein-2, CXCL6) IL-6 (Interleukin-6), Tissue Factor (TF), SAP (Serum Amyloid P), FGF-basic (Fibroblast Growth Factor-basic), MCP-3 (Monocyte Chemoattractant Protein-3, CCL7), IP-10 (CXCL), MIP-2, and Thrombopoetin.

Among the viruses described herein are viruses encoding cytokines, such as chemokines, including members of the C-X-C and C-C chemokine families. The chemokine-encoding viruses include, but are not limited to, viruses encoding IP-10 (e.g. mouse IP-10 having the sequence of amino acids set forth in SEQ ID NO: 24, and human IP-10 having the sequence of amino acids set forth in SEQ ID NO: 485), MCP-1 (e.g. mouse MCP-1 having the sequence of amino acids set forth in SEQ ID NO: 50, and human MCP-1, having the sequence of amino acids set forth in SEQ ID NO: 135); RANTES (see SEQ ID NO: 142 (human); SEQ ID NO: 57 (mouse); MIP1-alpha (see SEQ ID NO: 130 (human); SEQ ID NO: 44 (mouse)); eotaxin (see SEQ ID NO: 88 (human); SEQ ID NO: 10 (mouse)); and MIP1-beta (see SEQ ID NO: 131 (human); SEQ ID NO: 45 (mouse)). These viruses can be used in treatment of subjects, such as human patients. For example, the viruses can be formulated and administered to a subject for treating tumors, *e.g.* by promoting anti-tumor immunity, preventing angiogenesis, preventing tumor growth, and other anti-tumor activities. A plurality of immunostimulatory cytokines promote anti-cancer immune responses and/or inhibit growth of tumors, for example, by preventing angiogenesis (reviewed in Oppenhiem et al., Clin. Cancer Res. 3, 2682-2686 (1997)).

### a. IP-10 encoding viruses

Among the described viruses are viruses encoding interferon-gamma-induced protein (IP-10; also known as Chemokine (C-X-C motif) ligand 10 (CXCL10), 10 kDa interferon-gamma-induced protein, C7, crg-2, Gamma-IP 10 (γ-IP10), gIP-10, IFI10, INP10, mob-1, SCYB10, and Small inducible cytokine B10 precursor), including viruses encoding mouse IP-10 (mIP-10, such as mIP-10 having the sequence of amino acids set forth in SEQ ID NO: 24 and mIP-10 encoded by the sequence of nucleic acids set forth in SEQ ID NO: 397) and viruses encoding human IP-10 (such as IP-10 having the sequence of amino acids set forth in SEQ ID NO: 485, and IP-10 encoded by the sequence of nucleic acids set forth in SEQ ID NO: 484). Exemplary IP-10 encoding viruses are described in Example 21, below. IP-10, a member of the C-X-C chemokine family, is a small cytokine (10 kDa) secreted by monocytes, endothelial cells, fibroblasts and other cells in response to IF-γ production. IP-10 binds to chemokine receptor CXCR3, acting as chemoattractant for a plurality of immune cells, including macrophages, monocytes, T cells, NK cells, and dendritic cells, and promoting cell adhesion (Luster et al., Nature 315: 672-676 (1985); Dufour et al., J. Immunol. 168: 3195-3204 (2002).

The IP-10 encoding viruses can be used in treatment of subjects, such as human patients. For example, the viruses can be administered to a subject for treating tumors, *e.g*., by promoting anti-tumor immunity, and/or blocking tumor cell proliferation, tumor growth and angiogenesis. C-X-C chemokines, including IP-10, have been shown to have anti-tumor activities, including suppression of tumor growth and angiogenesis and promotion of anti-tumor immunity (Angiolillo et al., J. Exp. Med 182 155-162 (1995); Oppenhiem et al., Clin. Cancer Res. 3, 2682-2686 (1997)). For example, IP-10 reportedly inhibited bone marrow colony formation and angiogenesis; transfection of IP-10 into murine tumor cells promoted anti-tumor immunity (Luster et al., J. Exp. Med. 178, 1057-1065 (2003)).

### b. MCP-1 encoding viruses

Also among the viruses described herein are viruses encoding monocyte chemoattractant protein-1 (MCP-1, also known as chemokine (C-C motif) ligand 2 (CCL2)), including mouse MCP-1 (such as MCP-1 having the amino acid sequence set forth in SEQ ID NO: 50) and human MCP-1 (hMCP-1, such as hMCP-1 having the sequence of amino acids set forth in SEQ ID NO: 135 and hMCP-1 encoded by the sequence of nucleic acids set forth in SEQ ID NO: 483). Exemplary MCP-1 encoding viruses are described in Example 21, below. MCP-1 is generated *in vivo* from a protein precursor by cleavage of a 23 amino acid signal peptide, to yield the mature MCP-1 protein, which is 76 amino acids in length, and approximately 13 kilodaltons (kDa). MCP-1 is a C-C family chemokine that recruits monocytes, memory T cells and dendritic cells and binds to receptors including CCL2, CCR2 and CCR4 (Gu et al., J. Leuk Biol. 62: 577-580 (1997); Carr et al., Proc. Natl. Acad Sci. USA 91: 3652-3656 (1994).

The MCP-1-encoding viruses can be used in treatment of subjects, such as human patients. For example, the viruses can be administered to a subject for treating tumors, *e.g*. by promoting anti-tumor immunity, and/or blocking tumor cell proliferation and tumor growth. Anti-tumor effects of MCP-1 have been observed in a plurality of cancers, including colon carcinoma and renal adenocarcinoma; MCP-1 expression positively correlated with patient survival rate and immune cell infiltration and inversely correlated with tumor proliferation in pancreatic cancer (reviewed in Craig et al., Cancer Metastasis Rev 25:611-619 (2006)). MCP-1 can promote migration of monocytes/macrophages to tumors and was observed to promote monocyte-mediated inhibition of tumor growth *in vitro* (Matushima et al., J. Exp. Med. 169: 1485-1490 (1987). MCP-1 expression in tumor cells *blocked in vivo* tumor formation in nude mice (Rollins and Sunday, Mol. Cell. Biol. 11(6), 3125-3131 (1991). Other reports indicate that MCP-1 can attract tumor cells and promote tumor metastasis, *e.g.* bone metastasis, in some cancers (Oppenhiem et al., Clin. Cancer Res. 3, 2682-2686 (1997); see also Pellegrino et al., Recenti Prog. Med. 93(11): 642-654 (2002); Craig et al., Cancer Metastasis Rev 25:611-619 (2006).

### c. TIMP-1, 2, 3 encoding viruses

In some examples, the virus can encode TIMP-1. TIMP-1 has been identified herein as having an increased expression level in cells which permit a good level of replication of a therapeutic virus or which respond favorably to viral therapy. TIMP-1 is a multifunctional protein that plays contrasting roles during angiogenesis and metastasis. In some studies TIMP-1 has been reported to regulate matrix metalloproteinase activity, act as a growth stimulator and inhibit apoptosis. For example, Yamazaki *et al.* have shown that transgenic mice carrying a transgene of TIMP-1 linked to the albumin promoter (Alb) have a greatly decreased tumor incidence (Yamazaki M *et al.* (2004) 25(9):1735-46). In particular, in an initial mammary carcinogenesis study, heterozygous Alb-TIMP-1 mice had a 25% tumor incidence compared to wild-type littermates with a 83.3% tumor incidence. Further analysis of the tumors in the Alb-TIMP-1 mice showed evidence of decreased proliferative activity and inhibition of apoptosis. In another study, C57BL/6j-CBA mice overexpressing human TIMP-1 in the liver under the control of the mouse albumin promoter/enhancer were shown to have an increased tumor angiogenic response (de Lorenzo MS *et al.* (2003) 17(1):45-50). In addition to the increased tumor angiogenic response, transgenic animals also showed an early subcutaneous growth advantage compared to wild-type hybrid mice. de Lorenzo *et al.* postulated that TIMP-1 displays paradoxical effects on tumor progression and that circulating TIMP-1 can be efficient in suppressing lung colonization of melanoma cells.

In some examples, the virus can encode TIMP-2. TIMP-2 can inhibit the invasive activities of matrix metalloproteinases in malignant tumors, such as malignant gliomas. In one study, a gene encoding TIMP-2 was transferred *in vitro* to malignant glioma cells using a defective herpes simplex virus (HSV) (Hoshi M, et al., Antitumoral effects of defective herpes simplex virus-mediated transfer of tissue inhibitor of metalloproteinases-2 gene in malignant glioma U87 in vitro: consequences for anti-cancer gene therapy. Cancer Gene Ther. 2000 7(5):799-805). The defective HSV vector, dvSRaTIMP2, was engineered to express human TIMP-2 using a replication-competent temperature-sensitive HSV-tsK mutant. U87 human glioblastoma cells infected with the vector *in vitro* showed an inhibition of invasive activity.

In some examples, the virus can encode TIMP-3. In experiments where neuroblastoma and malignant peripheral nerve sheath (MPNS) tumor xenografts were infected with an oncolytic virus expressing human TIMP-3, an enhancement of antitumor efficacy has been observed (Mahller YY, et al (2008) Cancer Res. 68(4):1170-9). The antitumor efficacy of oncolytic herpes simplex viruses (oHSV) expressing either human TIMP-3, or luciferase was evaluated in infected neuroblastoma and malignant peripheral nerve sheath tumor (MPNST) xenografts. Cells infected with the virus expressing TIMP-3 showed increased cytotoxicity and reduced metalloproteinase activity. Tumors treated with the virus expressing TIMP-3 showed delayed tumor growth, increased peak levels of infectious virus, immature collagen extracellular matrix, and reduced tumor vascular density. In addition, treatment with the virus expressing TIMP-3 reduced circulating endothelial progenitors.

### 3. Viruses an Agent which Reduces the Level of Expression of a Marker Protein

In other examples, a therapeutic virus can contain a heterologous nucleic acid encoding an agent which reduces the level of expression of a marker whose level of expression is decreased in cells which respond favorably to viral therapy or in cells which permit a good level of replication of a therapeutic virus. Methods to decrease the level of expression of a protein can include providing a therapeutic virus to a cell, where the virus can express a protein that inhibits the expression of the marker.

In some examples, the level of expression of a marker protein in a cell can be decreased by providing a therapeutic virus encoding a nucleic acid that reduces the level of expression of a marker whose level of expression is decreased in cells that respond favorably to viral therapy. In such methods the therapeutic agent can include an antisense nucleic acid targeted against a nucleic acid encoding the marker, small inhibitory RNA (siRNA) targeted against a nucleic acid encoding the marker, or a ribozyme targeted against a nucleic acid encoding the marker.

Methods to decrease the level of expression of a marker protein using antisense nucleic acids are well known in the art. Antisense sequences can be designed to bind to the promoter and other control regions, exons, introns or even exon-intron boundaries of a gene. Antisense RNA constructs, or DNA encoding such antisense RNAs, can be employed to inhibit gene transcription or translation or both within a host cell, either *in vitro* or *in vivo,* such as within a host animal, including a human subject. While all or part of the gene sequence can be employed in the context of antisense construction, statistically, any sequence 17 bases long should occur only once in the human genome and, therefore, suffice to specify a unique target sequence.

Methods to decrease the level of expression of a marker protein using siRNA are well known in the art. For example, the design of a siRNA can be readily determined according to the mRNA sequence encoding of a particular protein. Some methods of siRNA design and downregulation are further detailed in U.S. Patent Application Publication No. 20030198627.

Methods to decrease the level of expression of a particular protein using a ribozyme are well known in the art. Several forms of naturally-occurring and synthetic ribozymes are known, including Group I and Group II introns, RNaseP, hairpin ribozymes and hammerhead ribozymes (Lewin A S and Hauswirth W W, Trends in Molecular Medicine 7: 221-228, 2001). In some examples, ribozymes can be designed as described in int. PCT Patent Application Publication No. WO 93/23569 and PCT Patent Application Publication No. WO 94/02595. US 7,342,111 describes general methods for constructing vectors encoding ribozymes.

In some examples, the decrease in the level of expression of a marker protein in a cell contacted with a therapeutic virus encoding an agent which decreases the level of expression of the marker compared with a cell not contacted with the virus can be about less than 2-fold, about 2-fold, about 3-fold, about 4-fold, about 5-fold, about 6-fold, about 7-fold, about 8-fold, about 9-fold, about 10-fold, about 20-fold, about 30-fold, about 40-fold, about 50-fold, about 60-fold, about 70-fold, about 80-fold, about 90-fold, about 100-fold or greater than about 100-fold. In some examples, the level of expression of a marker protein in a cell contacted with a therapeutic virus containing a nucleic acid encoding an agent which decreases the level of expression of the marker protein can be zero.

In some examples, the agent can decrease the level of expression of one or more proteins whose level of expression is known to be decreased in cells that respond favorably to viral therapy. In some examples, the agent can be directed to one or more of the proteins listed in Table 2. In other examples, the virus can encode an agent which decreases the level of expression of one or more proteins selected from among MIP-1 beta (Macrophage Inflammatory Protein-1beta), MDC (Macrophage-Derived Chemokine, CCL22), MIP-1 alpha (Macrophage Inflammatory Protein-1 alpha, CCL3), KC/GROalpha (Melanoma Growth Stimulatory Activity Protein), VEGF (Vascular Endothelial Cell Growth Factor), Endothelin-1, MIP-3beta (Macrophage Inflammatory Protein-3beta, also know as Exodus-3 or ELC), RANTES (Regulation Upon Activation, Normal T-Cell Expressed and Secreted, CCL5), IL-5 (Interleukin-5), IL-1 alpha (Interleukin-1 alpha), EGF (Epidermal Growth Factor), Lymphotactin (XCL1), GM-CSF (Granulocyte Macrophage-Colony Stimulating Factor), MIP-1 gamma (Macrophage Inflammatory Protein-1 gamma, CCL4) and IL-1beta (Interleukin-1beta).

In some examples, the virus can encode an agent which alters the expression of a protein whose expression is altered in cells that respond poorly to viral therapy. For example, in some examples the virus can encode an agent which increases the level of expression of a marker for which the level of expression is decreased in cells which respond poorly to viral therapy. Alternatively, the virus can encode a marker protein whose expression is decreased in cells that respond poorly to viral therapy, thereby increasing the level of expression of the marker protein. In other examples, the virus can encode an agent which decreases the level of expression of a marker whose level of expression is increased in cells which respond poorly to viral therapy.

In some examples, the virus contain a regulatory sequence operatively linked to a nucleic acid sequence encoding a marker whose level of expression is increased in cells which respond favorably to viral therapy or cells which permit good viral replication. In other examples, the virus contains a regulatory sequence operatively linked to a nucleic acid encoding an agent which reduces the level of expression of a marker whose level of expression is decreased in cells which respond favorably to viral therapy or cells which permit good viral replication. In still further examples, the virus contains a regulatory sequence operably linked to a nucleic acid encoding a protein whose level of expression is decreased in cells which respond poorly to viral therapy or cells which permit poor viral replication. In other examples, the virus contains a regulatory sequence operatively linked to a nucleic acid encoding an agent which decreases the level of expression of a marker whose level of expression is increased in cells which respond poorly to viral therapy or cells which permit poor viral replication. Regulatory sequences can include a constitutive promoter, an inducible promoter, or an enhancer. In such examples, a regulatory sequence can include a natural or synthetic vaccinia virus promoter. In another embodiment, the regulatory sequence can contain a poxvirus promoter. In some examples, strong late promoters can be used to achieve high levels of expression of the foreign genes. Early and intermediate-stage promoters, however, can also be used. In one embodiment, the promoters contain early and late promoter elements, for example, the vaccinia virus early/late promoter p7.5, vaccinia late promoter pl 1, a synthetic early/late vaccinia pE/L promoter (Patel et al. (1988) Proc. Natl. Acad. Sci. USA 85, 9431-9435); Davison and Moss (1989) J Mol Biol 210, 749-769; Davison et al., (1990), Nucleic Acids Res. 18, 4285-4286; Chakrabarti et al. (1997) BioTechniques 23, 1094-1097. In exemplary examples, an inducible promoter system can include a chimeric transcription factor containing a progesterone receptor fused to the yeast GAL4 DNA-binding domain and to the activation domain of the herpes simplex virus protein VP16, and a synthetic promoter containing a series of GAL4 recognition sequences upstream of the adenovirus major late E1B TATA box, linked to one or more exogenous genes. In such a system, administration of RU486 to a subject can result in induction of the therapeutic agent.

### D. HOST CELLS

Described herein are host cells that contain a therapeutic virus. Such host cells can include any of a variety of mammalian, avian and insect cells and tissues that are susceptible to viruses. Examples of cells can include, for example, but not limited to, PANC-1 (pancreatic carcinoma), MIA PaCa-2 (pancreatic carcinoma), 01-101A (breast cancer), SiHa (cervical cancer), NCI-H1299 (lung carcinoma), HT-29 (colon adenocarcinoma), HeLa (cervical cancer), CCRF-CEM (leukemia), HL-60 (leukemia), P388 (leukemia), P388/ADR (leukemia), KG1a (leukemia), THP-1 (leukemia), K-562 (leukemia), MOLT-4 (leukemia), RPMI-8226 (leukemia), SR (leukemia), A549 (ATCC) (non-small cell lung cancer), EKVX (non-small cell lung cancer), HOP-62 (non-small cell lung cancer), HOP-92 (non-small cell lung cancer), NCI-H226 (non-small cell lung cancer), NCI-H23 (non-small cell lung cancer), NCI-H322M (non-small cell lung cancer), NCI-H460 (non-small cell lung cancer), NCI-H522 (non-small cell lung cancer), LXFL 529 (non-small cell lung cancer), DMS 114 (small cell lung cancer), SHP-77 (small cell lung cancer), COLO 205 (colon cancer), HCC-2998 (colon cancer), HCT-116 (colon cancer), HCT-15 (colon cancer), KM12 (colon cancer), SW-620 (colon cancer), DLD-1 (colon cancer), KM20L2 (colon cancer), SF-268 (central nervous system), SNB-78 (central nervous system), TNF 498 (central nervous system), SF-295 (central nervous system), SF-539 (central nervous system), SNB-19 (central nervous system), SNB-75 (central nervous system), U251 (central nervous system), LOX IMVI (melanoma), RPMI-7951 (melanoma), M19-MEL (melanoma), MALME-3M (melanoma), M14 (melanoma), SK-MEL-2 (melanoma), SK-MEL-28 (melanoma), SK-MEL-5 (melanoma), UACC-257 (melanoma), UACC-62 (melanoma), IGR-OVI (ovarian cancer), OVCAR-3 (ovarian cancer), OVCAR-4 (ovarian cancer), OVCAR-5 (ovarian cancer), OVCAR-8 (ovarian cancer), SK-OV-3 (ovarian cancer), 786-0 (renal cancer), A498 (renal cancer), RXF-631 (renal cancer), SN12K1 (renal cancer), ACHN (renal cancer), CAKI-1 (renal cancer), RXF 393 (renal cancer), SN12C (renal cancer), TK-10 (renal cancer), UO-31 (renal cancer), PC-3 (prostate cancer), DU-145 (prostate cancer), MCF7 (breast cancer), MDA-MB-468 (breast cancer), NCI/ADR-RES (breast cancer), MDA-MB-231 (ATCC) (breast cancer), MDA-N (breast cancer), BT-549 (breast cancer), T-47D (breast cancer), HS 578T (breast cancer), and MDA-MB-435 (breast cancer). Additional examples of tumor cells can be found in the art and are publicly available, such as for example the National Cancer Insitute Respository of Cancer cell lines. Methods of transforming these host cells and selecting for transformants are well known in the art.

The tumor cells can be from solid tumors or hematopoietic neoplasms, and from any cell lineage. For example, the tumor cells can be of epithelial origin (carcinomas), arise in the connective tissue (sarcomas), or arise from specialized cells such as melanocytes (melanomas), lymphoid cells (lymphomas), myeloid cells (myelomas), brain cells (gliomas), mesothelial cells (mesotheliomas) or any other cell type. Furthermore, the neoplastic cells can be derived from primary tumors or metastatic tumors.

### 1. Harvesting tumor cells from patient

In some examples, where primary tumor cells are assayed in the methods provided herein, the initial step in the assay involves isolation of tumor cells from a subject, such as a patient that has cancer. This can be performed before, during, or after the patient has undergone one or more rounds of radiation and/or chemotherapy treatment. When the tumor is a solid tumor, isolation of tumor cells is typically achieved by surgical biopsy. When the cancer is a hematopoietic neoplasm, tumor cells can be harvested by methods including, but not limited to, bone marrow biopsy, needle biopsy, such as of the spleen or lymph nodes, and blood sampling. Biopsy techniques that can be used to harvest tumor cells from a patient include, but are not limited to, needle biopsy, aspiration biopsy, endoscopic biopsy, incisional biopsy, excisional biopsy, punch biopsy, shave biopsy, skin biopsy, bone marrow biopsy, and the Loop Electrosurgical Excision Procedure (LEEP). Typically, a non-necrotic, sterile biopsy or specimen is obtained that is greater than 100 mg, but which can be smaller, such as less than 100 mg, 50 mg or less, 10 mg or less or 5 mg or less; or larger, such as more than 100 mg, 200 mg or more, or 500 mg or more, 1 gm or more, 2 gm or more, 3 gm or more, 4 gm or more or 5 gm or more. The sample size to be extracted for the assay can depend on a number of factors including, but not limited to, the number of assays to be performed, the health of the tissue sample, the type of cancer, and the condition of the patient. The tumor tissue is placed in a sterile vessel, such as a sterile tube or culture plate, and can be optionally immersed in an appropriate media. Typically, the tumor cells are dissociated into cell suspensions by mechanical means and/or enzymatic treatment as is well known in the art. In some examples, the cells from a tumor tissue sample can be subjected to a method to enrich for the tumor cells, such as by cell sorting *(e.g*. fluorescence activated cell sorting (FACS)).

Once harvested, the tumor cells can be used immediately, or can be stored under appropriate conditions, such as in a cryoprotectant at -196°C. In some examples, the cells are maintained or grown in appropriate media under the appropriate conditions (*e.g*., 37°C in 5% CO₂) to facilitate attachment of the cells to the surface of the culture plate and, in some instances, formation of a monolayer. Any media useful in culturing cells can be used, and media and growth conditions are well known in the art (see *e.g*., U.S. Pat. Nos. 4,423,145, 5,605,822, and 6,261,795, and *Culture of Human Tumor Cells* (2004) Eds. Pfragner and Freshney). In some examples, the culture methods used are designed to inhibit the growth of non-tumor cells, such as fibroblasts. For example, the tumor cells can be maintained in culture as multicellular particulates until a monolayer is established (U.S. Pat. No. 7,112,415), or the cells can be cultured in plates containing two layers of different percentage agar (U.S. Pat. No. 6,261,705). The tumor cells can be grown to the desired level, such as for example, a confluent monolayer, or a monolayer displaying a certain percentage confluency, such as 30% or more, 40% or more, 50% or more, 60% or more, 70% or more, 80% ore more, or 90% or more. In some examples, the cells are incubated for a short period of time, long enough to facilitate attachment to the culture plate, dish or flask. In still further examples, the cells are added to the culture dish in appropriate media and, optionally, either allowed to settle to the bottom of the culture dish by gravity, or forced to the bottom by, for example, centrifugation, and the assay is then continued without any substantial incubation or growth. Other examples can use cells in suspension.

### E. PHARMACEUTICAL COMPOSITIONS

Described herein are pharmaceutical compositions that contain a therapeutic virus and a suitable pharmaceutical carrier. The virus can be any of the viruses described herein. The pharmaceutical compositions can contain an additional therapeutic agent, such as an anticancer agent. Exemplary anticancer agents are provided elsewhere herein.

Examples of suitable pharmaceutical carriers are known in the art and include phosphate buffered saline solutions, water, emulsions, such as oil/water emulsions, various types of wetting agents, sterile solutions. Such carriers can be formulated by conventional methods and can be administered to the subject at a suitable dose. Colloidal dispersion systems that can be used for delivery of viruses include macromolecule complexes, nanocapsules, microspheres, beads and lipid-based systems including oil-in-water emulsions (mixed), micelles, liposomes and lipoplexes. An exemplary colloidal system is a liposome. Organ-specific or cell-specific liposomes can be used in order to achieve delivery only to the desired tissue. The targeting of liposomes can be carried out by the person skilled in the art by applying commonly known methods. This targeting includes passive targeting (utilizing the natural tendency of the liposomes to distribute to cells of the RES in organs which contain sinusoidal capillaries) or active targeting (for example by coupling the liposome to a specific ligand, for example, an antibody, a receptor, sugar, glycolipid, protein etc., by well known methods). In the present methods, monoclonal antibodies can be used to target liposomes to specific tissues, for example, tumor tissue, via specific cell-surface ligands.

In some examples, the pharmaceutical composition can contain a therapeutic agent that increases the level of expression of a protein whose level of expression is increased in cells that respond favorably to viral therapy. In other examples, the pharmaceutical compound can contain a therapeutic agent that decreases the level of expression of a protein whose level of expression is decreased in cells that respond favorably to viral therapy. Such therapeutic agents can include proteins and/or nucleic acids. In certain examples, the therapeutic agent can include a chemical compound, a protein, a nucleic acid encoding a protein, a nucleic acid encoding an antisense nucleic acid, a nucleic acid encoding a siRNA, or a nucleic acid encoding a ribozyme.

In some examples, antisense nucleic acids described herein can be synthesized so as to increase their stability under *in vivo* conditions. Such antisense nucleic acids typically include one or more chemical modifications to the nucleic acid backbone, bases and/or sugar moieties. For example, such nucleic acids are those in having internucleotide phosphate residues with methylphosphonates, phosphorothioates, phosphoramidates, and phosphate esters. Nonphosphate intemucleotide analogs such as siloxane bridges, carbonate brides, thioester bridges, as well as many others known in the art can also be used in modified nucleic acids.

Modified nucleic acids also can contain α-anomeric nucleotide units and modified nucleotides such as 1,2-dideoxy-d-ribofuranose, 1,2-dideoxy-1-phenylribofuranose, and N⁴, N⁴-ethano-5-methyl-cytosine are contemplated for use in the methods and compositions herein. Modified nucleic acids can also be peptide nucleic acids in which the entire deoxyribose-phosphate backbone has been exchanged with a chemically completely different but structurally homologous, polyamide (peptide) backbone containing 2-aminoethyl glycine units.

In certain examples, one can employ antisense constructs which include other elements, for example, those which include C-5 propyne pyrimidines. Oligonucleotides which contain C-5 propyne analogues of uridine and cytidine have been shown to bind RNA with high affinity and to be potent antisense inhibitors of gene expression.

In other examples, siRNA and ribozymes can be used and applied in much the same way as described for antisense nucleic acids.

### F. METHODS OF ADMINISTERING VIRAL THERAPY

Also described herein are methods and compositions that relate to administering viral therapy to a subject. Such therapeutic methods can include one or more steps.

In some examples, administering viral therapy can include administering a pharmaceutical composition containing a therapeutic virus to a subject. The virus can be any of the viruses described herein. Pharmaceutical compositions include those described herein. Routes of administering viral therapy can include parenteral, e.g., intravenous, intradermal, subcutaneous, oral (*e.g.*, inhalation), transdermal (topical), transmucosal, and rectal administration.

In some examples, administering viral therapy can include administering more than one therapeutic virus to a subject. In such examples, each virus can have synergistic features for viral therapy. For example, a first therapeutic virus can be co-administered with a second virus encoding a therapeutic agent.

In some examples, administering viral therapy can include co-administering a therapeutic virus with a therapeutic agent to the subject. Therapeutic agents can include chemical compounds, proteins and/or nucleic acids. In certain examples, the therapeutic agent can include a protein, a nucleic acid encoding a protein, a nucleic acid encoding an antisense nucleic acid, a nucleic acid encoding a siRNA, or a nucleic acid encoding a ribozyme. Therapeutic agents can be co-administered to a subject at the same time as a therapeutic virus, or at a different time. Therapeutic agents can be administered as pharmaceutical compositions according to the methods provided herein. Exemplary therapeutic agents are provided elsewhere herein.

### 1. Monitoring the progress of viral therapy

Described herein are methods of monitoring the progress of viral therapy in a subject. Such methods of monitoring can include determining whether the expression of at least one marker is altered in a biological sample, such as a tumor sample, from the subject obtained at a plurality of time points.

In some examples, methods to monitor the progress of viral therapy can include one or more steps. For example, the method can include obtaining a biological sample, such as a biopsy of a tumor, from a subject; measuring the level of expression of at least one marker in a first biological sample at a first time point; obtaining a second biological sample from a subject; measuring the level of expression in the same at least one marker in the second biological sample at a second time point; and determining whether the level of expression of the at least one marker has increased, decreased or remained substantially the same during the interval between the first and second time points.

In some examples, the level of expression of at least one marker can be measured in a first biological sample, such as a tumor sample, at a first time point. The first time point can be before viral therapy is administered, at the time viral therapy is administered, or during viral therapy. In further examples, the level of expression of at least one marker can be measured in the second biological sample, such as a second tumor sample, at least a second time point. The time between the first time point and the at least second time point can be about 30 minutes, about 1 hour, about 6 hours about 12 hours, about I day, about 2 days, about 3 days, about 4 days, about 5 days, about 6 days, about 7 days, about 8 days, about 9 days, about 10 days, about 11 days, about 12 days, about 13 days, about 14 days, about 2 weeks, about 3 weeks, about 4 weeks, and about 1 month.

In certain examples, the first biological sample can be obtained from the same anatomical site as the second biological sample.

In some examples, the marker can be a protein whose level of expression is increased in cells that respond favorably to viral therapy, a protein whose level of expression is decreased in cells that respond favorably to viral therapy, or a protein whose level of expression is substantially the same in cells that respond favorably to viral therapy.

In some examples, the at least one marker can be selected from among the markers listed in Table 1, Table 2 and Table 3. In some examples, the at least one marker encompasses a plurality of markers selected from among the markers listed in Table 1, Table 2, and Table 3. In some examples, the expression of at least 1, at least 5, at least 10, at least 15, or at least 20 markers can be selected from among the markers listed in Table 1, Table 2 and Table 3 can be determined. In some examples, the expression level of all the markers in Table 1, Table 2, and Table 3 can be determined. In other examples, the at least one marker can be a marker identified by methods described herein.

In some examples, the marker can be a host protein whose level of expression is increased a tumor that is responding favorably to viral therapy, a host protein whose level of expression is decreased in cells that respond favorably to viral therapy, or a host protein whose level of expression is substantially the same in cells that respond favorably to viral therapy. Such proteins are produced by the host (not the tumor cells themselves), but are found within a tumor tissue sample.

In some examples, measuring the level of expression of at least one marker can be carried out using methods well known in the art. For protein levels, examples of methods can include, but are not limited to microarray analysis, ELISA assays, Western blotting, or any other technique for the quantitation of specific proteins. For RNA levels, examples of techniques include microarray analysis, quantative PCR, Northern hybridization, or any other technique for the quantitation of specific nucleic acids.

In some examples, the step of determining whether the level expression of the at least one selected marker in a first biological sample, such as a tumor sample, contacted with a therapeutic virus has decreased, increased, or remained substantially the same, as compared to the expression of the same at least one selected marker in a second biological sample, such as a second tumor sample, can be performed by comparing quantitative or semi-quantitative results obtained from the measuring step. In some examples, the difference in expression of the same selected marker between the first biological sample and the second biological sample can be about less than 2-fold, about 2-fold, about 3-fold, about 4-fold, about 5-fold, about 6-fold, about 7-fold, about 8-fold, about 9-fold, about 10-fold, about 20-fold, about 30-fold, about 40-fold, about 50-fold, about 60-fold, about 70-fold, about 80-fold, about 90-fold, about 100-fold or greater than about 100-fold.

In those examples where the at least one marker is selected from Table 1, an increase in expression of the selected marker in second biological sample as compared to expression of the same marker in a first biological sample can indicate that the subject is responding favorably to viral therapy. Conversely, no increase can indicate that the subject is responding poorly to viral therapy. In those examples where the at least one marker is selected from Table 2, a decrease in expression of the selected marker in a second biological sample as compared to expression of the same marker in a first biological sample can indicate that the subject is responding favorably to viral therapy. Conversely, no decrease in expression of the selected marker can indicate that the subject is responding poorly to viral therapy. In those examples where the at least one marker is selected from Table 3, no substantial change in the level of expression of the selected marker in a second biological sample as compared to expression of the same marker in a first biological sample can indicate that the subject is responding favorably to viral therapy. In such examples, evidence of a subject's favorable or poor response to viral therapy can be corroborated by measuring the level of expression of markers from Table 1 and Table 2, respectively.

In some examples, a subject's response to viral therapy can be used to determine whether viral therapy is effective and as a basis for further therapeutic decisions, for example, whether to modify viral therapy, to continue viral therapy, or to administer alternative therapy.

In examples where the subject responds poorly to viral therapy containing a first therapeutic virus, the subject can be re-assessed to determine whether it is likely to respond favorable or poorly to a second therapeutic virus using the methods described herein.

### G. IDENTIFYING MARKERS ASSOCIATED WITH A RESPONSE TO VIRAL THERAPY

Described herein are methods of identifying markers associated with a favorable or poor response to viral therapy. Such methods can include determining whether the level of expression of a candidate marker is altered by contact with a therapeutic virus in a cell in which a therapeutic virus replicates well or poorly.

In some examples, methods to identify a marker associated with a subject's response to viral therapy can include one or more steps. Such steps can include comparing the level of expression of a candidate marker in a cell contacted with the virus to the level of expression of the candidate marker in a cell which has not been contacted with the virus.

In some examples, a single biological sample is obtained and divided into two test samples. One test sample is not contacted with the virus, while the other test sample is contacted with the virus. The level of expression of the candidate marker in the two test samples is compared.

In some examples, the cell can contain a cell known to respond favorably to viral therapy vectors or a cell which permits good viral replication. In some examples, the cell can be from a cell-line. Cell-lines that respond favorably or poorly to viral therapy vectors are described herein. The virus can be any virus described herein. Examples of cell-lines known to respond favorably viral therapy vectors can include, but are not limited to PANC-1, MIA PaCa-2, A549, OVCAR-3 and GI-101A, A549, DU145, MEL-888 and MEL-1858.

In other examples, the cell can be a cell known to respond poorly to viral therapy or to permit a poor level of viral replication. Examples of cell-lines known to respond poorly to viral therapy vectors can include, but are not limited to, PC-3, SiHa, NCI-H1299, MDA-MB-23, MEL-1936 and HT-29.

Any therapeutic virus described herein can be used in conjunction with the methods to identify markers associated with a biological sample's response to viral therapy, such an anti tumor response. In some examples, the virus can be a virus which is known to be effective in viral therapy. For example, in some examples the virus contains the GLV-1h68 virus. The GLV-1h68 virus is described in U.S. Patent Application No. 10/872,156.

In some examples, methods of identifying a candidate marker include culturing the cells before measuring the level of expression of the candidate marker. The cells can be cultured *in vitro* according to methods known in the art. Alternatively, the cells can be cultured *in vivo.* In such methods, the cells can be implanted subcutaneously into an organism, such as a nude mouse. Where the cells are cultured *in vivo,* the expression levels of markers in the implanted cells or in the tissues of the host organism can be measured.

The cells contacted with the therapeutic virus can be cultured for a period of time sufficient to detect a response to the virus. In some examples, the period of time can be determined by the sensitivity of the method used to measure the level of expression of at least one marker. For example, the cells contacted with the therapeutic virus can be cultured for about 30 minutes, about 1 hour, about 6 hours about 12 hours, about 1 day, about 2 days, about 3 days, about 4 days, about 5 days, about 6 days, about 7 days, about 8 days, about 9 days, about 10 days, about 11 days, about 12 days, about 13 days, about 14 days, about 2 weeks, about 3 weeks, about 4 weeks, and about 1 month.

The level of expression of the candidate marker can be measured using methods well known in the art. For example, for RNA, techniques such as microarray analysis, Quantative PCR, Northern hybridization, or any other technique for the quantitation of specific nucleic acids can be used. For a protein marker, methods such as microarray analysis, ELISA assays, Western blotting, or any other technique for the quantitation of specific proteins can be used to measure the expression of a candidate protein marker can be used.

In some examples of the methods for identifying a marker associated with a favorable or poor response to viral therapy described herein, the step of determining whether the level expression of the candidate marker in cells contacted with a therapeutic virus has decreased, increased, or remained substantially the same, as compared to the expression of the candidate marker in non-contacted cells can be performed by comparing quantitative or semi-quantitative results. In some examples, the difference in expression of the candidate marker between the contacted and non-contacted cells can be about less than 2-fold, about 2-fold, about 3-fold, about 4-fold, about 5-fold, about 6-fold, about 7-fold, about 8-fold, about 9-fold, about 10-fold, about 20-fold, about 30-fold, about 40-fold, about 50-fold, about 60-fold, about 70-fold, about 80-fold, about 90-fold, about 100-fold or greater than about 100-fold.

### H. IDENTIFYING A VIRUS FOR VIRAL THERAPY

Also provided herein are methods of identifying a candidate virus for viral therapy. Such methods can include assessing whether a candidate virus is likely to be effective in viral therapy, and in particular, whether the candidate virus alters the level of expression of at least one marker in a cell contacted with the candidate virus. In some examples, the cell can be a cell which is known to be responsive to viral therapy vectors. If the expression of the at least one marker is altered by the candidate virus in a manner associated with a favorable response to viral therapy vectors, the candidate virus is likely to be successful as a viral therapy vector.

In certain examples, methods to identify a candidate virus for viral therapy can include one or more steps. Such steps can include measuring the level of expression of at least one marker in a first cell; measuring the level of expression in the same at least one marker in a second cell contacted with the candidate virus; and determining whether the level of expression of the at least one marker has increased, decreased or remained substantially the same in response to contacting the biological sample, such as a tumor sample, with the candidate virus.

In some examples, at least one marker can be selected from among the proteins listed in Table 1, Table 2 and Table 3. In some examples, at least one marker encompasses a plurality of markers selected from among the proteins listed in Table 1, Table 2, and Table 3. In some examples, at least 1, at least 5, at least l0, at least 15, at least 20 markers can be selected from among the proteins listed in Table 1, Table 2 and Table 3. In some examples, at least one marker selected for the determining step includes all the proteins listed in Table 1, Table 2, and Table 3. In other examples, at least one marker can be a protein identified by methods described herein.

In some examples, the cell can be a cell known to respond favorably to viral therapy vectors. Cell-lines that respond favorably or poorly to viral therapy vectors also are described in U.S. Provisional Patent Application Attorney Docket No. 117 "Systems and Methods for Viral Therapy" filed on October 25, 2007 and November 14, 2007. Examples of cell-lines known to respond favorably to the viral therapy vectors can include, but are not limited to, PANC-1, MIA PaCa-2, A549, OVCAR-3 and GI-101A, A549, DU145, MEL-888 and MEL-1858.

Any therapeutic virus described herein can be used in conjunction with the methods to identify a virus for use in viral therapy.

In some examples, the cells can be cultured *in vitro* according to methods known in the art. Alternatively, the cells can be cultured *in vivo.* In such methods, the cells can be implanted subcutaneously into an organism, such as a nude mouse. Where the cells are cultured *in vivo,* the level of expression of host markers in response to the candidate virus can be determined.

The cells contacted with the candidate virus can be cultured for a period of time sufficient to detect a response to the virus. In some examples, the period of time can be determined by the sensitivity of the method used to measure the level of expression of at least one marker. For example, the cells contacted with the therapeutic virus can be cultured f for about 30 minutes, about 1 hour, about 6 hours about 12 hours, about 1 day, about 2 days, about 3 days, about 4 days, about 5 days, about 6 days, about 7 days, about 8 days, about 9 days, about 10 days, about 11 days, about 12 days, about 13 days, about 14 days, about 2 weeks, about 3 weeks, about 4 weeks, and about 1 month.

The level of expression of the candidate marker can be measured using methods well known in the art. For example, for RNA, techniques such as microarray analysis, Quantative PCR, Northern hybridization, or any other technique for the quantitation of specific nucleic acids can be used. For a protein marker, methods include microarray analysis, ELISA assays, Western blotting, or any other technique for the quantitation of specific proteins can be used to measure the expression of a candidate protein marker.

In some examples of the methods for assessing whether a candidate virus is likely to be effective in viral therapy described herein, the step of determining whether the level expression of the at least one selected marker in a cell contacted with the candidate virus has decreased, increased, or remained substantially the same, as compared to the expression of the same at least one selected marker in a non-contacted cell can be performed by comparing quantitative or semi-quantitative measurements of the expression levels on the at least one marker. In some examples, the difference in expression of the same selected marker between the contacted and non-contacted cells can be about less than 2-fold, about 2-fold, about 3-fold, about 4-fold, about 5-fold, about 6-fold, about 7-fold, about 8-fold, about 9-fold, about 10-fold, about 20-fold, about 30-fold, about 40-fold, about 50-fold, about 60-fold, about 70-fold, about 80-fold, about 90-fold, about 100-fold or greater than about 100-fold.

In those examples where the at least one marker is selected from the markers listed in Table 1, an increase in expression of the selected marker in contacted cells as compared to expression of the same marker in a non-contacted cells can indicate that the candidate virus is likely to be effective for viral therapy. Conversely, no increase can indicate that the candidate virus is likely to be less effective for viral therapy. In those examples where the at least one marker is selected from the markers listed in Table 2, a decrease in expression of the selected marker in contacted cells as compared to expression of the same marker in non-contacted cells can indicate that candidate virus is likely to be effective for viral therapy. Conversely, no decrease can indicate that the candidate virus is likely to be less effective for viral therapy. In those examples where the at least one marker is selected from the markers listed in Table 3, no substantial change in the level of expression of the selected marker in a contacted cell as compared to expression of the same marker in non-contacted cells can indicate that the candidate virus is likely to be effective for viral therapy. In such examples where the at least one marker is selected from among the markers listed in Table 3, if desired, evidence of whether a candidate virus is likely to be effective for viral therapy can be corroborated by measuring the level of expression of markers one or more markers from Table 1 and Table 2.

### I. ARTICLES OF MANUFACTURE AND KITS

Also described herein are kits. Kits can contain reagents, devices or instructions for use thereof. A kit can contain a variety of components. Components can include reagents to measure the expression level of at least one marker associated with a favorable or a poor response to viral therapy; at least one therapeutic virus; a therapeutic agent; a pharmaceutical composition; a reagent or device to administer viral therapy; a host cell containing a therapeutic virus; a reagent or device to obtain a biological sample; or reagents to measure the presence of a therapeutic virus in a subject.

In some examples, a kit can contain reagents to measure the expression level of one or more markers associated with a favorable and/or poor response to viral therapy. Such kits can contain means for, or components for measuring particular protein levels in a biological sample, such as, monoclonal antibodies specific to a particular protein; or a means or component for measuring particular mRNA levels in a biological sample, such as, nucleic acid probes specific for RNA encoding the marker.

In some examples, a kit can contain at least one therapeutic virus. In some kits, a plurality of viruses designed for viral therapy can be supplied. Such kits can be provided to determine whether a subject responds favorably to any virus of the kit.

In one example, a kit can contain instructions. Instructions typically include a tangible expression describing the virus and, optionally, other components included in the kit, and methods for administration, including methods for determining the health status of the subject, the proper dosage amount, and the proper administration method, for administering the virus. Instructions can also include guidance for monitoring the progress of the subject over the duration of the treatment time.

In some examples, a kit can include a device for administering a virus to a subject. Any of a variety of devices known in the art for administering medications or vaccines can be included in the kits provided herein. Exemplary devices include a hypodermic needle, an intravenous needle, a catheter, a needle-less injection device, an inhaler, and a liquid dispenser such as an eyedropper. Typically, the device for administering a virus of the kit will be compatible with the virus of the kit; for example, a needle-less injection device such as a high pressure injection device can be included in kits with viruses not damaged by high pressure injection, but is typically not included in kits with viruses damaged by high pressure injection.

In some examples, a kit can include a device for administering a therapeutic agent to a subject. Any of a variety of devices known in the art for administering medications to a subject can be included in the kits provided herein. Exemplary devices include a hypodermic needle, an intravenous needle, a catheter, a needle-less injection device, an inhaler, and a liquid dispenser. Typically the device for administering the therapeutic agent will be compatible with the desired method of administration of the therapeutic agent. For example, a therapeutic agent to be delivered subcutaneously can be included in a kit with a hypodermic needle and syringe.

### J. EXAMPLES

The following examples are included for illustrative purposes only and are not intended to limit the scope of the invention.

### Example 1

### Construction of Viruses for use in Anti-cancer Efficacy Assays

Viruses for use in exemplary assays to assess the efficacy of viruses for anti-cancer treatment were generated by modification of the vaccinia virus strain designated LIVP (a vaccinia virus strain, originally derived by adapting the Lister strain (ATCC Catalog No. VR-1549) to calf skin (Institute for Research on Virus Preparations, Moscow, Russia, Al'tshtein et al. (1985) Dokl. Akad. Nauk USSR 285:696-699). The LIVP strain (whose genome sequence is set forth in SEQ ID NO: 1) from which the viral strains were generated contains a mutation in the coding sequence of the thymidine kinase (*TK*) gene in which a substitution of a guanine nucleotide with a thymidine nucleotide (nucleotide position 80207 of SEQ ID NO: 1) introduces a premature STOP codon within the coding sequence. The LIVP strain was further modified to generate the GLV-1h68 virus (SEQ ID NO: 2; U.S. Patent Publication No. 2005-0031643 and Japanese Patent No. 3,934,673).

As described in U.S. Patent Publication No. 2005/0031643 and Japanese Patent No. 3,934,673 (see particularly Example 1 in each application), GLV-1 h68 was generated by inserting expression cassettes encoding detectable marker proteins into the *F14.5L* (also designated in LIVP as *F3*) gene, thymidine kinase (*TK*) gene, and hemagglutinin (*HA*) gene loci of the vaccinia virus LIVP strain. All cloning steps were performed using vaccinia DNA homology-based shuttle plasmids generated for homologous recombination of foreign genes into target loci in the vaccinia virus genome through double reciprocal crossover (see Timiryasova et al. (2001) BioTechniques 31(3) 534-540). As described in U.S. Patent Publication 2005/0031643 and Japanese Patent No. 3,934,673, the GLV-1h68 virus was constructed using plasmids pSC65 (Chakrabarti et al. (1997) Biotechniques 23:1094-1097) and pVY6 (Flexner et al. (1988) Virology 166:339-349) to direct insertions into the *TK* and *HA* loci of LIVP genome, respectively. Recombinant viruses were generated by transformation of shuttle plasmid vectors using the FuGENE 6 transfection reagent (Roche Applied Science, Indianapolis, IN) into CV-1 cells (ATCC Cat No. CR1-1469), which were pre-infected with the LIVP parental virus, or one of its recombinant derivatives.

The expression cassettes were inserted in the LIVP genome in three separate rounds of recombinant virus production. In the first round, an expression cassette containing a *Ruc-GFP* cDNA (a fusion of DNA encoding *Renilla* luciferase and DNA encoding GFP) under the control of a vaccinia synthetic early/late promoter P_{SEL} was inserted into the Not I site of the *F14.5L* gene locus. In the second round, the resulting recombinant virus from the first round was further modified by insertion of an expression cassette containing DNA encoding beta-galactosidase (LacZ) under the control of the vaccinia early/late promoter P_{7.5k} (denoted (P_{7.5k})/lacZ) and DNA encoding a rat transferrin receptor positioned in the reverse orientation for transcription relative to the vaccinia synthetic early/late promoter P_{SEL} (denoted (P_{SEL})*rTrfR*) was inserted into the *TK* gene (the resulting virus does not express transferrin receptor protein since the DNA encoding the protein is positioned in the reverse orientation for transcription relative to the promoter in the cassette). In the third round, the resulting recombinant virus from the second round was then further modified by insertion of an expression cassette containing DNA encoding β-glucuronidase under the control of the vaccinia late promoter P₁₁ₖ (denoted (P₁₁ₖ)*gusA*) was inserted into the *HA* gene. The resulting virus containing all three insertions is designated GLV-1h68. The complete sequence of GLV-1h68 is shown in SEQ ID NO:2.

The expression of the *Ruc-GFP* fusion protein by the recombinant virus was confinned by luminescence assay and fluorescence microscopy. Expression of β-galactosidase and β-glucuronidase were confirmed by blue plaque formation upon addition of 5-bromo-4-chloro-3-indolyl-β-D-galactopyranoside (X-gal, Stratagene, La Jolla, CA) and 5-bromo-4-chloro-3-indolyl-β-D-glucuronic acid (X-G1cA, Research Product International Corporation, Mt. Prospect, IL), respectively. Positive plaques formed by recombinant virus were isolated and purified. The presence of expression cassettes in the F14.5L, *TK* and *HA* loci were also confirmed by PCR and DNA sequencing.

High titer viral preparations were obtained by centrifugation of viral precipitates in sucrose gradients (Joklik WK (1962) Virol. 18:9-18). For testing infection, CV-1 (1 x 10⁵) and GI-101A (4 x 10⁵) cells (Dr. A. Aller, Rumbaugh-Goodwin Institute for Cancer Research, Inc., Plantation, Florida) were seeded onto 24-well plates. After 24 hours in culture, the cells were infected with individual viruses at a multiplicity of infection (MOI) of 0.001. The cells were incubated at 37°C for 1 hour with brief agitation every 10 minutes to allow infection to occur. The infection medium was removed, and cells were incubated in fresh growth medium until cell harvest at 24, 48, 72, or 96 hours after infection. Viral particles from the infected cells were released by a quick freeze-thaw cycle, and the titers determined as pfu/ml of medium in duplicate by plaque assay in CV-1 cell monolayers. The same procedure was followed using a resting CV-1 cell culture, which was obtained by culturing a confluent monolayer of CV-1 cells for 6 days in DMEM supplemented with 5% FBS, before viral infection.

### Example 2

### Replication of GLV-1h68 vaccinia virus in different tumor cell types

### Materials and Methods

### Cell Lines Employed

A panel of well-characterized human cancer cell lines of different histological derivation was employed in the studies described herein. All cell lines except noted were purchased from American Type Culture Collection (Manassas).

MDA MB-231 (ATCC Cat No. HTB-26), PANC-1 (ATCC Cat No. CRL-1469), CV-1 (ATCC Cat No. CRL-1469) and PC-3 (ATCC Cat No. CRL-1435) cells were cultured in Dulbecco's modified Eagle's medium (DMEM) supplemented with 10% fetal bovine serum (FBS) and 1% antibiotic-antimycotic solution (AA) (100 U/ml penicillin G, 250 ng/ml amphotericin B, 100). MIA PaCa-2 (ATCC Cat No. CRL-1420) cells were cultured under similar conditions in DMEM media supplemented with 12.5% FBS and 2 mM L-glutamine. SiHa (ATCC Cat No. HTB-35) cells were cultured in Eagle's minimal essential medium (EMEM) supplemented with 10% FBS, I % non-essential amino acids (NEAA), 1 mM sodium pyruvate and 1% AA.

All other cells were cultured in Roswell Park Memorial Institute medium (RPMI) supplemented with the following compounds: A549 (ATCC Cat No. CCL-185) and HT-29 (ATCC Cat No. HTB-38) cells (10% FBS and 1% AA); GI-101A cells (Dr. A. Aller, Rumbaugh-Goodwin Institute for Cancer Research, Inc., Plantation, Florida; 20% FBS, 4.5 g/L glucose, 10 mM HEPES, 1mM sodium pyruvate, 1% AA and 4 ng/ml β-estradiol, 5 ng/ml progesterone); NCI-H1299 (ATCC Cat No. CRL-580) cells (10% FBS, 4.5 g/L glucose, 10 mM HEPES, 1 mM sodium pyrucate, 1% AA); and OVCAR-3 (ATCC Cat No. HTB-161) cells (20% FBS, 2.3 g/L glucose, 10 mM HEPES, 1mM sodium pyruvate, 1% AA and 4 ng/ml β-estradiol, 5 ng/ml progesterone and human Insulin). Three additional cell lines from distinct cutaneous melanoma metastases obtained from patient 888 (Dr. Francesco Marincola, National Institutes of Health; Wang et al. (2006) J Invest Dermatol. 126(6):1372-7; Sabatino, M., et al. (2008) Cancer Res 68:222-231) were included in the tests. 888-MEL, 1858-MEL and 1936-MEL cells were cultured in RPMI supplemented with 10% FBS, 1 mM HEPES, 1 mM Ciprofloxacin and L-glutamine/penicillin/streptomycin. All cell cultures were carried out at 37°C under 5% CO₂.

### In vitro viral replication assay

The cells, described above, were seeded in 24-well plates at a density of 2-4 x 10⁵ cells per well and were infected with GLV-1h68 at a MOI of 0.01 after 24 hours of culture (Zhang, Q. et al. (2007) Cancer Res 67:10038-10046). The cells were incubated at 37°C for 1 h with brief agitation every 10 min to allow infection to occur. The infection medium was removed, and cells were incubated in fresh growth medium until cell harvest at 24, 48 or 72 h after infection. Viral particles from the infected cells were released by a quick freeze-thaw cycle, and the titers determined in duplicate as pfu/ml of medium by standard plaque assay in CV-1 cell monolayers. The same procedure was followed using a resting CV-1 cell culture, which was obtained by culturing a confluent monolayer of CV-1 cells for 6 days in DMEM supplemented with 5% FBS before viral infection. Data for the average viral titers at 0, 24, 48 and 72 hours post infection is shown in Tables 5A-5L.

**Table 5A**

| **Viral titer values for PANC-1** | | |
|---|---|---|
| **Hours Post Infection** | **Average Viral Titer (Log pfu/10⁶ cells)** | **Standard Deviation (Log pfu/10⁶ cells)** |
| 0 | 4.00 | 0.00 |
| 24 | 4.45 | 0.07 |
| 48 | 6.47 | 0.07 |
| 72 | 7.06 | 0.06 |

**Table 5B**

| **Viral titer values for MIA PaCa-2** | | |
|---|---|---|
| **Hours Post Infection** | **Average Viral Titer (Log pfu/10⁶ cells)** | **Standard Deviation (Log pfu/10⁶ cells)** |
| 0 | 4.00 | 0.00 |
| 24 | 5.31 | 0.11 |
| 48 | 7.07 | 0.16 |
| 72 | 7.45 | 0.06 |

**Table 5C**

| **Viral titer values for A549** | | |
|---|---|---|
| **Hours Post Infection** | **Average Viral Titer (Log pfu/10⁶ cells)** | **Standard Deviation (Log pfu/10⁶ cells)** |
| 0 | 4.00 | 0.00 |
| 24 | 6.26 | 0.05 |
| 48 | 7.37 | 0.19 |
| 72 | 7.55 | 0.09 |

**Table 5D**

| **Viral titer values for OVCAR-3** | | |
|---|---|---|
| **Hours Post Infection** | **Average Viral Titer (Log pfu/10⁶ cells)** | **Standard Deviation (Log pfu/10⁶ cells)** |
| 0 | 4.00 | 0.00 |
| 24 | 4.59 | 0.06 |
| 48 | 6.12 | 0.34 |
| 72 | 6.20 | 0.03 |

**Table 5E**

| **Viral titer values for GI-101A** | | |
|---|---|---|
| **Hours Post Infection** | **Average Viral Titer (Log pfu/10⁶ cells)** | **Standard Deviation (Log pfu/10⁶ cells)** |
| 0 | 4.00 | 0.00 |
| 24 | 5.24 | 0.27 |
| 48 | 6.04 | 0.02 |
| 72 | 6.60 | 0.07 |

**Table 5F**

| **Viral titer values for PC-3** | | |
|---|---|---|
| **Hours Post Infection** | **Average Viral Titer (Log pfu/10⁶ cells)** | **Standard Deviation (Log pfu/10⁶ cells)** |
| 0 | 4.00 | 0.00 |
| 24 | 4.11 | 0.07 |
| 48 | 5.95 | 0.12 |
| 72 | 5.90 | 0.11 |

**Table 5G**

| **Viral titer values for SiHa** | | |
|---|---|---|
| **Hours Post Infection** | **Average Viral Titer (Log pfu/10⁶ cells)** | **Standard Deviation (Log pfu/10⁶ cells)** |
| 0 | 4.00 | 0.00 |
| 24 | 4.09 | 0.07 |
| 48 | 5.57 | 0.15 |
| 72 | 5.93 | 0.41 |

**Table 5H**

| **Viral titer values for NCI-H1299** | | |
|---|---|---|
| **Hours Post Infection** | **Average Viral Titer (Log pfu/10⁶ cells)** | **Standard Deviation (Log pfu/10⁶ cells)** |
| 0 | 4.00 | 0.00 |
| 24 | 4.08 | 0.07 |
| 48 | 5.08 | 0.10 |
| 72 | 6.17 | 0.08 |

**Table 5I**

| **Viral titer values for MDA-MB-231** | | |
|---|---|---|
| **Hours Post Infection** | **Average Viral Titer (Log pfu/10⁶ cells)** | **Standard Deviation (Log pfu/10⁶ cells)** |
| 0 | 4.00 | 0.00 |
| 24 | 3.38 | 0.11 |
| 48 | 4.90 | 0.16 |
| 72 | 5.82 | 0.13 |

**Table 5J**

| **Viral titer values for 888-MEL** | | |
|---|---|---|
| **Hours Post Infection** | **Average Viral Titer (Log pfu/10⁶ cells)** | **Standard Deviation (Log pfu/10⁶ cells)** |
| 0 | 4.00 | 0.00 |
| 24 | 4.94 | 0.02 |
| 48 | 6.19 | 0.12 |
| 72 | 6.39 | 0.12 |

**Table 5K**

| **Viral titer values for 1858-MEL** | | |
|---|---|---|
| **Hours Post Infection** | **Average Viral Titer (Log pfu/10⁶ cells)** | **Standard Deviation (Log pfu/10⁶ cells)** |
| 0 | 4.00 | 0.00 |
| 24 | 4.98 | 0.07 |
| 48 | 5.63 | 0.05 |
| 72 | 6.27 | 0.01 |

**Table 5L**

| **Viral titer values for 1936-MEL** | | |
|---|---|---|
| **Hours Post Infection** | **Average Viral Titer (Log pfu/10⁶ cells)** | **Standard Deviation (Log pfu/10⁶ cells)** |
| 0 | 4.00 | 0.00 |
| 24 | 5.06 | 0.10 |
| 48 | 6.01 | 0.03 |
| 72 | 6.08 | 0.20 |

The cell lines were divided into groups of predicted responders and non-responders based on the ability of the virus to exhibit significant replication within the cells within 24 hours post infection. Cell types that exhibited an approximate 4-fold or greater increase in viral titer over input titer were designated as *in vitro* responders. Table 6 displays the fold increase in viral titer between consecutive time points.

**Table 6**

| **Predicted Responders and Non-responders based on *in vitro* replication assays** | | | | |
|---|---|---|---|---|
| | **Cell Line** | **Fold Increase** | | |
| | | **24 h.p.i./Input** | **48 h.p.i./24 h.p.i.** | **72 h.p.i./48 h.p.i.** |
| ***In vitro* responders** | A549 | 183.69 | 12.85 | 1.49 |
| | HT-29 | 24.96 | 6.39 | 1.53 |
| | MIA PaCa-2 | 20.53 | 56.68 | 2.43 |
| | GI-101A | 17.38 | 6.31 | 3.63 |
| | 888-MEL | 8.78 | 17.59 | 1.58 |
| | 1858-MEL | 9.45 | 4.48 | 4.43 |
| | 1936-MEL | 11.53 | 8.82 | 1.19 |
| | PANC-1 | 2.81 | 104.92 | 3.88 |
| | OVCAR-3 | 3.93 | 33.38 | 1.21 |
| ***In vitro* non-responders** | SiHa | 1.22 | 30.55 | 2.27 |
| | NCI-H1299 | 1.21 | 9.87 | 12.52 |
| | MDA MB-231 | 0.24 | 33.63 | 8.34 |
| | PC-3 | 1.28 | 70.01 | 0.88 |

| | | | | |
|---|---|---|---|---|
| h.p.i. = hours post viral infection | | | | |

### Example 3

### In vivo tumor regression following GLV-1h68 vaccinia virus treatment of different xenograft tumors

In order to determine whether the *in vitro* replication profile correlated with efficacy *in vivo*, each cell line was tested for sensitivity to the oncolytic activity of systemically administered GLV-1h68 using tumor xenograft mouse models (Sabatino, M. et al. (2008) Cancer Res 68:222-231; Jones, C.B. et al. (1997) Cancer Chemother. Pharmacol. 40:475-483; Schultz, R.M. et al. (1993) Oncol. Res. 5:223-228; Roschke, A.V. et al. (2003) Cancer Res. 63:8634-8647; Ahn, W.S. et al. (2005) Gene Ontology. Int. J Gynecol. Cancer 15:94-106).

6-8 week old nude mice (NCI:Hsd:Athymic Nude-*Foxnl^{nu}*, Harlan) were inoculated with 5 x 10⁶ cells per mouse to obtain subcutaneous xenografts as previously described (Zhang, Q. et al. (2007) Cancer Res 67:10038-10046). Thirty days after tumor cell implantation, a single intravenous inoculation of I x 10⁶ pfu of GLV-1h68 virus in a final volume of 100 µl PBS or PBS only as a control was delivered to the mice by femoral vein injection. Tumor growth was measured once a week before and after viral infection and tumor mass was reported in mm³. After inoculation with GLV-1h68, the expression of green fluorescent protein (due to the *Ruc-GFP* fusion protein encoded by the GLV-1h68 virus) within the tumors was also monitored under UV-light.

Two characteristic patterns of tumor growth were identified: some tumors progressively continued their growth independent of therapy (*i.e.* PC-3, HT-29; see Tables 7A and 7B), while some exhibited three phases of growth (Zhang, Q. et al. (2007) Cancer Res 67:10038-10046). The first phase during the first few weeks is characterized by a slightly faster expansion in the mass of tumors receiving treatment compared to control tumors. This initial rapid expansion may be related to an ongoing inflammatory process. The expansion phase was followed by a plateau phase and then by shrinkage of the tumor masses (*i.e.* GI-101A; see Table 7C). These patterns were cell line-specific and highly reproducible independent of GLV-1h68 plaque forming units (PFUs) injected or cancer cells inoculated. While the kinetics of growth and disappearance varied according to the various experimental conditions applied (PFUs of virus administered and number of cancer cells administered), the final outcome (tumor growth versus tumor regression) was highly reproducible for each tumor type tested.

During the initial growth phase, fluorescent light emission from tumors non-responding to therapy (*i.e.* HT-29) displayed a patchy pattern that did not significantly differ from that of tumors eventually responding to treatment (*i.e.* GI-101A), though the GI-101 tumor had a higher expression of the Ruc-GFP fusion protein.

**Table 7A**

| **Median tumor volumes at different time points after i.v. injection of GLV-1h68 into nude mice bearing PC-3 tumors** | | |
|---|---|---|
| **Days post-implantation of tumor cells** | **Median tumor volume (mm³)** | |
| | **No Treatment** | **GLV-1h68** |
| 63 | 323.5 | 373.15 |
| 71 | 322.05 | 446.1 |
| 78 | 408.9 | 546.15 |
| 86 | 549.7 | 679.8 |
| 105 | 1114.85 | 1335.35 |
| 114 | 1522.8 | 1499.9 |
| 133 | 2719.8 | 2685.2 |
| 149 | 3120.1 | 3342.45 |

**Table 7B**

| **Median tumor volumes at different time points after i.v. injection of GLV-1h68 into nude mice bearing HT-29 tumors** | | |
|---|---|---|
| **Days post-implantation of tumor cells** | **Median tumor volume (mm³)** | |
| | **No Treatment** | **GLV-1h68** |
| 16 | 180.06 | 197.39 |
| 23 | 472.56 | 472.79 |
| 30 | 1038.11 | 1049.85 |
| 37 | 1879.03 | 1734.03 |
| 44 | 2919.53 | 2687.96 |
| 50 | 3638.63 | 3036.29 |
| 57 | 5255.73 | 4244.63 |

**Table 7C**

| **Median tumor volumes at different time points after i.v. injection of GLV-1h68 into nude mice bearing GI-101A tumors** | | |
|---|---|---|
| **Days post-implantation of tumor cells** | **Median tumor volume (mm³)** | |
| | **No Treatment** | **GLV-1h68** |
| 33 | 240.8 | 248.4 |
| 36 | 263.6 | 243.8 |
| 43 | 579.1 | 550.4 |
| 50 | 636.4 | 761.3 |
| 57 | 671.6 | 852.0 |
| 64 | 904.3 | 1118.2 |
| 71 | 1235.9 | 1302.0 |
| 78 | 1431.8 | 1225.2 |
| 82 | 1888.1 | 1233.5 |
| 85 | 2166.5 | 1295.9 |
| 89 | 2548.0 | 1083.2 |
| 92 | 2715.6 | 1053.6 |
| 97 | 2918.3 | 962.2 |
| 102 | 3471.5 | 809.1 |
| 110 | nd | 818.4 |
| 118 | nd | 629.9 |

To provide a single parameter descriptive of individual cell line responsiveness to virus therapy, a therapeutic index (T.I.) was calculated for each tumor by integrating the areas between the median growth of control and treated xenografts (eight animals per group) during the first 45 days of treatment. The therapeutic index was calculated as: $therapeutic index = \left(A-B\right) / A$
where A is the area under the untreated control curve, and B is the area under the virus treatment curve, with both areas being from time of virus infection to 45 days post virus treatment. Table 8 lists the therapeutic indices calculated for the various xenograft tumors tested.

**Table 8**

| **Responders** | | | **Poor/Non-Responders** | | |
|---|---|---|---|---|---|
| **Cell Line Name** | **Description** | **TI** | **Cell Line Name** | **Description** | **TI** |
| 1858-MEL | Melanoma | 90.1 | MB-231 | Breast Adenocarcinoma | 21.6 |
| 888-MEL | Melanoma | 88.0 | SiHa | Cervical Squamous Cell Carcinoma | 15.6 |
| MIA PaCa-2 | Pancreatic Carcinoma | 80.1 | 1936-MEL | Melanoma | 13.7 |
| A549 | Lung Carcinoma | 62.8 | PC-3 | Prostate Adenocarcinoma | 8.6 |
| OVCAR-3 | Ovarian Carcinoma | 56.2 | NCI-H1299 | Breast Adenocarcinoma | -2.3 |
| PANC-1 | Pancreatic Carcinoma | 50.9 | HT-29 | Colorectal Carcinoma | -19.0 |
| DU145 | Prostate Cancer | 48.4 | | | |
| GI-101A | Breast Carcinoma | 27.9 | | | |

| | | | | | |
|---|---|---|---|---|---|
| TI = Therapeutic Index | | | | | |

### Comparison of in vitro and in vivo test results

In the *in vitro* replication assay, four of four cell lines that resisted virus replication during the first 24 hours following infection (MDA MB-231, PC-3, SiHa and NCI-H 1299) uniformly produced xenografts non-responding to vaccinia virus (VACV) therapy *in vivo*. Eight out of ten cell lines that allowed viral replication in the first 24 hours yielded xenografts responsive to vaccinia virus treatment *in vivo*, while two cell lines (HT-29 and 1936-MEL) yielded xenografts that did not respond to virus treatment. Notwithstanding the two outliers, the relationship between the permissivity of a given cell line to *in vitro* replication rate of GLV-1h68 and the *in vivo* responsiveness of the corresponding xenograft was statistically significant (Fisher exact test p₂-value = 0.005).

The difference in responder versus non-responder cell lines was not limited to particular cell lines of diverse ontogeny. 888-MEL and 1936-MEL, two autologous melanoma cell lines, exhibited differences in *in vivo* responsiveness to virus treatment, though they displayed the same degree of permissivity *in vitro* to GLV-1h68 replication. 888-MEL and 1936-MEL are derived from the same ancestral progenitor though established from two distinct melanoma metastases (Sabatino, M. et al. (2008) Cancer Res 68:222-231; Wang, E. et al. (2006) J Invest Dermatol 126:1372-1377). The first cell line (888-MEL) was removed in 1989 during earlier stages of disease at a time when the patient underwent a complete remission of all metastatic disease following adoptive transfer of tumor infiltrating lymphocytes; the second (1936-MEL), was expanded 12 years later from a metastasis excised at a time when the patient had rapidly progressing disease and did not respond to further therapy. The early cell line was highly sensitive to treatment (T.I. = 88.0) and was completely eradicated by the oncolytic virus administration while the later cell line 1936-MEL was completely resistant (T.I. = 13.7), continuing its growth with identical kinetics between treated and untreated animals. These data suggest that responsiveness is related to biological characteristics of the tumors independent of their ontogeny and is more likely related to evolving phenotypic alterations occurring during the natural history of the disease.

### Example 4

### In vivo viral replication in Responder versus Poor/Non-responder tumor types

To further investigate the reasons for the lack of *in vivo* responsiveness of xenografts derived by distinct cell lines, the *in vivo* behavior of three cell lines (GI-101A, HT-29 and PC-3), which demonstrated different and representative patterns of *in vitro* permissivity to GLV-1h68 replication and *in vivo* outcomes, was examined. Although viral replication *in vivo* was similar in the growth phase of GI-101A and HT-29 xenografts and comparable light emitting properties were observed, viral titer studies showed that viral replication was delayed *in vivo* in HT-29 xenografts as they continued their growth while it remained elevated in the regressing GI-101A xenografts.

Table 9 shows the *in vivo* viral titers (PFU/gram of xenograft) as an average of eight individual experiments for each treatment group comparing the permissivity of three xenografts derived from GI-101A colorectal cancer, HT-29 breast cancer and PC-3 prostate adenocarcinoma tumors. A dichotomy was observed between responding and non-responding xenografts equally permissive to GLV-1h68 *in vitro* but not *in vivo.* Moreover, PC-3 xenografts, in spite of delaying *in vitro* replication of vaccinia virus, allowed replication *in vivo* of GLV-1 h68 at a rate similar to HT-29 at day 21 and intermediate between HT-29 and GI-101A at day 42. In view of the replication and tumor regression data described above, delayed replication *in vivo* or *in vitro* appeared to decrease the likelihood of tumor regression while tumor regression was associated with effective viral replication *in vitro* and *in vivo.*

**Table 9**

| **Virus replication in *vivo*** | | | | | | |
|---|---|---|---|---|---|---|
| **Days Post Infection** | **GI-101A** | | **HT-29** | | **PC-3** | |
| | **pfu/g** | **STDEV** | **pfu/g** | **STDEV** | **pfu/g** | **STDEV** |
| 7 | 1.05E+07 | 4.90E+06 | 3.89E+06 | 1.11E+06 | 2.65E+07 | 3.56E+07 |
| 21 | 3.53E+08 | 1.90E+08 | 6.45E+07 | 9.12E+07 | 7.54E+07 | 8.05E+07 |
| 42 | 3.77E+08 | 2.42E+08 | 1.12E+08 | 1.56E+08 | 3.16E+08 | 4.03E+08 |

### Example 5

### Viral infectivity of various tumor cell lines

To determine whether the results obtained in the *in vitro* viral replication assay and/or *in vivo* tumor regression study were affected by the ability of vaccinia virus to infect the tumor cells, a test for viral infectivity of three vaccinia viruses (GLV-1h68, LIVP and Western Reserve Strain WR) was performed. Cells were plated in 24 well plates at a density of 2 x 10⁵ cells/well (A549, HT-29, MIA PaCa-2, GI-101A, PANC-1, OVCAR-3, SiHa, NCI-H1299 and MDA MB-231) or 3 x 10⁵ cells/well (PC-3 and DU-145, ATCC Catalog No. HTB-81). Cells were infected in duplicate or triplicate with 1 x 10⁵, 1 x 10⁶, 1 x 10⁷, 5 x 10⁷ dilutions of each virus (GLV-1h68, LIVP or WR) for 1h and then overlaid with 1 ml of corresponding cell medium. Viral titer was then determined by crystal violet staining (plaque assay). The infectivity of the various tumor cell lines tested is shown in Table 10. The results show that the degree of viral infectivity does not correlate with the efficacy of the virus to cause tumor regression or replicate within the tumor cell.

**Table 10**

| **Viral infectivity of various tumor cell lines** | |
|---|---|
| **Cell Type** | **Titer (pfu)** |
| A549 | 3.63E+09 |
| HT-29 | 1.63E+09 |
| MIA PaCa-2 | 1.50E+09 |
| GI-101A | 1.05E+09 |
| PANC-1 | 7.25E+08 |
| DU-145 | 1.35E+08 |
| OVCAR-3 | 6.50E+07 |
| SiHa | 3.50E+09 |
| NCI-H1299 | 1.27E+09 |
| MDA MB-231 | 6.00E+08 |
| PC-3 | 1.65E+08 |

### Example 6

### Microarray Analysis of in vivo and in vitro gene expression in Responder versus Poor/Non-responder tumor types

The comparison between *in vitro* and/or *in vivo* virus replication patterns and *in vivo* regression of different cell lines exposed to GLV-1h68 suggested a complex relationship between vaccinia virus, cancer cells and the host. To further examine these relationships, the gene expression patterns of vaccinia virus, human cancer cells and mouse host cells in responding and non-responding xenografts excised at times relevant to the kinetics of their response were examined. Transcriptional profiling was achieved though the use of organism-specific microarray platforms.

### Preparation of test samples

*In* vivo tumor samples from virus treated and untreated tumors were prepared for microarray analysis. Tumor xenograft models for GI-101A breast carcinoma, HT-29 colorectal carcinoma or PC-3 prostate adenocarcinoma were prepared as described above. Thirty days after tumor cell implantation, 1 x 10⁶ pfu of GLV-1h68 virus (in 100 µl PBS) or PBS only as control was administered to the mice by femoral vein injection. At selected days post virus infection, 3-4 animals from each of the treatment groups and 3-4 animals from the control groups were sacrificed and the tumors were excised. For GI-101A, the treatment groups included 1, 7, 21 and 42 days post virus infection. For HT-29 and PC-3, the treatment groups included 21 and 42 days post virus infection.

*In vitro* cell culture samples also were prepared for microarray analysis. GI-101A, HT-29 and PC-3 cells were seeded in 24-well plates and infected with GLV-1h68 at a MOI of 0.01. Cells were harvested at six and twelve hours post virus infection.

Total RNA from excised tumors was isolated after homogenization using Trizol (Invitrogen) reagent according to the manufacturer's instructions. Total RNA from cell cultures was isolated with the Qiagen RNeasy Mini kit according to the manufacturer's instructions and the quality of obtained total RNA was tested with an Agilent Bioanalyzer 2000 (Agilent Technologies). For expression studies based on cDNA and oligo array techniques, total RNA was amplified into antisense RNA as described in Wang, E. et al. (2000) Nature Biotech 17:457-459 and Wang, E. (2005) J. Transl. Med. 3:28.

Mouse reference RNA was prepared by homogenization and pooling of selected mouse tissues (lung, heart, muscle, kidneys, liver and spleen) from three female C57B1/6 mice. Reference RNA for human arrays was obtained by pooling peripheral blood mononuclear cells (PBMCs) from four normal donors. Human and mouse reference total RNA was amplified into antisense RNA as described in Wang, E. et al. (2000) Nature Biotech 17:457-459 and Wang, E. (2005) J. Transl. Med. 3:28. Five µg RNA of selected tumor and cell samples were amplified according to the Affymetrix, manual using the GeneChip® One-Cycle Target Labeling and Control kit.

### Microarray performance and statistical analysis

Confidence in array quality was determined as described Jin, P. et al. (2004) BMC Genomics 5:55). For 36k whole genome mouse and human array performances, reference and amplified test RNA were directly labeled using a ULS™ aRNA Fluorescent Labeling kit (Kreatech Biotechnology) with Cy3 for reference and Cy5 for test samples and were co-hybridized to the slides (Worschech, A. et al. (2008) Cancer Res. 68:2436-2446). 17k human cDNA arrays were carried out as described according to standard methods for labeling and array hybridization (Basil, C.F. et al. (2006) Cancer Res 66:2953-2961). A customized Vaccinia Virus-GLV-1h68 Affymetrix expression array was specifically prepared for this study. Amplified RNA from tumor or cell samples was handled according to the manufacturer's instructions for eukaryotic sample processing and hybridized to the arrays. After 16h incubation in the hybridization oven at 45°C, the arrays were washed and stained in the Fluidics station using the GeneChip® Hybridization, Wash, and Stain Kit (Affymetrix).

Resulting data files from both array types, Affymetrix and in house spotted arrays, were uploaded to the mAdb databank (nciarray.nci.nih.gov) and further analyzed using BRBArrayTools developed by the Biometric Research Branch, National Cancer Institute (linus.nci.nih.gov/BRB-ArrayTools.html) (Simon, R. et al. (2007) Cancer Informatics 2:11-17) and Cluster and TreeView software (Eisen, M.B. et al. (1998) Proc Natl. Acad. Sci. USA 95:14863-14868). Multiple dimensional scaling was performed using the BRB-array tool.

Retrieved data from the Affymetrix platform were normalized using median over entire array as reference because of single color labeling technology. For all array types, unsupervised analysis was used for class confirmation using the Stanford Cluster program (80% gene presence across all experiments and at least 3-fold ratio change) and Treeview program for visualization. Gene ratios were average corrected across experimental samples and displayed according to an uncentered correlation algorithm. Class comparison was performed using parametric unpaired Student's t test or three-way ANOVA to identify differentially expressed genes among GLV-1h68 infected and uninfected tumors or cells at various time points using different significance cutoff levels as demanded by the statistical power of each test. Subsequent filtering (80% gene presence across all experiments and at least 3-fold ratio change) narrowed down the number of genes that were expressed differentially between experimental groups.

Statistical significance and adjustments for multiple test comparisons were based on univariate and multivariate permutation test as previously described. Previous studies have shown that the present method for RNA amplification is robust yielding results comparable to those obtained by quantitative PCR (qPCR) (Jin, P. et al. (2004) BMC Genomics 5:55; Feldman, A.L. et al. (2002) Biotechniques 33:906-914; Nagorsen, D. et al. (2005) Genome Biol 6:R15). Additional quantitative PCR can be performed to confirm changes in expression of individual genes.

### Microarray Results

### 1. Transcriptional differences between responding xenografts versus non-responding xenografts to systemic GLV- 1h68 administration: vaccinia virus (VACV) signatures

Vaccinia virus (VACV) gene expression was assessed by a custom-made VACV array platform (VACGLa520445F, Affymetrix, CA). The array platform (See Table 11) included 308 probes representing 219 genes that covered the combined genome of several vaccinia virus strains; exogenous constructs specific to GLV-1h68 virus, such as the *Renilla* luciferase-*Aequorea* green fluorescent fusion protein; and 393 probes representing 337 human or mouse "house keeping" genes.

**Table 11**

| **VACV Array Platform Genes** | | | |
|---|---|---|---|
| **Name** | **SEQ ID NO** | **COP** | **Annotation** |
| VACGL001 | 159 | C23L/B29R | chemokine-binding protein |
| VACGL003 | 160 | - | fragment of Tumor necrosis factor receptor |
| VACGL004 | 161 | C22L/B28R | TNF-alpha-receptor-like |
| VACGL005 | 162 | - | ankyrin-like protein[Vaccinia] |
| VACGL006 | 163 | C18L/B24R | similar to putative C18L[VacCop] |
| VACGL007 | 164 | C17L/B23R | similar to putative C17L[VacCop] |
| VACGL008 | 165 | C17L/B23R | similar to putative C17L[VacCop] |
| VACGL009 | 166 | C12L | serine protease inhibitor-like SPI-1 |
| VACGL010 | 167 | C11R | secreted epidermal growth factor-like |
| VACGL011 | 168 | C10L | unknown |
| VACGL013 | 169 | - | zinc finger-like protein |
| VACGL014 | 170 | - | zinc finger-like protein |
| VACGL015 | 171 | - | interleukin-18-binding protein |
| VACGL016 | 172 | - | ankyrin-like protein |
| VACGL017 | 173 | - | ankyrin-like protein |
| VACGL018 | 174 | - | ankyrin-like protein |
| VACGL019 | 175 | - | ankyrin-like protein[vaccinia] |
| VACGL020 | 176 | - | "unknown, orthologous to TC10L[Tian tan]" |
| VACGL021 | 177 | C9L | ankyrin-like protein |
| VACGL023 | 178 | C8L | unknown |
| VACGL025 | 179 | C7L | host-range protein |
| VACGL026 | 180 | C6L | unknown |
| VACGL027 | 181 | C5L | unknown |
| VACGL028 | 182 | C4L | unknown |
| VACGL029 | 183 | C3L | secreted complement binding |
| VACGL030 | 184 | - | similar to putative C ORF A[VacCop] |
| VACGL031 | 185 | C2L | kelch-like protein |
| VACGL032 | 186 | C1L | unknown |
| VACGL033 | 187 | N1L | virokine |
| VACGL034 | 188 | N2L | alpha-amanitin target |
| VACGL035 | 189 | M1L | ankyrin-like protein |
| VACGL036 | 190 | M2L | unknown |
| VACGL037 | 191 | K1L | ankyrin-like protein |
| VACGL038 | 192 | K2L | serine protease inhibitor-like |
| VACGL041 | 193 | - | "hypothetical protein, orthologous to m0036R[Vaccinia]" |
| VACGL042 | 194 | K3L | interferon resistance protein |
| VACGL043 | 195 | K4L | phospholipase-D-like protein |
| VACGL044 | 196 | - | putative monoglyceride lipase[Vaccinia] |
| VACGL045 | 197 | K5L | putative monoglyceride lipase |
| VACGL046 | 198 | K6L | putative monoglyceride lipase |
| VACGL047 | 199 | K7R | unknown |
| VACGL049 | 200 | F1L | unknown |
| VACGL050 | 201 | F2L | dUTPase |
| VACGL051 | 202 | F3L | kelch-like protein |
| VACGL053 | 203 | F4L | ribonucleotide reductase small subunit |
| VACGL056 | 204 | F5L | unknown |
| VACGL057 | 205 | F6L | unknown |
| VACGL058 | 206 | F7L | unknown |
| VACGL059 | 207 | F8L | protein with iActA-like proline repeats |
| VACGL060 | 208 | F9L | S-S bond formation pathway protein |
| VACGL061 | 209 | F10L | ser/thr kinase |
| VACGL062 | 210 | - | similar to (VacCop) putative F ORF D |
| VACGL063 | 211 | F11L | unknown |
| VACGL064 | 212 | F12L | involved in plaque and EEV formation |
| VACGL066 | 213 | F13L | palmytilated EEV membrane protein |
| VACGL067 | 214 | F14L | unknown |
| VACGL068 | 215 | F14.5L | "F14.5L, hypothetical protein, orthologous to m0062L[Vaccinia]" |
| VACGL069 | 216 | F15L | unknown |
| VACGL070 | 217 | F16L | unknown |
| VACGL071 | 218 | F17R | putative DNA-binding phosphoprotein |
| VACGL073 | 219 | E1L | poly-A polymerase catalytic subunit VP55 |
| VACGL074 | 220 | E2L | unknown |
| VACGL075 | 221 | E3L | double-stranded RNA binding protein |
| VACGL076 | 222 | E4L | DNA-dependent RNA polymerase subunit rpo30 |
| VACGL077 | 223 | E5R | abundant component of virosome |
| VACGL079 | 224 | E6R | unknown |
| VACGL080 | 225 | E7R | "soluble, myristylprotein" |
| VACGL082 | 226 | E8R | membrane protein |
| VACGL084 | 227 | E9L | DNA polymerase |
| VACGL086 | 228 | E10R | sulfhydryl oxidase |
| VACGL087 | 229 | E11L | virion core protein |
| VACGL088 | 230 | O1L | unknown |
| VACGL090 | 231 | - | "unknown protein, orthologous to CPXV078A[Cowpox virus]" |
| VACGL091 | 232 | O2L | nonessential glutaredoxin |
| VACGL092 | 233 | I1L | DNA-binding core protein |
| VACGL093 | 234 | I2L | "hypothetical protein, orthologous to m0086L[Vaccinia]" |
| VACGL094 | 235 | I3L | ssDNA-binding phosphoprotein |
| VACGL095 | 236 | I4L | ribonucleotide reductase large subunit |
| VACGL098 | 237 | I5L | IMV protein VP 13 |
| VACGL099 | 238 | I6L | unknown |
| VACGL100 | 239 | I7L | viral core cysteine proteinase |
| VACGL101 | 240 | I8R | "RNA-helicase, DExH-NPH-II" |
| VACGL102 | 241 | G1L | insulin metalloproteinase-like protein |
| VACGL103 | 242 | G3L | unknown |
| VACGL104 | 243 | G2R | late transcription elongation factor |
| VACGL105 | 244 | G4L | thioredoxin-like protein |
| VACGL106 | 245 | G5R | unknown |
| VACGL107 | 246 | G5.5R | DNA-dependent RNA polymerase subunit rpo7 |
| VACGL108 | 247 | G6R | unknown |
| VACGL109 | 248 | G7L | virion structural protein |
| VACGL111 | 249 | - | similar to (VacCop) putative G ORF B |
| VACGL112 | 250 | G8R | late gene transcription VLTF-1 |
| VACGL113 | 251 | G9R | myristylprotein |
| VACGL114 | 252 | L1R | IMV membrane protein |
| VACGL115 | 253 | L2R | unknown |
| VACGL116 | 254 | L3L | unknown |
| VACGL117 | 255 | L4R | core protein vp8 |
| VACGL118 | 256 | L5R | putative membrane protein |
| VACGL119 | 257 | J1R | virion protein |
| VACGL120 | 258 | J2R | thymidine kinase |
| VACGL121 | 259 | J2R | thymidine kinase |
| VACGL122 | 260 | J3R | multifunctional poly-A polymerase subunit |
| VACGL123 | 261 | J4R | DNA-dependent RNA polymerase subunit rpo22 |
| VACGL124 | 262 | J5L | late 16kDa putative membrane protein |
| VACGL125 | 263 | J6R | DNA-dependent RNA polymerase subunit rpo 147 |
| VACGL127 | 264 | H1L | tyr/ser protein phosphatase |
| VACGL128 | 265 | H2R | unknown |
| VACGL129 | 266 | H3L | IMV heparin binding surface protein |
| VACGL130 | 267 | H4L | RAP94 |
| VACGL131 | 268 | H5R | "morphogenesis-related, substrate of B1R kinase" |
| VACGL132 | 269 | H6R | topoisomerase type IB |
| VACGL133 | 270 | - | "unknown, orthologous to CPXV116[Cowpox virus]" |
| VACGL134 | 271 | H7R | unknown |
| VACGL135 | 272 | D1R | large subunit of mRNA capping enzyme |
| VACGL137 | 273 | D2L | virion core protein |
| VACGL139 | 274 | D3R | virion core protein |
| VACGL140 | 275 | D4R | uracil-DNA glycosylase |
| VACGL141 | 276 | - | similar to (VacCop) putative D ORF C |
| VACGL142 | 277 | D5R | NTPase interacts with A20R |
| VACGL145 | 278 | D6R | 70kDa small subunit of early gene transcription factor VETF |
| VACGL147 | 279 | D7R | DNA-dependent RNA polymerase subunit rpo 18 |
| VACGL148 | 280 | D8L | IMV membrane protein |
| VACGL149 | 281 | D9R | contains mutT-like motif of NTP-phosphohydrolase for DNA repair |
| VACGL150 | 282 | D10R | contains mutT-like motif of NTP-phosphohydrolase for DNA repair |
| VACGL151 | 283 | D11L | "ATPase, nucleoside triphosphate phosphohydrolase-I, NPH-I" |
| VACGL155 | 284 | D12L | small subunit ofmRNA capping enzyme |
| VACGL157 | 285 | - | "unknown, orthologous to unknown protein[Tian tan]" |
| VACGL158 | 286 | D13L | rifampicin target |
| VACGL160 | 287 | A1L | late gene transcription factor VLTF-2 |
| VACGL161 | 288 | A2L | late gene transcription factor VLTF-3 |
| VACGL162 | 289 | A2.5L | S-S bond formation pathway |
| VACGL163 | 290 | A3L | p4b precursor of core protein 4b |
| VACGL165 | 291 | A4L | 39kDa core protein |
| VACGL167 | 292 | A5R | DNA-dependent RNA polymerase subunit rpo 19 |
| VACGL168 | 293 | A6L | unknown |
| VACGL169 | 294 | A7L | 82kDa large subunit of early gene transcription factor VETF |
| VACGL172 | 295 | A8R | 32kDa small subunit of transcription factor VITF-3 |
| VACGL173 | 296 | A9L | IMV membrane protein |
| VACGL174 | 297 | A10L | precursor p4a of core protein 4a |
| VACGL178 | 298 | A11R | unknown |
| VACGL179 | 299 | A12L | core protein |
| VACGL180 | 300 | A13L | IMV membrane protein |
| VACGL181 | 301 | A14L | phosphorylated IMV membrane protein |
| VACGL182 | 302 | A14.5L | nonessential hydrophobic IV amd IMV membrane protein[Vaccinia] |
| VACGL183 | 303 | A15L | unknown |
| VACGL184 | 304 | A16L | soluble myristylprotein |
| VACGL185 | 305 | A17L | IMV membrane protein |
| VACGL186 | 306 | A18R | DNA helicase |
| VACGL187 | 307 | A19L | unknown |
| VACGL188 | 308 | A21L | unknown |
| VACGL189 | 309 | A20R | viral DNA polymerase processivity factor |
| VACGL192 | 310 | A22R | palmitylprotein |
| VACGL193 | 311 | A23R | 45kDa large subunit of intermediate gene transcription factor VITF-3 |
| VACGL194 | 312 | A24R | DNA-dependent RNA polymerase subunit rpo132 |
| VACGL196 | 313 | A25L | DNA-directed RNA polymerase subunit[Vaccinia] |
| VACGL197 | 314 | - | cowpox A-type inclusion protein |
| VACGL199 | 315 | - | cowpox A-type inclusion protein |
| VACGL201 | 316 | A26L | cowpox A-type inclusion protein |
| VACGL203 | 317 | A27L | IMV surface protein |
| VACGL204 | 318 | A28L | unknown |
| VACGL205 | 319 | A29L | DNA-dependent RNA polymerase rpo35 |
| VACGL206 | 320 | - | similar to (VacCop) putative A ORF K |
| VACGL207 | 321 | A30L | IMV protein |
| VACGL208 | 322 | A31R | unknown |
| VACGL209 | 323 | A32L | putative ATPase |
| VACGL211 | 324 | A33R | EEV membrane phosphoglycoprotein |
| VACGL212 | 325 | A34R | EEV glycoprotein. |
| VACGL213 | 326 | - | similar to (VacCop) putative A ORF M |
| VACGL214 | 327 | A35R | unknown |
| VACGL215 | 328 | A36R | IEV transmembrane phosphoprotein |
| VACGL216 | 329 | A37R | unknown |
| VACGL218 | 330 | - | unknown |
| VACGL220 | 331 | A38L | CD47-like putative membrane protein |
| VACGL221 | 332 | A39R | similar to (VacCop) putative A39R |
| VACGL223 | 333 | A40R | C-type lectin-like type-II membrane protein |
| VACGL224 | 334 | A41L | secreted glycoprotein |
| VACGL225 | 335 | A42R | profilin-like protein |
| VACGL226 | 336 | A43R | putative type-1 membrane glycoprotein |
| VACGL227 | 337 | 268 | "hypothetical protein, orthologous to m0215[Vaccinia]" |
| VACGL228 | 338 | A44L | hydroxysteroid dehydrogenase |
| VACGL229 | 339 | A45R | inactive Cu-Zn superoxide dismutase-like in virion |
| VACGL230 | 340 | A46R | Toll/IL 1-receptor |
| VACGL232 | 341 | A47L | unknown |
| VACGL233 | 342 | - | "unknown, orthologs to unknown protein[monkeypox virus]" |
| VACGL234 | 343 | A48R | thymidylate kinase |
| VACGL235 | 344 | A49R | unknown |
| VACGL236 | 345 | A50R | DNA ligase |
| VACGL239 | 346 | A51R | unknown |
| VACGL240 | 347 | A52R | Toll/IL1-receptor |
| VACGL241 | 348 | A53R | Tumor necrosis factor receptor[Vaccinia] |
| VACGL243 | 349 | - | putative protein orthologous to CPXV192[Camelpox virus] |
| VACGL244 | 350 | A55R | kelch-like protein |
| VACGL245 | 351 | A56R | hemagglutinin |
| VACGL246 | 352 | A57R | guanylate kinase |
| VACGL247 | 353 | B1R | ser/thr kinase |
| VACGL249 | 354 | B2R | unknown |
| VACGL251 | 355 | - | similar to (VacCop) putative B ORF C |
| VACGL252 | 356 | B3R | unknown |
| VACGL253 | 357 | - | unknown[Vaccinia] |
| VACGL255 | 358 | B4R | ankyrin-like protein |
| VACGL256 | 359 | B5R | EEV type-I membrane glycoprotein |
| VACGL257 | 360 | B6R | ankyrin-like protein |
| VACGL259 | 361 | B7R | 21kDa precursor protein |
| VACGL260 | 362 | B8R | soluble interferon-gamma receptor-like protein |
| VACGL261 | 363 | - | Potential protein orthologous to RPXV 171 [Rabbitpox virus] |
| VACGL262 | 364 | B9R | 6kDa intracellular viral protein |
| VACGL263 | 365 | B10R | unknown |
| VACGL264 | 366 | B11R | unknown |
| VACGL265 | 367 | B12R | ser/thr protein kinase-like protein |
| VACGL266 | 368 | B13R | "SPI-2/CrmA inhibits Fas-mediated apoptosis, IL-1 convertase, lipoxygenase pathway" |
| VACGL267 | 369 | B14R | "SPI-2/CrmA inhibits Fas-mediated apoptosis, IL-1 convertase, lipoxygenase pathway" |
| VACGL268 | 370 | B15R | unknown |
| VACGL270 | 371 | B16R | IL-1-beta-inhibitor |
| VACGL272 | 372 | B17L | unknown |
| VACGL273 | 373 | B18R | ankyrin-like protein |
| VACGL274 | 374 | B19R | IFN-alpha/beta-receptor-like secreted glycoprotein |
| VACGL275 | 375 | B20R | similar to (VacCop) putative B20R |
| VACGL277 | 376 | - | interleukin-18-binding protein |
| VACGL278 | 377 | - | zinc finger-like protein |
| VACGL279 | 378 | - | zinc finger-like; apoptosis |
| VACGL280 | 379 | C10L | unknown |
| VACGL282 | 380 | C11R | secreted epidermal growth factor-like protein |
| VACGL283 | 381 | C12L | serine protease inhibitor-like SPI-1 |
| VACGL284 | 382 | B23R/C17L | similar to (VacCop) putative C17L |
| VACGL285 | 383 | B23R/C17L | similar to (VacCop) putative C17L |
| VACGL286 | 384 | B24R/C18L | similar to (VacCop) putative C18L |
| VACGL287 | 385 | B28R/C22L | TNF-alpha-receptor-like protein |
| VACGL288 | 386 | - | fragement of tumor necrosis factor receptor II[Cowpox] |
| VACGL289 | 387 | B29R/C23L | chemokine-binding protein |
| lacZ | 388 | - | *E. coli* beta-galactosidase |
| gusA | 389 | - | *E. coli* beta-glucuronidase |
| ruc-gfp | 390 | - | renilla luciferase-green fluorescent protein fusion |

With this array, it was possible to compare the expression of VACV transcripts *in vivo* in the responding xenograft tumor, GI-101A, and the two non-responding xenografts tumors, HT-29 (characterized by normal *in vitro* but delayed *in vivo* replication) and PC-3 (characterized by delayed *in vitro* but intermediate *in vivo* replication). In all cases, there was a correlation between *Ruc-GFP* transcript expression and the overall expression of VACV genes independent of cell line analyzed, suggesting that the exogenous construct accurately represented GLV-1h68 replication. Furthermore, although some variation in VACV gene expression was observed among cell lines or among individual experiments using the same cell line, a clear dichotomy was observed between replicating and non-replicating cases. The overall VACV transcriptional pattern correlated to viral titers observed *in vivo* (Table 9). For example, most GI-101A xenografts demonstrated replication with three out of four samples expressing VACV genes at day 7, and four out of four samples at days 21 and 42. In contrast, HT-29 and PC-3 displayed delayed replication *in vivo* with only a small proportion of xenografts displaying full VACV gene expression at day 21 (two out of four in either case). After 42 days the expression of VACV genes was turned on in all four PC-3 xenografts and in only one of four HT-29 xenografts, consistent with direct viral load analysis. These data suggest that delayed *in vivo,* but not complete lack of, replication is a predictor of *in vivo* outcome.

For the array analysis on the *in vitro* infected tumor cell samples, VACV gene expression analysis confirmed a lack of differences in the transcriptional pattern of *in vitro* replication between HT-29 and GI-101A with two out of three cell cultures demonstrating active viral replication in either case in the first 24 hours after infection. The transcriptional pattern associated with PC-3 replication *in vitro* at 24 hours was not tested by the array platform since it was clearly absent according to viral load and *Ruc-GFP* analysis.

The kinetics of VACV gene expression in xenografts were measured by a time-course analysis performed on BRB-Array tool based on a time threshold p-value < 0.001 and a false discovery rate of 0.1. Differences in VACV gene expression between non-responding (HT-29 and PC-3) and responding xenografts (GI-101A) were observed 21 days after intravenous injection of GLV-1h68. At that time point, only three out of eight non responding xenografts (one HT-29 and two PC-3) demonstrated active replication compared with four out of four xenografts derived from GI-101A (Fisher test p-value = 0.03). Consistent with these results, a Student's *t* test comparing the number of VACV genes differentially expressed between xenografts at day 21 or 42 with baseline conditions (uninfected xenografts or xenografts excised from mice infected only 24 hours prior) identified significant differences (multivariate permutation p-value < 0.001) only in GI-101 A xenografts at day 21, while significant differences were observed in PC-3 xenogafts at day 42 only. Table 12 shows the number of VACV-genes differentially expressed between baseline and 21 or 42 days in the three exemplary xenografts (Student's *t* test cutoff <0.001, multivariate permutation test p-value is shown).

**Table 12**

| **Experimental Groups** | | **Probe #** | **gene #** | **Permutation Test** |
|---|---|---|---|---|
| | **House keeping VACV** | **393** | **337** | **p-value** |
| | | **308** | **219** | |
| GI-101A | House keeping VACV | 3 | 2 | n.s |
| 7 days | | 0 | 0 | n.s |
| GI-101A | House keeping VACV | 261 | 232 | <0.001 |
| 21 days | | 307 | 219 | <0.001 |
| GI-101A | House keeping VACV | 267 | 237 | <0.001 |
| 42 days | | 299 | 216 | <0.001 |
| HT-29 | House keeping VACV | 3 | 3 | n.s |
| 21 days | | 0 | 0 | n.s |
| HT-29 | House keeping VACV | 10 | 10 | n.s |
| 42 days | | 0 | 0 | n.s |
| PC-3 | House keeping VACV | 38 | 38 | n.s |
| 21 days | | 0 | 0 | n.s |
| PC-3 | House keeping VACV | 52 | 50 | n.s |
| 42 days | | 201 | 195 | <0.001 |

| | | | | |
|---|---|---|---|---|
| n.s.= not significant | | | | |

The number of genes differentially expressed in replicating tumors reflected almost completely the number of probes and annotations present in the array platform (308 and 219) respectively, demonstrating that GLV-1h68 replication is either absent or complete in xenografts. A near complete overlap of VACV probes or genes expressed at day 21 and 42 was observed in the GI-101A xenografts. A reverse behavior was observed in the pattern of expression of human house keeping genes represented in the VACV array platform; these genes were significantly down-regulated in permissive cell lines, suggesting a shut off of cellular metabolism in virally infected cells that correlated inversely with viral transcription as previously described (Guerra, S. et al. (2007) J Virol. 81:8707-8721).

### 2. Transcriptional differences between responding xenografts versus non-responding xenografts to systemic GLV- 1h68 administration: human cancer signatures

A time course analysis evaluating the *in vivo* effects of viral replication on the permissive GI-101 A human xenografts was performed using a previously described custom-made 17.5k human cDNA array platform (Panelli, M.C. et al. (2006) Genome Biol 8:R8. Four experimental groups were tested that included animals receiving systemic GLV-1h68 administration 1, 7, 21 and 42 days before xenograft excision; 4 animals were tested for each experimental group. As expected based on the analysis of viral replication *in vivo,* significant changes in the transcriptional profile of infected tumors occurred only 21 days after GLV-1h68 administration and increased at 42 days. Since the time course demonstrated that in permissive xenografts the most significant changes occurred only after 21 days, the analysis of xenografts representative of non-responding tumors was limited to days 21 and 42. It was previously observed that the use of species-specific cDNA arrays as well as oligo probes can distinguish the expression patterns in mixed cell populations in which human tissues (cancer cells) are infiltrated with host normal cells (Zhang, Q. et al. (2007) Cancer Res. 67:10038-10046). This is due to a lack or reduced cross-hybridization between non-related species was comparable to closely related ones, such as primate to primate comparisons. Although partial cross-hybridization may occur, this can be flagged and eliminated by applying an appropriate intensity signal cutoff. Since cDNA arrays contain probes of relatively large size (600 to 2,000 bases), to increase the specificity of the hybridization, the same material was tested on custom-made 36 kb oligo array platforms, constituting 70-base-length oligo-probes (Operon) as well as cDNA probes using identical statistical parameters. The results were concordant between platforms.

To test whether delayed replication affected the transcriptional program of cancer cell lines, the transcriptional profile of responding (GI-101A) and non-responding (HT-29) xenografts was compared. Comparisons were made between the transcriptional profiles of infected and non-infected GI-101A and HT-29 xenografts at days 21 and 42. HT-29 was selected among the non-responding tumor cell lines because of the different behavior observed in *in vivo* experiments compared to the responding GI-101A. An overview of the global differences among experimental conditions was provided by multiple dimensional scaling based on the complete data set of 36K oligo probes. This analysis demonstrated that infected GI-101A xenografts completely segregated in Euclidian space from non infected xenografts, while HT-29 xenografts clustered together whether or not they received GLV-1h68 treatment. Similar results were observed based on the cDNA-based array platform.

To test overall differences between xenografts from infected and non-infected animals, a Student's *t* test (cutoff p₂-value <0.001) comparing GI-101A infected xenografts to non-infected xenografts and HT-29 101A infected xenografts to non-infected xenografts was applied. Comparison of GI-101A xenografts identified 1,073 genes differentially expressed between infected and non-infected xenografts at the level of significance (permutation test p value = 0). By contrast, only 9 genes were found to be differentially expressed by HT-29 xenografts excised from infected compared to non-infected animals at the same statistical stringency (permutation test non significant). Among the genes differentially expressed in the GI-101A xenografts excised from GLV-1h68 infected animals, the large majority were down-regulated, particularly, in xenografts excised at day 42 suggesting that, as observed *in vitro,* viral replication induces depression of cellular function. A smaller cluster of genes was specifically over-expressed by GI-101A xenografts from infected animals. Among these genes, allograft inflammatory factor-1 (AIF-1), tissue inhibitor of metalloproteinase 2 (TIMP-2) and the IL-2 receptor common γ chain were found to be strongly up regulated.

A multivariate analysis (F test, p-value cutoff < 0.001) comparing the four groups at days 21 and 42 (HT-29 and GI-101A in infected and non-infected mice) identified 2,241 and 1,984 clones, respectively, that were differentially expressed among the four groups based on the oligo arrays. Comparison of the 17k cDNA arrays similarly identified 1,467 cDNA clones representative of the four groups at day 42. In either platform, most of the differences in expression pattern were tumor cell specific and segregated the HT-29 xenografts from GI-101A xenografts independent of GLV-1h68 administration. However, a subgroup of genes was observed to be specific for GI-101A infected xenografts (exemplified using the cDNA array platform displaying 149 clones. The GLV-1h68 infection-specific signatures were enriched of genes associated with immune function (35 genes) with a significantly higher than expected frequency (1.88) according to Genontology assignment of biological processes. Among the genes up-regulated in the in GI-101A xenografts excised from GLV-1h68 infected mice, several were strongly associated with activation of innate immune mechanisms including the Toll-like receptor (TLR)-2, the interferon regulatory factor (IRF)-7, signal transducer and activator of T cell (STAT)-3 and tumor necrosis factor (TNF)-α This enrichment was not as clearly observed in the oligo array based arrays, suggesting that these signatures could be potentially attributed to host infiltrating immune cells whose genes could cross-hybridize to the less stringent cDNA array probes.

### 3. Transcriptional differences between responding xenografts versus non-responding xenografts to systemic GLV-1h68 administration: mouse host signatures

To further define the host involvement in the oncolytic process, HT-29 and GI-101A xenografts were analyzed using a custom-made, whole genome mouse array platform. All four GI-101A xenografts excised at day 42 from infected mice were utilized, while only three of four xenograft were utilized for the human arrays described above due to degradation of human mRNA in one of the regressing xenografts. Gene expression was only significantly affected in GI-101A xenografts excised from GLV-1h68 infected mice (see Table 13). This modulation was particularly evident at day 42 when 768 genes were altered in expression in GI-101A xenografts excised from GLV-1h68 infected animals.

**Table 13**

| **Genes differentially expressed between xenografts excised from GLV-1h68-infected vs non-infected animals** | | | | |
|---|---|---|---|---|
| (Cut off p₂-value < 0.001 (unpaired Student *t* test) | | | | |
| 36k whole genome mouse array platform | | | | |
| | | | | |

| Day | Experimental Group | # genes | Permutation test | |
|---|---|---|---|---|
| | | | | |
| 21 | HT-29 | 0 | N.S. | |
| 42 | HT-29 | 6 | N.S. | |
| 21 | GI-101A | 88 | <0.01 | |
| 42 | GI-101A | 768 | <0.001 | |

A statistical overview of gene expression modulation of GI-101A xenografts from GLV-1h68 infected grafts gave an opposite picture compared with that obtained with the human arrays. Most mouse genes where up regulated in xenografts excised from infected animals suggesting that, while the metabolism of cancer cell was declining, the activation of host cells was actively enhanced. An F test was performed to compare xenogafts at days 21 and 42. At day 21, 1,066 genes demarcated the differences among the four experimental groups. This number increased to 1,471 by day 42 (permutation test p-value = 0 in either case).

Genes up-regulated in GI-101A xenografts excised from infected animals included several genes with immune effector function including several lymphokines, chemokines and interferon-stimulated genes (ISGs) (see Table 14, I11 8bp (SEQ ID NO:406), I118 (SEQ ID NO:407), I115 (SEQ ID NO: 124), I110ra (SEQ ID NO:408), Cxcl 11 (SEQ ID NO:409), Cxcl9 (SEQ ID NO:410), Cxcl12 (SEQ ID NO:411), Ccl5 (SEQ ID NO:142), Ccl9 (SEQ ID NO:46), Ccl7 (SEQ ID NO:412), Ccl27 (SEQ ID NO:413), Igtp (SEQ ID NO:474), Ifi27 (SEQ ID NO:414), Ifi47 (SEQ ID NO:475), Iigp2 (SEQ ID NO:476), Mx1 (SEQ ID NO:415), Ifi204 (SEQ ID NO:477), Irf1 (SEQ ID NO:416), Stat1 (SEQ ID NO:417), Ifit1 (SEQ ID NO:418), Iigp1 (SEQ ID NO:478), Ifnar2 (SEQ ID: NO 419), Irf5 (SEQ ID NO:420), Stat3 (SEQ ID NO:421), Ly6f (SEQ ID NO:479), Aif1 (SEQ ID NO:422), Ly6c (SEQ ID NO:480), Ripk1 (SEQ ID NO:423), Sell (SEQ ID NO:424), Tax1bp (SEQ ID NO:425), Ikbkap (SEQ ID NO:426), Ly6a (SEQ ID NO:481), Ctla2b (SEQ ID NO:482), Arts1 (SEQ ID NO:427), Nkiras2 (SEQ ID NO:428), Ly96 (SEQ ID NO:429), Ly6e (SEQ ID NO:430), and Tlr2 (SEQ ID NO:431)).

Among the cytokines, IL-18 and the IL-18 binding protein appeared to play a prominent role, while IL-15 was also strongly up regulated. Several CXCR-3, CXCR4 and CCR5 ligands were up regulated among chemokines, suggesting a strong pro-inflammatory switch capable of recruiting activated natural killer cells. Among them, the expression of CXC-12/SDF-1 was previously reported to be associated with the rejection of metastatic melanoma during IL-2 therapy (Wang, E. et al. (2002) Cancer Res. 62:3581-3586). A large number of ISGs predominantly associated with interferon-alpha (IFN-α) function were also up regulated including IRF-1, also previously described in association with rejection of melanoma metastases during IL-2 based immunotherapy. ISGs were among the most up-regulated genes including interferon-γ-induced GTPase, whose expression was increased 48-fold in GI-101A tumors excised from GLV-1h68-infected animals compared with control xenografts. TLR-2, AIF-1 and STAT-3 were also found to be up-regulated according to the mouse array platform similarly to the findings in the human platform suggesting a cross hybridization of these genes likely expressed by host immune cells.

**Table 14**

| **Immune genes up-regulated in regressing GI-101A tumors (F test p₂-value <0.001)** | | | | | | | |
|---|---|---|---|---|---|---|---|
| **Gene** | | | | **HT-29 Control** | **HT-29 GLV-1h68** | **GI-101A Control** | **GI-101 GLV-1h** |
| | **ID#** | **Symbol** | **Name** | | | | |
| | | | | | | | |

| **Interleukins and Receptors** | | | | | | | |
|---|---|---|---|---|---|---|---|
| | 16068 | II18bp | interleukin 18 binding protein | 1.31 | 2.18 | 1.00 | 13.28 |
| | 16173 | II18 | interleukin 18 | 1.12 | 1.40 | 1.00 | 10.89 |
| | 16168 | II15 | interleukin 15 | 1.02 | 1.51 | 1.00 | 5.20 |
| | 16154 | II10ra | interleukin 10 receptor alpha | 0.74 | 0.90 | 1.00 | 3.51 |
| | | | | | | | |

| **Chemokines** | | | | | | | |
|---|---|---|---|---|---|---|---|
| | | Cxcl11 | Cxcl11/I-TAC | 0.92 | 1.75 | 1.00 | 13.57 |
| | 17329 | Cxcl9 | Cxcl9/Mig | 1.01 | 1.07 | 1.00 | 11.74 |
| | 20315 | Cxcl12 | Cxcl12/SDF-1/PBSF | 0.41 | 0.58 | 1.00 | 5.23 |
| | 20304 | Ccl5 | Ccl5/RANTES | 1.00 | 2.69 | 1.00 | 13.33 |
| | 20308 | Ccl9 | Ccl9/MRP-2/CCF18/MIP-1γ | 1.56 | 3.14 | 1.00 | 12.03 |
| | 20304 | Ccl5 | Ccl5/RANTES | 1.11 | 2.57 | 1.00 | 9.81 |
| | 20306 | Ccl7 | Ccl7/MARC | 0.84 | 1.18 | 1.00 | 5.86 |
| | 20301 | Ccl27 | Ccl27/ALP/CTACK/ILC/Eskine | 1.86 | 1.91 | 1.00 | 5.17 |
| | 20308 | Ccl9 | Ccl9/MRP-2/CCF18/MIP-1γ | 1.28 | 1.42 | 1.00 | 4.04 |
| | | | | | | | |

| **ISGs** | | | | | | | |
|---|---|---|---|---|---|---|---|
| | 16145 | Igtp | interferon gamma induced GTPase | 1.15 | 3.31 | 1.00 | 48.21 |
| | 76933 | Ifi27 | interferon alpha-inducible protein 27 | 0.76 | 0.91 | 1.00 | 12.84 |
| | | Ifi47 | interferon gamma inducible protein 47 | 0.66 | 0.94 | 1.00 | 11.09 |
| | | ligp2 | interferon inducible GTPase 2 | 0.64 | 1.41 | 1.00 | 10.05 |
| | 16145 | Igtp | interferon gamma induced GTPase | 0.70 | 1.28 | 1.00 | 9.70 |
| | 17857 | Mx1 | myxovirus (influenza virus) resistance 1 | 0.62 | 1.46 | 1.00 | 9.46 |
| | | Ifi204 | interferon activated gene 204 | 0.86 | 1.77 | 1.00 | 8.94 |
| | 16362 | Irf1 | interferon regulatory factor 1 | 0.59 | 0.98 | 1.00 | 7.28 |
| | 20846 | Stat1 | signal transducer and activator of transcription 1 | 0.57 | 0.81 | 1.00 | 6.84 |
| | 20846 | Stat1 | signal transducer and activator of transcription 1 | 0.66 | 0.97 | 1.00 | 6.17 |
| | 15957 | Ifit1 | interferon-induced protein with tetratrieopeptide repeats 1 | 0.79 | 1.03 | 1.00 | 5.77 |
| | 60440 | ligp1 | interferon inducible GTPase 1 | 0.77 | 1.00 | 1.00 | 4.19 |
| | 15976 | Ifnar2 | interferon (alpha and beta) receptor 2 | 1.10 | 1.60 | 1.00 | 3.73 |
| | | Irf5 | interferon regulatory factor 5 | 0.53 | 0.70 | 1.00 | 3.47 |
| | 20848 | Stat3 | signal transducer and activator of transcription 3 | 1.16 | 1.17 | 1.00 | 2.55 |
| | | | | | | | |

| **Other** | | | | | | | |
|---|---|---|---|---|---|---|---|
| | 17071 | Ly6f | Lymphocyte antigen 6 complex, locus F | 0.56 | 0.73 | 1.00 | 8.56 |
| | 11629 | Aif1 | allograft inflammatory factor 1 | 0.90 | 1.33 | 1.00 | 8.46 |
| | 17067 | Ly6c | Lymphocyte antigen 6 complex, locus C | 1.24 | 1.22 | 1.00 | 7.35 |
| | | Ripk1 | receptor (TNFRSF)-interacting serine-threonine kinase 1 | 0.82 | 1.42 | 1.00 | 6.97 |
| | 17067 | Ly6c | lymphocyte antigen 6 complex, locus C | 0.99 | 1.12 | 1.00 | 6.03 |
| | 17071 | Ly6f | lymphocyte antigen 6 complex, locus F | 0.80 | 0.89 | 1.00 | 5.66 |
| | 20343 | Sell | selectin, lymphocyte | 0.61 | 0.79 | 1.00 | 5.03 |
| | 76281 | Tax1bp1 | Tax1 (human T-cell leukemia virus type I) binding protein 1 | 1.01 | 1.51 | 1.00 | 5.02 |
| | 230233 | Ikbkap | inhibitor of kappa light polypeptide enhancer in B-cells | 0.81 | 0.93 | 1.00 | 4.55 |
| | 110454 | Ly6a | lymphocyte antigen 6 complex, locus A | 0.96 | 0.98 | 1.00 | 4.13 |
| | 13025 | Ctla2b | Cytotoxic T lymphocyte-associated protein 2 beta | 0.68 | 1.20 | 1.00 | 4.12 |
| | | Arts1 | type 1 tumor necrosis factor receptor shedding aminopeptidase regulator | 0.73 | 1.31 | 1.00 | 3.87 |
| | 71966 | Nkiras2 | NFKB inhibitor interacting Ras-like protein 2 | 1.07 | 1.27 | 1.00 | 3.80 |
| | 17087 | Ly96 | lymphocyte antigen 96 | 1.13 | 1.43 | 1.00 | 3.77 |
| | 17069 | Ly6e | Lymphocyte antigen 6 complex, locus E | 0.77 | 1.15 | 1.00 | 3.28 |
| | 24088 | Tir2 | toll-like receptor 2 | 0.25 | 0.36 | 1.00 | 1.60 |

### 4. Delayed in vitro replication of GLV-1h68 can be predicted by specific transcriptional signatures suggestive of intrinsic activation of anti-viral mechanisms in the non-permissive cancer cell lines

From the *in vivo* studies; it could be concluded that GLV-1h68 administration to human cancer xenograft-bearing mice resulted in a dichotomy of expression of viral genes that in turn resulted in differences in the expression patterns within the cancer cell lines with a strong reduction of cellular metabolism in presence of viral replication. These changes were also responsible for the activation of powerful innate immune mechanisms, suggesting that tumor eradication is at least in part due to the activation of immune effector mechanism. As described above, *in vitro* behavior of non-permissive cell lines was a strong predictor of *in vivo* behavior, where delayed replication of GLV-1h68 *in vitro* was consistently associated with lack of *in vivo* responsiveness of the corresponding xenografts. This in turn could decrease the *in vivo* ability of GLV-1h68 to control tumor growth either through oncolytic or immunological mechanisms. The expression profile of the different cancer cell lines *in vitro* in base line conditions (in the absence of GLV-1h68) and during the active phases of GLV-1h68 replication were examined. The following time points were compared for each cell line in the presence or absence of virus: 0, 3 and 12 hours.

GLV-1h68 infection induced several changes in gene expression that were time dependent and tightly correlated with the expression of VACV-genes. In particular, a shut off of several host cell genes was observed with time in accordance with previous studies (Guerra, S. et al. (2007) J Virol. 81:8707-8721). The most significant differences between the cell lines that allowed or inhibited early replication were noted in non-infected controls. Non-infected controls behaved similarly at the three *in vitro* culture time points (0, 3 and 12 hours) and no statistically significant differences could be identified among the three time points. Therefore, their transcriptional profile was analyzed together when comparing different cell lines. A class comparison was performed based on an unpaired Student's *t* test between non-permissive (MDA-231, NCI-H1229, SiHa, PC-3) and permissive (888-MEL, 1858-MEL, 1936-MEL, GI-101A, MIA PaCa-2, HT-29 and A549) cell lines; OVCAR-3 and PANC-1 were not included in the Student's *t* test analysis because of their intermediate behavior in allowing GLV-1h68 replication in culture. This analysis identified 1,736 genes that were differentially expressed between permissive and non-permissive cell lines at a p-value cut off of 0.01 (permutation test p-value = 0). The most specific signatures were obtained for the constitutive expression of genes that characterized the non-permissive cell lines. It was observed that non-permissive cell lines expressed a significant number of interferon-stimulated genes (ISGs) and other immune regulatory genes based on Gene Ontology classification, while such genes were completely absent in the permissive cell lines (Table 15, IL6 (SEQ ID NO:117), IL13RA1 (SEQ ID NO:432), IL8 (SEQ ID NO:119), IL7R (SEQ ID NO:433), CCRL2 (SEQ ID NO:434), CCL5 (SEQ ID NO:142), IFIT1 (SEQ ID NO:418), EBI3 (SEQ ID NO:435), interferon-induced transmembrane protein 3(SEQ ID NO:436), IFIT1 (SEQ ID NO:418), IFNGR2 (SEQ ID NO:437), IFITM2 (SEQ ID NO:438), MX1 (SEQ ID NO:415), STAT1 (SEQ ID NO:417), IFITM1 (SEQ ID NO:439), OASL (SEQ ID NO:440), IRF7 (SEQ ID NO:441), IRF1 (SEQ ID NO:442), NGFRAP1 (SEQ ID NO:443), TGFB1I1 (SEQ ID NO:444), TAX1BP3 (SEQ ID NO:445), BAX (SEQ ID NO:446), PTX3 (SEQ ID NO:447), BAG5 (SEQ ID NO:448), TIMP1 (SEQ ID NO:449), IER3 (SEQ ID NO:450), NFAT5 (SEQ ID NO:451), BAT5 (SEQ ID NO:452), TNFRSF9 (SEQ ID NO:453), LY6G6E (SEQ ID NO:454), CSF2 (SEQ ID NO:455), TNFSF10 (SEQ ID NO:456), CRLF2 (SEQ ID NO:457), LTB4R (SEQ ID NO:458), B2M (SEQ ID NO:459), GBP2 (SEQ ID NO:460), PBX4 (SEQ ID NO:461), NFKBIA (SEQ ID NO:462), HLA-C (SEQ ID NO:463), HLA-H (SEQ ID NO:464), HLA-G (SEQ ID NO:465), BCL6 (SEQ ID NO:466), HLA-F (SEQ ID NO:467), HLA-B (SEQ ID NO:468), HLA CLASS 1 HISTOCOMPATIBILITY ANTIGEN, ALPHA CHAIN F (SEQ ID NO:469), BAG3 (SEQ ID NO:470), HLA-DMA (SEQ ID NO:471), TRBC1 (SEQ ID NO:472), HLA-DRB4 (SEQ ID NO:473)).

Thus, it was concluded that non-permissive cell lines delay viral replication during the initial 24 hours through a constitutive activation of innate immune responses. In particular, genes associated with the initiation of anti-viral immune responses were observed to be constitutively expressed, including MX1, NF*k*BIA, STAT-1, IL-6, CXCL-8 (IL-8), CCL5 (RANTES), and several interferon regulatory factors (IRFs) including IRF-1 and IRF-7 while IRF-4, a classic inhibitor of TLR signaling and pro-inflammatory cytokine production downstream of MyD88 was the only ISG relatively under-expressed by non-permissive cells compared to permissive cells. Since GLV-1h68 replication *in vitro* is only dependent upon the interaction between the virus and the host cell without the participation of any immune mechanisms, the intracellular immune mechanism or paracrine cross talk among cancer cells may be sufficient to limit the ability of GLV-1h68 to replicate during the first 24 hours.

**Table 15**

| **Immune genes up-regulated in non-permissive tumors (F test p₂-value <0.001)** | | | | | |
|---|---|---|---|---|---|
| **Gene** | | | | **Non-permissive cells Control** | **Permissive c Control** |
| | **ID #** | **Symbol** | **Name** | | |
| | | | | | |

| **Interleukins and Receptors** | | | | | |
|---|---|---|---|---|---|
| | 3569 | IL6 | interleukin 6 (interferon, beta 2) | 2.23 | 0.64 |
| | 3597 | IL13RA1 | interleukin 13 receptor, alpha 1 | 2.02 | 0.99 |
| | 3576 | IL8 | interleukin 8 | 0.27 | 0.06 |
| | 3575 | IL7R | PREDICTED: interleukin 7 receptor | 0.09 | 0.04 |
| | | | | | |

| **Chemokines** | | | | | |
|---|---|---|---|---|---|
| | 9034 | CCRL2 | chemokine (C-C motif) receptor-like 2 | 1.15 | 0.68 |
| | 6352 | CCL5 | chemokine (C-C motif) ligand 5 | 0.10 | 0.06 |
| | | | | | |

| **ISGs** | | | | | |
|---|---|---|---|---|---|
| | 3434 | IFIT1 | interferon-induced protein with tebatricopeptide repeats 1 | 4.76 | 0.69 |
| | 10148 | EBI3 | Epstein-Barr virus induced gene 3 | 1.93 | 1.20 |
| | | | LOC144383-PREDICTED: similar to interferon-induced transmembrane protein 3 | 1.74 | 0.39 |
| | 3460 | IFNGR2 | interferon gamma receptor 2 (interferon gamma transducer 1) | 1.35 | 0.86 |
| | 10581 | IFITM2 | interferon induced transmembrane protein 2 | 1.26 | 0.47 |
| | 10581 | IFITM2 | interferon induced transmembrane protein 2 | 1.18 | 0.25 |
| | 4599 | MX1 | myxovirus (influenza virus) resistance 1, interferon-inducible protein p78 | 0.76 | 0.24 |
| | 6772 | STAT1 | signal transducer and activator of transcription 1 | 0.65 | 0.22 |
| | | IFITM1 | interferon induced transmembrane protein 1 | 0.62 | 0.04 |
| | 8638 | OASL | 2'-5'-oligoadenylate synthetase-like | 0.50 | 0.23 |
| | 3665 | IRF7 | interferon regulatory factors 7 | 0.35 | 0.14 |
| | 3659 | IRF1 | interferon regulatory factor 1 | 0.31 | 0.19 |
| | | | | | |

| **Other** | | | | | |
|---|---|---|---|---|---|
| | | | | | |
| | 27018 | NGFRAP1 | nerve growth factor receptor (TNFRSF16) associated protein 1 | 6.74 | 2.22 |
| | 7041 | TGFB1I1 | transforming growth factor beta 1 induced transcript 1 | 3.46 | 1.39 |
| | 30851 | TAX1BP3 | Tax1 (human T-cell leukemia virus type I) binding protein 3 | 3.16 | 1.23 |
| | 581 | BAX | BCL2-associated X protein | 3.14 | 1.75 |
| | 5806 | PTX3 | pentraxin-related gene, rapidly induced by IL-1 beta | 3.10 | 0.98 |
| | 9529 | BAG5 | BCL2-associated athanogene 5 | 2.67 | 1.31 |
| | 7076 | TIMP1 | TIMP metallopeptidase inhibitor 1 | 2.02 | 0.43 |
| | 8870 | IER3 | immediate early response 3 | 2.00 | 0.84 |
| | 10725 | NFAT5 | nuclear factor of activated T -cells 5, tonicity-responsive | 1.92 | 1.08 |
| | 7920 | BAT5 | HLA-B associated transcript 5 | 1.75 | 1.03 |
| | 3604 | TNFRSF9 | tumor necrosis factor receptor superfamily, member 9 | 1.65 | 0.80 |
| | | LY6G6E | lymphocyte antigen 6 complex, locus G6E | 1.56 | 0.60 |
| | 1437 | CSF2 | colony stimulating factor 2 (granulocyte-macrophage) | 1.49 | 0.77 |
| | 8743 | TNFSF10 | tumor necrosis factor (ligand) superfamily, member 10 | 1.24 | 0.42 |
| | 64109 | CRLF2 | cytokine receptor-like factor 2 | 0.97 | 0.70 |
| | 1241 | LTB4R | leukotriene B4 receptor | 0.87 | 0.25 |
| | | B2M | beta-2-microglobulin | 0.84 | 0.50 |
| | 2634 | GBP2 | guanylate binding protein 2, interferon-inducible | 0.80 | 0.46 |
| | 80714 | PBX4 | pre-B-cell leukemia homeobox 4 | 0.79 | 0.37 |
| | 4792 | NFKBIA | nuclear factor of kappa light polypeptide gene enhancer in B-cells inhibitor, alpha | 0.75 | 0.14 |
| | 4792 | NFKBIA | nuclear factor of kappa light polypeptide gene enhancer in B-cells inhibitor, alpha | 0.74 | 0.15 |
| | | B2M | beta-2-microglobulin | 0.70 | 0.08 |
| | 3107 | HLA-C | major histocompatibility complex, class I, C | 0.63 | 0.19 |
| | | B2M | beta-2-microglobulin | 0.61 | 0.09 |
| | 3136 | HLA-H | Major histocompatibility complex, class I. H | 0.57 | 0.26 |
| | 3135 | HLA-G | HLA-G histocompatibility antigen, class I, G | 0.52 | 0.35 |
| | | BCL6 | B-cell CLL/lymphoma 6 (zinc finger protein 51) | 0.48 | 0.17 |
| | 3135 | HLA-G | HLA-G histocompatibility antigen, class I**,** G | 0.36 | 0.09 |
| | 3134 | HLA-F | Major histocompatibility complex, class I. F | 0.35 | 0.16 |
| | 3106 | HLA-B | major histocompatibility complex, class I, B | 0.28 | 0.14 |
| | | | HLA CLASS I HISTOCOMPATIBILITY ANTIGEN, ALPHA CHAIN F PRECURSOR (HL | 0.28 | 0.11 |
| | | BAG3 | BCL2-associated athanogene 3 | 0.23 | 0.15 |
| | 3108 | MLA-DMA | major histocompatibility complex, class II, DM alpha | 0.12 | 0.04 |
| | 28639 | TRBC1 | T cell receptor beta constant 1 | 0.08 | 0.03 |
| | 3126 | HLA-DRB4 | major histocompatibility complex, class II, DR beta 4 | 0.08 | 0.04 |

### Example 7

### Identification of differentially expressed host proteins in hosts bearing PANC-1 xenograft tumors, which respond favorably to viral therapy

To identify host proteins which are differentially expressed in hosts that respond favorably to viral therapy, PANC-1 tumor cells (human pancreatic ductal carcinoma cells) were implanted subcutaneously into nude mice (male or female). Once the tumors reached a desired size (approximately 200-400 mm³), the mice were injected intravenously with 5x10⁶ pfu/100 µl PBS/mouse of vaccinia virus GLV-1h68. Mice were sacrificed at 21 days and 42 days post-injection. Tumors were excised and prepared in Tris buffer. Protein expression profiles were determined by Rules-Based Medicine, Inc. using the RodentMAP^{™} multi-analyte profile immunoassay. The assay measure mouse protein levels in the extracted tumor samples. Table 16 shows the fold change in the host protein expression levels for tumors injected with GLV-1h68 (treated) and non-injected control tumors (untreated) at 21 days and 42 days post-injection (n=2). The data in Table 16 is expressed as Treated/Untreated and Untreated/Treated.

**Table 16**

| **Fold Change in Host Protein Expression Levels Between GLV-1h68 treated and untreated PANC-1 human pancreatic xenograft tumors** | | | | |
|---|---|---|---|---|
| | **Fold change in protein level** | | | |
| | **Treated / Untreated** | | **Untreated / Treated** | |
| **Mouse Protein (expressed by host)** | **Day 21** | **Day 42** | **Day 21** | **Day 42** |
| Apo A1 (Apolipoprotein A1) | 6.87 | 6.77 | 0.15 | 0.15 |
| CD40 | 1.56 | 1.90 | 0.64 | 0.53 |
| CD40 Ligand | 1.89 | 2.12 | 0.53 | 0.47 |
| CRP (C Reactive Protein) | 3.98 | 3.29 | 0.25 | 0.30 |
| EGF (Epidermal Growth Factor) | 0.40 | 1.60 | 2.53 | 0.62 |
| Endothelin-1 | 0.68 | 1.39 | 1.48 | 0.72 |
| Eotaxin | 7.44 | 5.56 | 0.13 | 0.18 |
| Factor VII | 1.26 | 0.98 | 0.80 | 1.02 |
| FGF-9 (Fibroblast Growth Factor-9) | 1.46 | 1.15 | 0.69 | 0.87 |
| FGF-basic (Fibroblast Growth Factor-basic) | 1.89 | 0.35 | 0.53 | 2.88 |
| Fibrinogen | 5.67 | 1.36 | 0.18 | 0.73 |
| GCP-2 (Granulocyte Chemotactic Protein-2) | 5.49 | 3.69 | 0.18 | 0.27 |
| GM-CSF (Granulocyte Macrophage-Colony Stimulating Factor) | 0.49 | 1.63 | 2.03 | 0.61 |
| GST-alpha (Glutathione S-Transferase alpha) | n/a | n/a | n/a | n/a |
| Haptoglobin | 1.87 | 1.53 | 0.53 | 0.65 |
| IFN-gamma (Interferon-gamma) | 2.20 | 1.25 | 0.45 | 0.80 |
| IgA (Immunoglobulin A) | 1.57 | 0.62 | 0.64 | 1.62 |
| IL-10 (Interleukin-10) | 1.89 | 1.86 | 0.53 | 0.54 |
| IL-11 (Interleukin-11) | 6.02 | 4.06 | 0.17 | 0.25 |
| IL-12p70 (Interleukin-12p70) | 1.70 | 1.50 | 0.59 | 0.67 |
| IL-17 (Interleukin-17) | 2.19 | 1.93 | 0.46 | 0.52 |
| IL-18 (Interleukin-18) | 14.10 | 33.98 | 0.07 | 0.03 |
| IL-1 alpha (interieukin-1 alpha) | 0.58 | 1.65 | 1.72 | 0.61 |
| IL-1 beta (Interleukin-1 beta) | 0.33 | 0.91 | 3.06 | 1.10 |
| IL-2 (Interleukin-2) | 1.42 | 1.38 | 0.70 | 0.73 |
| IL-3 (Interleukin-3) | 1.51 | 1.23 | 0.66 | 0.81 |
| IL-4 (Interleukin-4) | 1.49 | 1.27 | 0.67 | 0.79 |
| IL-5 (interleukin-5) | 0.85 | 1.21 | 1.17 | 0.82 |
| IL-6 (Interleukin-6) | 4.08 | 7.82 | 0.25 | 0.13 |
| IL-7 (Interleukin-7) | 1.03 | 1.22 | 0.97 | 0.82 |
| IP-10 (Inducible Protein-10) | 10.48 | 11.24 | 0.10 | 0.09 |
| KC/GRO alpha (Melanoma Growth Stimulatory Activity Protein) | 1.93 | 3.66 | 0.52 | 0.27 |
| LIF (Leukemia Inhibitory Factor) | 1.47 | 0.96 | 0.68 | 1.04 |
| Lymphotactin | 0.34 | 0.55 | 2.93 | 1.83 |
| MCP-1 (Monocyte Chemoattractant Protein-1) | 14.02 | 21.80 | 0.07 | 0.05 |
| MCP-3 (Monocyte Chemoattractant Protein-3) | 10.59 | 12.23 | 0.09 | 0.08 |
| MCP-5 (Monocyte Chemoattractant Protein-5) | 25.59 | 39.05 | 0.04 | 0.03 |
| M-CSF (Macrophage-Colony Stimulating Factor) | 2.20 | 4.06 | 0.45 | 0.25 |
| MDC (Macrophage-Derived Chemokine) | 0.68 | 1.59 | 1.46 | 0.63 |
| MIP-1 alpha (Macrophage Inflammatory Protein-1 alpha) | 0.55 | 0.54 | 1.80 | 1.85 |
| MIP-1 beta (Macrophage Inflammatory Protein-1 beta) | 0.35 | 0.46 | 2.89 | 2.17 |
| MIP-1gamma (Macrophage Inflammatory Protein-1gamma) | 0.10 | 0.08 | 9.70 | 13.11 |
| MIP-2 (Macrophage Inflammatory Protein-2) | 4.82 | 3.59 | 0.21 | 0.28 |
| MIP-3 beta (Macrophage Inflammatory Protein-3 beta) | 0.35 | 0.99 | 2.89 | 1.01 |
| MMP-9 (Matrix Metalloproteinase-9) | 10.70 | 6.38 | 0.09 | 0.16 |
| MPO (Myeloperoxidase) | 5.62 | 2.65 | 0.18 | 0.38 |
| Myoglobin | - | - | - | - |
| OSM (Oncostatin M) | 1.13 | 1.31 | 0.89 | 0.77 |
| RANTES (Regulation Upon Activation, Normal T-Cell Expressed and Secreted) | 0.23 | 0.13 | 4.41 | 7.45 |
| SAP (Serum Amyloid P) | 2.33 | 1.90 | 0.43 | 0.53 |
| SCF (Stem Cell Factor) | 1.33 | 1.62 | 0.75 | 0.62 |
| SGOT (Serum Glutamic-Oxaloacetic Transaminase) | 1.18 | 0.82 | 0.85 | 1.22 |
| TIMP-1 (Tissue Inhibitor of Metalloproteinase Type-1) | 14.26 | 13.80 | 0.07 | 0.07 |
| Tissue Factor | 2.11 | 1.20 | 0.47 | 0.83 |
| TNF-alpha (Tumor Necrosis Factor-alpha) | 1.34 | 1.88 | 0.74 | 0.53 |
| TPO (Thrombopoietin) | 2.22 | 2.07 | 0.45 | 0.48 |
| VCAM-1 (Vascular Cell Adhesion Molecule-1) | 0.96 | 2.00 | 1.05 | 0.50 |
| VEGF (Vascular Endothelial Cell Growth Factor) | 1.37 | 0.73 | 0.73 | 1.37 |
| vWF (von Willebrand Factor) | 2.28 | 1.76 | 0.44 | 0.57 |

### Example 8

### Identification of differentially expressed host proteins in hosts bearing xenograft tumors, which respond favorably (PANC-1) or poorly (HT29) to viral therapy

To identify host proteins which are differentially expressed in hosts having virus-treated tumors derived from cells known to respond favorably or cells known to respond poorly to viral therapy, PANC-1 tumor cells or HT29 tumor cells were implanted subcutaneously into nude mice (male or female). PANC-1 is a human pancreatic ductal carcinoma which responds favorably to viral therapy; HT29 is a human colorectal adenocarcinoma which responds poorly to viral therapy. Once the tumors reached a desired size (approximately 200-400 mm³), the mice were injected intravenously with 5x10⁶ pfu/100 µl PBS/mouse of vaccinia virus GLV-1h68. Mice were sacrificed and the tumors were excised and prepared in Tris buffer. Host protein profiles were determined by Rules-Based Medicine, Inc. using the RodentMAP^{™} multi-analyte profile immunoassay for tumors injected with GLV-1h68 (treated), and non-injected control tumors (untreated). The assay measures mouse protein levels in the extracted tumor samples. Table 17 shows the fold change in the host protein expression levels between GLV-1h68 treated and untreated tumor samples for tumors derived from either PANC-1 cells or HT29 cells. The data in Table 17 is expressed as either Untreated/Treated or Treated/Untreated for each tumor type. Table 18 shows the fold difference in protein expression levels between tumors derived from PANC-1 cells versus HT29 cells in GLV-1h68 (treated), or non-injected (untreated) tumors. The data in Table 18 is expressed as either PANC-1/HT29 or HT29/PANC-1 for either the treated or untreated tumor samples.

**Table 17**

| **Fold Change in Host Protein Expression Levels Between GLV-1h68 treated and untreated PANC-1 human pancreatic and HT29 human colorectal carcinoma xenograft tumors** | | | | |
|---|---|---|---|---|
| | **Fold change in protein levels** | | | |
| | **Untreated / Treated** | | **Treated / Untreated** | |
| **Mouse Protein (expressed by host)** | **PANC-1** | **HT29** | **PANC-1** | **HT29** |
| Apo A1 (Apolipoprotein A1) | 0.2 | 0.8 | 5.2 | 1.2 |
| CD40 | 1.1 | 0.5 | 0.9 | 2.0 |
| CD40 Ligand | 1.6 | - | 0.6 | - |
| CRP (C Reactive Protein) | 0.2 | 1.0 | 4.8 | 1.0 |
| EGF (Epidermal Growth Factor) | 4.9 | 0.4 | 0.2 | 2.6 |
| Endothelin-1 | 2.7 | 0.8 | 0.4 | 1.2 |
| Eotaxin | 0.3 | 0.7 | 2.9 | 1.5 |
| Factor VII | 1.3 | 1.0 | 0.8 | 1.0 |
| FGF-9 (Fibroblast Growth Factor-9) | 0.8 | 1.0 | 1.2 | 1.0 |
| FGF-basic (Fibroblast Growth Factor-basic) | 0.5 | 0.7 | 2.0 | 1.4 |
| Fibrinogen | 0.2 | 2.8 | 4.8 | 0.4 |
| GCP-2 (Granulocyte Chemotactic Protein-2) | 0.4 | 1.3 | 2.8 | 0.8 |
| GM-CSF (Granulocyte Macrophage-Colony Stimulating Factor) | 6.9 | 0.2 | 0.1 | 4.3 |
| GST-alpha (Glutathione S-Transferase alpha) | - | - | - | - |
| Haptoglobin | 0.6 | 0.9 | 1.6 | 1.1 |
| IFN-gamma (Interferon-gamma) | 1.2 | 0.6 | 0.8 | 1.6 |
| IgA (Immunoglobulin A) | 0.6 | 1.7 | 1.8 | 0.6 |
| IL-10 (Interleukin-10) | 0.8 | 1.0 | 1.2 | 1.0 |
| IL-11 (Interleukin-11) | 0.2 | 1.2 | 6.2 | 0.9 |
| IL-12p70 (Interleukin-12p70) | 0.9 | 1.1 | 1.1 | 0.9 |
| IL-17 (Interleukin-17) | 0.7 | 1.0 | 1.4 | 1.0 |
| IL-18 (Interleukin-18) | 0.1 | 0.4 | 13.9 | 2.3 |
| IL-1 alpha (Interleukin-1 alpha) | 3.7 | 2.2 | 0.3 | 0.5 |
| IL-1 beta (Interleukin-1 beta) | 17.0 | 2.2 | 0.1 | 0.5 |
| IL-2 (Interleukin-2) | 1.1 | 0.6 | 0.9 | 1.6 |
| IL-3 (Interleukin-3) | 1.0 | 0.7 | 1.0 | 1.4 |
| IL-4 (Interleukin-4) | 1.0 | 0.9 | 1.1 | 1.1 |
| IL-5 (Interteukin-5) | 3.6 | 3.5 | 0.3 | 0.3 |
| IL-6 (Interleukin-6) | 0.4 | 0.9 | 2.6 | 1.2 |
| IL-7 (Interleukin-7) | 1.6 | 1.4 | 0.6 | 0.7 |
| IP-10 (Inducible Protein-10) | 0.8 | 0.2 | 1.3 | 4.5 |
| KC/GRO alpha (Melanoma Growth Stimulatory Activity Protein) | 2.2 | 1.3 | 0.5 | 0.8 |
| LIF (Leukemia Inhibitory Factor) | 1.1 | 1.3 | 0.9 | 0.8 |
| Lymphotactin | 5.4 | 0.7 | 0.2 | 1.4 |
| MCP-1 (Monocyte Chemoattractant Protein-1) | 0.2 | 1.5 | 5.7 | 0.7 |
| MCP-3 (Monocyte Chemoattractant Protein-3) | 0.5 | 0.8 | 2.0 | 1.3 |
| MCP-5 (Monocyte Chemoattractant Protein-5) | 0.1 | 0.5 | 8.9 | 2.0 |
| M-CSF (Macrophage-Colony Stimulating Factor) | 0.9 | 1.0 | 1.1 | 1.0 |
| MDC (Macrophage-Derived Chemokine) | 2.1 | 1.2 | 0.5 | 0.8 |
| MIP-1 alpha (Macrophage Inflammatory Protein-1 alpha) | 2.2 | 1.4 | 0.5 | 0.7 |
| MIP-1 beta (Macrophage Inflammatory Protein-1 beta) | 2.0 | 2.8 | 0.5 | 0.4 |
| MIP-1gamma (Macrophage Inflammatory Protein-1 gamma) | 14.0 | 11.7 | 0.1 | 0.1 |
| MIP-2 (Macrophage Inflammatory Protein-2) | 0.6 | 1.0 | 1.5 | 1.0 |
| MIP-3 beta (Macrophage Inflammatory Protein-3 beta) | 2.9 | 1.7 | 0.3 | 0.6 |
| MMP-9 (Matrix Metalloproteinase-9) | 0.3 | 1.4 | 3.2 | 0.7 |
| MPO (Myeloperoxidase) | 0.2 | 1.5 | 5.2 | 0.7 |
| Myoglobin | - | - | - | - |
| OSM (Oncostatin M) | 1.6 | 1.4 | 0.6 | 0.7 |
| RANTES (Regulation Upon Activation, Normal T-Cell Expressed and Secreted) | 3.3 | 0.7 | 0.3 | 1.5 |
| SAP (Serum Amyloid P) | 0.5 | 1.2 | 2.1 | 0.8 |
| SCF (Stem Cell Factor) | 1.1 | 1.0 | 0.9 | 1.0 |
| SGOT (Serum Glutamic-Oxaloacetic Transaminase) | 0.7 | 1.0 | 1.3 | 1.0 |
| TIMP-1 (Tissue Inhibitor of Metalloproteinase Type-1) | 0.2 | 1.6 | 5.0 | 0.6 |
| Tissue Factor | 0.4 | 0.9 | 2.3 | 1.2 |
| TNF-alpha (Tumor Necrosis Factor-alpha) | 1.5 | 1.3 | 0.7 | 0.8 |
| TPO (Thrombopoietin) | 1.1 | 1.5 | 0.9 | 0.7 |
| VCAM-1 (Vascular Cell Adhesion Molecule-1) | 1.4 | 0.9 | 0.7 | 1.2 |
| VEGF (Vascular Endothelial Cell Growth Factor) | 2.5 | 1.4 | 0.4 | 0.7 |
| vWF (von Willebrand Factor) | 0.6 | 1.2 | 1.6 | 0.8 |

**Table 18**

| **Fold Difference in Host Protein Expression Levels Between GLV-1h68 treated and untreated PANC-1 human pancreatic and HT29 human colorectal carcinoma xenograft tumors** | | | | |
|---|---|---|---|---|
| | **Fold difference in protein levels** | | | |
| | **Treated** | | **Untreated** | |
| **Mouse Protein (expressed by host)** | **PANC-1 / HT29** | **HT29/PA NC-1** | **PANC-1 / HT29** | **HT29/ PANC-1** |
| Apo A1 (Apolipoprotein A1) | 0.3 | 3.9 | 0.1 | 17.0 |
| CD40 | 2.2 | 0.5 | 4.9 | 0.2 |
| CD40 Ligand | - | - | 1.0 | 1.0 |
| CRP (C Reactive Protein) | 0.6 | 1.7 | 0.1 | 8.4 |
| EGF (Epidermal Growth Factor) | 0.0 | 135.9 | 0.1 | 10.7 |
| Endothelin-1 | 0.5 | 2.1 | 1.6 | 0.6 |
| Eotaxin | 1.0 | 1.0 | 0.5 | 1.9 |
| Factor VII | 1.0 | 1.0 | 1.3 | 0.8 |
| FGF-9 (Fibroblast Growth Factor-9) | 0.4 | 2.5 | 0.3 | 2.9 |
| FGF-basic (Fibroblast Growth Factor-basic) | 0.6 | 1.7 | 0.4 | 2.4 |
| Fibrinogen | 1.7 | 0.6 | 0.1 | 8.0 |
| GCP-2 (Granulocyte Chemotactic Protein-2) | 2.2 | 0.5 | 0.6 | 1.7 |
| GM-CSF (Granulocyte Macrophage-Colony Stimulating Factor) | 0.7 | 1.4 | 21.4 | 0.0 |
| GST-alpha (Glutathione S-Transferase alpha) | - | - | - | - |
| Haptoglobin | 0.7 | 1.4 | 0.5 | 2.0 |
| IFN-gamma (Interferon-gamma) | 1.5 | 0.7 | 2.8 | 0.4 |
| IgA (Immunoglobulin A) | 0.8 | 1.3 | 0.3 | 4.0 |
| IL-10 (Interleukin-10) | 1.8 | 0.6 | 1.5 | 0.7 |
| IL-11 (Interleukin-11) | 4.2 | 0.2 | 0.6 | 1.7 |
| IL-12p70 (Interleukin-12p70) | 1.6 | 0.6 | 1.3 | 0.8 |
| IL-17 (interleukin-1 7) | 1.7 | 0.6 | 1.3 | 0.8 |
| IL-18 (Interleukin-18) | 11.8 | 0.1 | 1.9 | 0.5 |
| IL-1 alpha (Interleukin-1 alpha) | 2.5 | 0.4 | 4.2 | 0.2 |
| IL-1 beta (Interleukin-1 beta) | 1.2 | 0.9 | 8.9 | 0.1 |
| IL-2 (Interleukin-2) | 0.7 | 1.4 | 1.3 | 0.8 |
| IL-3 (Interieukin-3) | 1.2 | 0.9 | 1.7 | 0.6 |
| IL-4 (Interleukin-4) | 1.2 | 0.8 | 1.3 | 0.8 |
| IL-5 (Interleukin-5) | 1.0 | 1.0 | 1.1 | 0.9 |
| IL-6 (Interleukin-6) | 3.9 | 0.3 | 1.7 | 0.6 |
| IL-7 (Interleukin-7) | 1.7 | 0.6 | 1.9 | 0.5 |
| IP-10 (Inducible Protein-10) | 2.5 | 0.4 | 9.1 | 0.1 |
| KC/GRO alpha (Melanoma Growth Stimulatory Activity Protein) | 0.2 | 5.3 | 0.3 | 3.1 |
| LIF (Leukemia Inhibitory Factor) | 0.4 | 2.5 | 0.3 | 3.1 |
| Lymphotactin | 4.3 | 0.2 | 31.4 | 0.0 |
| MCP-1 (Monocyte Chemoattractant Protein-1) | 17.5 | 0.1 | 2.0 | 0.5 |
| MCP-3 (Monocyte Chemoattractant Protein-3) | 2.1 | 0.5 | 1.4 | 0.7 |
| MCP-5 (Monocyte Chemoattractant Protein-5) | 2.6 | 0.4 | 0.6 | 1.7 |
| M-CSF (Macrophage-Colony Stimulating Factor) | 2.7 | 0.4 | 2.5 | 0.4 |
| MDC (Macrophage-Derived Chemokine) | 1.3 | 0.8 | 2.2 | 0.5 |
| MIP-1 alpha (Macrophage Inflammatory Protein-1 alpha) | 1.0 | 1.0 | 1.6 | 0.6 |
| MIP-1 beta (Macrophage Inflammatory Protein-1 beta) | 3.5 | 0.3 | 2.6 | 0.4 |
| MIP-1gamma (Macrophage Inflammatory Protein-1 gamma) | 1.3 | 0.8 | 1.6 | 0.6 |
| MIP-2 (Macrophage Inflammatory Protein-2) | 2.0 | 0.5 | 1.2 | 0.8 |
| MIP-3 beta (Macrophage Inflammatory Protein-3 beta) | 1.5 | 0.7 | 2.5 | 0.4 |
| MMP-9 (Matrix Metalloproteinase-9) | 0.9 | 1.2 | 0.2 | 5.3 |
| MPO (Myeloperoxidase) | 2.2 | 0.5 | 0.3 | 3.7 |
| Myoglobin | - | - | - | - |
| OSM (Oncostatin M) | 1.8 | 0.6 | 2.1 | 0.5 |
| RANTES (Regulation Upon Activation, Normal T-Cell Expressed and Secreted) | 3.1 | 0.3 | 15.4 | 0.1 |
| SAP (Serum Amyloid P) | 1.0 | 1.0 | 0.4 | 2.6 |
| SCF (Stem Cell Factor) | 1.9 | 0.5 | 2.1 | 0.5 |
| SGOT (Serum Glutamic-Oxaloacetic Transaminase) | 1.9 | 0.5 | 1.4 | 0.7 |
| TIMP-1 (Tissue Inhibitor of Metalloproteinase Type-1) | 0.6 | 1.7 | 0.1 | 13.4 |
| Tissue Factor | 0.3 | 3.8 | 0.1 | 7.6 |
| TNF-alpha (Tumor Necrosis Factor-alpha) | 1.7 | 0.6 | 1.9 | 0.5 |
| TPO (Thrombopoietin) | 1.6 | 0.6 | 1.1 | 0.9 |
| VCAM-1 (Vascular Cell Adhesion Molecule-1) | 2.5 | 0.4 | 4.0 | 0.2 |
| VEGF (Vascular Endothelial Cell Growth Factor) | 0.1 | 7.1 | 0.2 | 4.0 |
| vWF (von Willebrand Factor) | 0.7 | 1.5 | 0.3 | 3.0 |

### Example 9

### Identification of proteins which are differentially expressed in tumors which respond favorably (DU145) or poorly (PC-3) to viral therapy

To identify human tumor proteins which are differentially expressed in tumors which respond favorably or unfavorably to viral therapy, DU145 or PC-3 cells were grown in 60 mm dishes and mock-infected or infected with the GLV-1h68 virus or WR virus at a MOI of 10. DU145 is a human prostate cancer cell line which responds favorably to viral therapy; PC-3 is a human prostate cancer cell line which responds poorly to viral therapy. Twenty-four hours after infection supernatants were collected and cells were prepared in lysis buffer (50 mM Tris, pH 7.5, 2 mM EDTA, 0.1% Triton X-100). Protein expression profiles were determined for the supernatants and cell lysates by Rules-Based Medicine, Inc. using the HumanMAP® multi-analyte profile immunoassay. The assay measures human protein expression levels in the supernatant and cell lysate samples. Tables 19 and 20 show the human protein expression profiles of mock-infected or GLV-1h68 virus-infected PC-3 and DU145 cells in collected supernatants or collected cell lysates, respectively.

**Table 19**

| **Fold Difference in Human Protein Expression Levels in DU145 versus PC-3 human Prostate Cancer Cells (supernatant)** | | | | | |
|---|---|---|---|---|---|
| | **Fold difference in protein levels** | | | | |
| **Human Protein (tumor cell culture supernatant)** | **Untreated DU145 / Untreated PC-3** | **1h68-treated DU145 / Untreated DU145** | **1h68-treated PC-3 / Untreated PC-3** | **WR-treated DU145 / Untreated DU145** | **WR-treated PC-3 / Untreated PC-3** |
| Alpha-1 Antitrypsin | 171.38 | 0.36 | 0.69 | 0.22 | 0.38 |
| Adiponectin | - | - | - | - | - |
| Alpha-2 Macroglobulin | - | - | - | - | - |
| Alpha-Fetoprotein | 0.45 | 2.02 | 0.52 | 2.93 | 0.73 |
| Apolipoprotein A1 | - | - | - | - | - |
| Apolipoprotein CIII | - | - | - | - | - |
| Apolipoprotein H | 18.19 | 0.23 | 0.62 | 0.10 | - |
| Beta-2 Microglobulin | 2.15 | 0.35 | 0.74 | 0.27 | 0.38 |
| Brain-Derived Neurotrophic Factor | 0.70 | 1.76 | 3.33 | 1.10 | 1.50 |
| Complement 3 | 1952.96 | 0.33 | - | 0.19 | - |
| Cancer Antigen 125 | 5.68 | 1.46 | 1.01 | 1.02 | 0.83 |
| Cancer Antigen 19-9 | 10.75 | 0.95 | 1.43 | 0.79 | 1.31 |
| Calcitonin | - | - | - | - | - |
| CD40 | 2.05 | 1.53 | 0.92 | 2.14 | 1.21 |
| CD40 Ligand | 0.85 | 2.09 | 0.86 | 3.56 | 0.96 |
| Carcinoembryonic Antigen | 0.18 | 0.56 | 4.47 | 0.49 | 0.65 |
| Creatine Kinase-MB | 0.85 | 0.58 | 0.85 | 1.02 | 0.96 |
| C Reactive Protein | - | - | 1.51 | - | 1.26 |
| EGF | 12.61 | 1.98 | 40.29 | 2.17 | 49.71 |
| ENA-78 | 5.21 | 0.23 | 2.07 | 0.11 | 1.07 |
| Endothelin-1 | 5.00 | 0.39 | 2.24 | 0.30 | 1.08 |
| EN-RAGE | - | 2.34 | - | 1.28 | - |
| Eotaxin | - | 0.53 | - | 0.35 | - |
| Erythropoietin | 2.81 | 0.96 | 0.96 | 0.86 | 0.67 |
| Fatty Acid Binding Protein | 0.62 | 0.65 | 0.43 | 0.33 | 0.49 |
| Factor VII | - | - | 0.96 | - | 0.96 |
| Ferritin | 56.73 | 0.90 | 0.80 | 0.19 | 0.51 |
| FGF basic | 4.14 | 0.94 | 1.34 | 0.71 | 0.93 |
| Fibrinogen | - | 0.16 | - | - | - |
| G-CSF | - | - | 0.41 | - | 0.22 |
| Growth Hormone | - | - | - | - | 1.59 |
| GM-CSF | 0.14 | 5.14 | 1.00 | 0.57 | 0.53 |
| Glutathione S-Transferase | - | - | - | - | - |
| Haptoglobin | - | - | - | - | - |
| ICAM-1 | 4.44 | 1.06 | 0.88 | 0.93 | 1.08 |
| IFN-gamma | - | 1.03 | - | - | - |
| IgA | - | - | - | - | - |
| IgE | - | - | - | - | - |
| IGF-1 | - | - | - | - | - |
| IgM | - | - | - | - | - |
| IL-10 | 1.35 | 1.20 | 0.70 | 1.19 | 0.66 |
| IL-12p40 | - | 0.00 | - | 1.02 | - |
| IL-12p70 | 1.06 | 1.14 | 1.20 | 0.82 | 0.79 |
| IL-13 | 1.14 | 0.59 | 1.02 | 0.73 | 0.82 |
| IL-15 | 4.43 | 0.50 | 1.71 | 0.68 | 0.96 |
| IL-16 | - | - | - | - | - |
| IL-18 | 0.50 | 7.19 | 4.82 | 0.99 | 0.60 |
| IL-1 alpha | - | - | 0.78 | - | 0.51 |
| IL-1 beta | 0.00 | 1.03 | 0.52 | - | 0.09 |
| IL-1 ra | 0.03 | 1.43 | 0.88 | 1.92 | 0.39 |
| IL-2 | - | - | 1.33 | - | - |
| IL-3 | - | - | - | - | - |
| IL-4 | - | - | - | - | - |
| IL-5 | - | - | 0.54 | - | - |
| IL-6 | 1.06 | 0.21 | 3.44 | 0.41 | 7.31 |
| IL-7 | 0.63 | 1.16 | 0.73 | 0.84 | 0.43 |
| IL-8 | <0.035 | 1.67 | - | 1.33 | - |
| Insulin | - | - | - | - | - |
| Leptin | - | - | - | - | - |
| Lipoprotein (a) | 1.75 | 1.32 | 1.49 | 0.72 | 1.62 |
| Lymphotactin | - | - | - | - | - |
| MCP-1 | 0.66 | 0.05 | 0.04 | 0.11 | 0.28 |
| MDC | 3.97 | 0.52 | 0.49 | 0.35 | 0.28 |
| MIP-1 alpha | 2.48 | 0.94 | 1.18 | 0.82 | 0.87 |
| MIP-1 beta | 0.14 | 1.03 | 1.12 | 2.84 | 1.16 |
| MMP-2 | 3.56 | 0.94 | 1.14 | 0.77 | 0.81 |
| MMP-3 | 0.03 | 0.42 | 0.52 | 0.33 | 0.18 |
| MMP-9 | - | - | 0.54 | - | 0.75 |
| Myeloperoxidase | - | - | - | - | - |
| Myoglobin | 0.35 | 1.78 | 1.02 | 1.00 | 0.62 |
| PAI-1 | 0.00 | 1.83 | 1.13 | 1.02 | 0.74 |
| Prostatic Acid Phosphatase | 0.22 | 0.22 | 0.33 | 0.11 | 0.21 |
| PAPP-A | - | 0.86 | - | 0.66 | - |
| Prostate Specific Antigen, Free | 19.42 | - | - | 0.11 | 0.96 |
| RANTES | 109.71 | 0.01 | 0.36 | 0.09 | 1.41 |
| Serum Amyloid P | - | - | - | - | - |
| Stem Cell Factor | 0.58 | 0.51 | 1.05 | 0.35 | 0.81 |
| SGOT | - | - | - | 0.23 | - |
| SHBG | 1.85 | 0.38 | 0.70 | 0.19 | - |
| Thyroxine Binding Globulin | - | - | - | - | - |
| Tissue Factor | 9.30 | 2.09 | 0.63 | 2.12 | 0.96 |
| TIMP-1 | 0.51 | 0.23 | 0.54 | 0.11 | 0.17 |
| TNF RII | 12.98 | 0.36 | 0.51 | 0.27 | 0.51 |
| TNF-alpha | - | - | 0.49 | - | 0.46 |
| TNF-beta | 2.39 | - | 1.02 | 3.25 | - |
| Thrombopoietin | - | - | 2.00 | - | 1.36 |
| Thyroid Stimulating Hormone | - | - | - | - | - |
| VCAM-1 | 1.66 | 1.03 | 1.65 | 0.89 | 1.28 |
| VEGF | 24.59 | 0.40 | 1.37 | 0.22 | 0.71 |
| von Willebrand Factor | - | - | - | - | - |

**Table 20**

| **Fold Difference in Human Protein Expression Levels in DU145 versus PC-3 human Prostate Cancer Cells (cell lysate)** | | | | | |
|---|---|---|---|---|---|
| | **Fold change in protein levels** | | | | |
| **Human Protein (tumor cell lysate)** | **Untreated DU145 / Untreated PC-3** | **1h68-treated DU145 / Untreated DU145** | **1h68-treated PC-3 / Untreated PC-3** | **WR-treated DU145 / Untreated DU145** | **WR-treated PC-3 / Untreated PC-3** |
| Alpha-1 Antitrypsin | 16.19 | 0.31 | 0.76 | 0.21 | 0.26 |
| Adiponectin | 0.73 | 0.83 | 1.83 | 0.74 | 0.72 |
| Alpha-2 Macroglobulin | 0.88 | 1.16 | 1.13 | 1.45 | 0.88 |
| Alpha-Fetoprotein | 1.82 | 0.64 | 1.12 | 0.66 | 0.98 |
| Apolipoprotein A1 | - | - | - | - | - |
| Apolipoprotein CIII | 0.30 | 0.77 | - | 1.04 | - |
| Apolipoprotein H | - | - | - | 0.00 | - |
| Beta-2 Microglobulin | 1.29 | 0.22 | 0.83 | 0.16 | 0.30 |
| Brain-Derived Neurotrophic Factor | 0.15 | 0.37 | 1.37 | 0.44 | 0.38 |
| Complement 3 | - | 0.02 | - | 0.00 | - |
| Cancer Antigen 125 | 1.61 | 0.25 | 0.72 | 0.22 | 0.30 |
| Cancer Antigen 19-9 | 3.52 | 0.52 | 0.58 | 0.49 | 0.19 |
| Calcitonin | 0.55 | 0.76 | 0.82 | 0.78 | 0.60 |
| CD40 | 1.45 | 0.69 | 1.29 | 0.74 | 0.85 |
| CD40 Ligand | 1.32 | 0.79 | 1.19 | 0.68 | 0.67 |
| Carcinoembryonic Antigen | 0.18 | 0.22 | 0.93 | 0.21 | 0.64 |
| Creatine Kinase-MB | 0.78 | 1.06 | 0.85 | 0.86 | 0.78 |
| C Reactive Protein | - | - | - | - | - |
| EGF | 0.46 | 0.11 | 0.45 | 0.11 | 0.32 |
| ENA-78 | 0.34 | 0.10 | 0.54 | 0.18 | 0.68 |
| Endothelin-1 | 0.34 | 0.51 | 0.38 | 0.46 | 0.43 |
| EN-RAGE | - | - | - | - | - |
| Eotaxin | 1.04 | 0.75 | 1.34 | 0.85 | 0.73 |
| Erythropoietin | 0.41 | - | - | - | - |
| Fatty Acid Binding Protein | 0.26 | 0.37 | 0.99 | 0.36 | 0.68 |
| Factor VII | 0.62 | 0.39 | 1.20 | 0.48 | 0.59 |
| Ferritin | 7.95 | 0.59 | 1.56 | 0.60 | 0.75 |
| FGF basic | 0.81 | 0.77 | 1.82 | 0.74 | 0.57 |
| Fibrinogen | - | - | - | - | - |
| G-CSF | 0.68 | 0.84 | 0.76 | 0.94 | 0.95 |
| Growth Hormone | 0.39 | 0.33 | 0.71 | - | 0.63 |
| GM-CSF | 0.04 | 2.46 | 0.78 | 0.42 | 0.25 |
| Glutathione S-Transferase | 0.71 | 0.76 | 1.17 | 0.84 | 0.95 |
| Haptoglobin | - | - | - | - | - |
| ICAM-1 | 1.62 | 0.52 | 0.73 | 0.71 | 0.51 |
| IFN-gamma | 1.09 | 0.72 | 1.29 | 0.74 | 1.23 |
| IgA | - | - | - | - | - |
| IgE | 0.13 | - | 0.29 | - | 0.99 |
| IGF-1 | - | - | - | - | - |
| Igm | - | - | - | - | - |
| IL-10 | 0.20 | 0.84 | 1.00 | 0.88 | 0.95 |
| IL-12p40 | 0.55 | 0.33 | 0.70 | 0.60 | 0.57 |
| IL-12p79 | 0.64 | 0.66 | 0.88 | 1.07 | 0.78 |
| IL-13 | 0.65 | 0.75 | 0.96 | 0.82 | 0.91 |
| IL-15 | 0.69 | 0.47 | 1.08 | 0.70 | 0.83 |
| IL-16 | 0.62 | 0.46 | 0.83 | 0.64 | 0.66 |
| IL-18 | 0.52 | 2.00 | 1.87 | 1.47 | 0.63 |
| IL-1 alpha | - | - | 1.13 | - | 0.98 |
| IL-1 beta | 0.01 | 0.51 | 0.53 | 0.35 | 0.05 |
| IL-1 ra | 0.03 | 0.90 | 0.92 | 1.01 | 0.61 |
| IL-2 | 0.35 | 0.27 | 0.55 | 0.26 | 0.47 |
| IL-3 | 0.58 | - | - | - | - |
| IL-4 | - | - | - | - | - |
| IL-5 | 0.53 | 0.78 | 0.61 | 0.72 | 0.85 |
| IL-6 | 4.05 | 0.13 | 2.21 | 0.14 | 1.63 |
| IL-7 | 0.27 | 0.66 | 0.81 | 0.77 | 0.60 |
| IL-8 | 0.01 | 0.55 | 1.04 | 0.52 | 0.62 |
| Insulin | - | - | - | - | 0.77 |
| Leptin | 1.68 | 0.39 | 1.29 | - | 1.36 |
| Lipoprotein (a) | - | - | - | - | - |
| Lymphotactin | 0.72 | 0.38 | 0.88 | 0.34 | 0.85 |
| MCP-1 | 0.11 | 0.72 | 0.21 | 0.89 | 0.28 |
| MDC | 1.00 | 0.10 | 0.38 | 0.75 | 0.23 |
| MIP-1 alpha | 0.73 | 0.40 | 1.06 | 0.51 | 0.72 |
| MIP-1 beta | 0.38 | 0.41 | 0.70 | 0.55 | 0.52 |
| MMP-2 | 1.05 | 0.10 | 0.56 | 0.04 | 0.18 |
| MMP-3 | 0.28 | - | 0.81 | - | 0.59 |
| MMP-9 | 0.86 | 0.69 | 1.29 | 0.57 | 0.86 |
| Myeloperoxidase | - | - | - | - | - |
| Myoglobin | 0.53 | 0.66 | 1.56 | 0.75 | 0.71 |
| PAI-1 | - | - | 0.76 | - | 0.44 |
| Prostatic Acid Phosphatase | 0.06 | 0.35 | 0.97 | 0.40 | 0.50 |
| PAPP-A | 1.10 | 0.79 | 1.08 | 0.74 | 0.80 |
| Prostate Specific Antigen, Free | 0.53 | 0.84 | 0.80 | 0.48 | 0.44 |
| RANTES | 6.85 | - | - | - | 0.14 |
| Serum Amyloid P | - | - | - | - | - |
| Stem Cell Factor | 0.62 | 0.26 | 0.77 | 0.46 | 0.67 |
| SGOT | 1.70 | 1.27 | 1.49 | 1.72 | 0.70 |
| SHBG | - | - | - | - | - |
| Thyroxine Binding Globulin | - | - | - | - | - |
| Tissue Factor | 14.27 | 0.45 | 1.18 | 0.42 | 0.56 |
| TIMP-1 | 0.20 | 0.04 | 0.51 | 0.04 | 0.22 |
| TNF RII | 1.40 | 0.09 | 0.20 | 0.11 | 0.07 |
| TNF-alpha | 0.19 | 0.27 | 0.50 | 0.67 | 0.44 |
| TNF-beta | 0.67 | 0.29 | 0.56 | 0.53 | 0.68 |
| Thrombopoietin | 0.64 | 0.36 | 1.05 | 0.59 | 0.84 |
| Thyroid Stimulating Hormone | 1.04 | 0.42 | 0.86 | - | 1.10 |
| VCAM-1 | - | - | - | - | - |
| VEGF | 1.14 | 0.16 | 0.51 | 0.09 | 0.17 |
| von Willebrand Factor | - | - | - | - | - |

### Example 10

### Identification of proteins which are differentially expressed in xenograft tumors which respond favorably (PANC-1) or poorly (HT-29) to viral therapy

To identify differentially expressed proteins in virus-infected tumors derived from cancer cells known to respond favorably to viral therapy versus cancer cells known to respond poorly to viral therapy, PANC-1 human tumor cells or HT-29 human tumor cells were implanted subcutaneously into nude mice (male or female). Once the tumors reached a desired size, (approximately 200-400 mm³), the mice were injected intravenously with 5x10⁶ pfu/100 µl PBS/mouse of vaccinia virus GLV-1h68. Mice were sacrificed at 42 days after injection. Tumors were excised and prepared in Tris buffer. Human protein profiles were determined by Rules-Based Medicine, Inc. using the HumanMAP® multi-analyte profile immunoassay for tumors injected with GLV-1h68 (treated) and non-injected control tumors (untreated). The assay measures the level of expression of human proteins in the tumor sample. Table 21 shows fold change in protein expression levels of human proteins between treated and untreated xenograft tumors derived from PANC-1 cells or HT29 cells.

**Table 21**

| **Fold Change in Human Protein Expression Levels in GLV-1h68 treated and untreated PANC-1 human pancreatic and HT29 human colorectal carcinoma xenograft tumors** | | | | |
|---|---|---|---|---|
| | **Fold change in protein level** | | | |
| | **Untreated / Treated** | | **Treated / Untreated** | |
| **Human protein** | **PANC-1** | **HT29** | **PANC-1** | **HT29** |
| Alpha-1 Antitrypsin | - | 0.8 | - | 1.2 |
| Adiponectin | - | 2.2 | - | 0.5 |
| Alpha-2 Macroglobulin | 1.3 | 1.2 | 0.7 | 0.8 |
| Alpha-Fetoprotein | 1.6 | 0.6 | 0.6 | 1.6 |
| Apolipoprotein A1 | - | - | - | - |
| Apolipoprotein CIII | - | - | - | - |
| Apolipoprotein H | - | 0.5 | - | 2.1 |
| Beta-2 Microglobulin | 2.1 | 1.0 | 0.5 | 1.0 |
| Brain-Derived Neurotrophic Factor | 0.4 | 0.4 | 2.3 | 2.5 |
| Complement 3 | | - | - | - |
| Cancer Antigen 125 | 0.6 | 1.2 | 1.6 | 0.8 |
| Cancer Antigen 19-9 | - | 0.7 | - | 1.5 |
| Calcitonin | - | 0.4 | - | 2.7 |
| CD40 | 1.0 | 0.8 | 1.1 | 1.2 |
| CD40 Ligand | 0.8 | 1.3 | 1.2 | 0.7 |
| Carcinoembryonic Antigen | 1.5 | - | 0.7 | - |
| Creatine Kinase-MB | 2.1 | 0.7 | 0.5 | 1.5 |
| C Reactive Protein | 1.7 | 0.9 | 0.6 | 1.1 |
| EGF | 10.7 | 0.5 | 0.1 | 2.0 |
| ENA-78 | 0.5 | 0.9 | 1.9 | 1.1 |
| Endothelin-1 | 1.6 | 0.9 | 0.6 | 1.1 |
| EN-RAGE | - | - | - | - |
| Eotaxin | 1.2 | 1.0 | 0.9 | 1.0 |
| Erythropoietin | - | 0.6 | - | 1.5 |
| Fatty Acid Binding Protein | 5.7 | 0.5 | 0.2 | 1.9 |
| Factor VII | 2.3 | 0.8 | 0.4 | 1.2 |
| Ferritin | 0.2 | 1.0 | 5.1 | 1.0 |
| FGF basic | 0.5 | 0.6 | 2.1 | 1.8 |
| Fibrinogen | - | - | - | - |
| G-CSF | 0.6 | 1.0 | 1.6 | 1.0 |
| Growth Hormone | 0.6 | 1.0 | 1.6 | 1.0 |
| GM-CSF | 1.0 | 0.7 | 1.0 | 1.4 |
| Glutathione S-Transferase | 1.2 | 0.9 | 0.8 | 1.1 |
| Haptoglobin | - | - | - | - |
| ICAM-1 | 1.0 | 0.5 | 1.0 | 2.2 |
| IFN-gamma | 0.9 | 1.3 | 1.1 | 0.8 |
| IgA | - | - | - | - |
| IgE | 1.6 | 0.7 | 0.6 | 1.3 |
| IGF-1 | 8.2 | - | 0.1 | - |
| IgM | - | - | - | - |
| IL-10 | 1.0 | 0.7 | 1.0 | 1.4 |
| IL-12p40 | 3.2 | 0.9 | 0.3 | 1.1 |
| IL-12p70 | 1.7 | 0.9 | 0.6 | 1.1 |
| IL-13 | 0.9 | 0.8 | 1.1 | 1.3 |
| IL-15 | 1.6 | 0.7 | 0.6 | 1.5 |
| IL-16 | 1.4 | 0.4 | 0.7 | 2.3 |
| IL-18 | 0.6 | 0.7 | 1.8 | 1.4 |
| IL-1 alpha | 0.5 | 0.9 | 2.1 | 1.1 |
| IL-1 beta | 1.9 | 2.8 | 0.5 | 0.4 |
| IL-1 ra | 2.2 | 0.7 | 0.4 | 1.4 |
| IL-2 | 3.0 | 0.3 | 0.3 | 2.9 |
| IL-3 | - | - | - | - |
| IL-4 | - | - | - | - |
| IL-5 | 1.3 | 0.7 | 0.8 | 1.4 |
| IL-6 | 0.6 | 0.5 | 1.7 | 1.9 |
| IL-7 | 1.1 | 0.7 | 0.9 | 1.4 |
| IL-8 | 1.1 | 1.4 | 0.9 | 0.7 |
| Insulin | - | - | - | - |
| Leptin | - | 0.9 | - | 1.1 |
| Lipoprotein (a) | - | 0.8 | - | 1.2 |
| Lymphotactin | - | 0.6 | - | 1.6 |
| MCP-1 | 0.4 | 5.4 | 2.7 | 0.2 |
| MDC | - | 2.2 | - | 0.4 |
| MIP-1 alpha | 1.5 | 0.9 | 0.7 | 1.1 |
| MIP-1 beta | 3.2 | 0.4 | 0.3 | 2.5 |
| MMP-2 | 1.5 | 0.9 | 0.7 | 1.1 |
| MMP-3 | 1.6 | 1.3 | 0.6 | 0.8 |
| MMP-9 | 1.6 | 0.7 | 0.6 | 1.4 |
| Myeloperoxidase | - | - | - | - |
| Myoglobin | - | - | - | - |
| PAI-1 | 0.2 | 2.1 | 4.6 | 0.5 |
| Prostatic Acid Phosphatase | 2.0 | 0.7 | 0.5 | 1.5 |
| PAPP-A | 0.4 | 0.7 | 2.3 | 1.4 |
| Prostate Specific Antigen, Free | - | 0.7 | - | 1.4 |
| RANTES | 2.1 | 5.4 | 0.5 | 0.2 |
| Serum Amyloid P | - | - | - | - |
| Stem Cell Factor | 1.8 | 0:7 | 0.5 | 1.4 |
| SGOT | 0.9 | 1.9 | 1.1 | 0.5 |
| SHBG | - | - | - | - |
| Thyroxine Binding Globulin | - | - | - | - |
| Tissue Factor | 0.3 | 0.9 | 3.4 | 1.1 |
| TIMP-1 | 0.4 | - | 2.6 | - |
| TNF RII | 0.6 | 0.6 | 1.6 | 1.8 |
| TNF-alpha | 1.3 | 1.2 | 0.7 | 0.8 |
| TNF-beta | 3.9 | 0.6 | 0.3 | 1.6 |
| Thrombopoietin | 1.9 | 0.6 | 0.5 | 1.6 |
| Thyroid Stimulating Hormone | 1.5 | 1.9 | 0.7 | 0.5 |
| VCAM-1 | 1.3 | 1.3 | 0.8 | 0.8 |
| VEGF | 1.5 | 1.2 | 0.7 | 0.8 |
| von Willebrand Factor | 0.4 | 1.0 | 2.8 | 1.0 |

### Example 11

### Specificity of Rodent Multi-Analyte Profiles (MAPs)

To demonstrate the specificity of rodent multi-analyte profile assays employed in examples above, human breast carcinoma GI-101A cells were seeded in a 60 mm dish at 5 x 10⁶ cells/dish. The following day, the medium was exchanged for fresh medium, and 24 hours later the supernatant was collected to determine antibody specificity using HumanMAP® and RodentMAP^{™} multi-analyte profile immunoassays carried out by Rules-Based Medicine, Inc. Table 22 shows the level of expression detected using RodentMAP^{™} or HumanMAP® multi-analyte profile assays.

**Table 22**

| **Cross-reactivity of Expressed Human Tumor Proteins in Human and Rodent Multi-analyte Profile Assays** | | | | |
|---|---|---|---|---|
| **Antigen** | | **Human MAP** | **Rodent MAP** | **Cross Reactivity (%)*** |
| Apolipoprotein A1 | mg/mL | 0.0E+00 | 0.000 | - |
| CD40 | ng/mL | 0.0E+00 | 0.000 | - |
| CD40 Ligand | ng/mL | 0.0E+00 | 0.000 | - |
| C Reactive Protein | ug/mL | 0.0E+00 | 0.000 | - |
| EGF | pg/mL | 9.4E+00 | 0.830 | 8.87 |
| Endothelin-1 | pg/mL | 2.5E+01 | 25.900 | 103.19 |
| Eotaxin | pg/mL | 2.8E+01 | 0.000 | 0.00 |
| Factor VII | ng/mL | 0.0E+00 | 0.000 | - |
| FGF basic | pg/mL | 3.9E+02 | 0.230 | 58.38 |
| Fibrinogen | mg/mL | 0.0E+00 | 0.000 | - |
| GM-CSF | pg/mL | 0.0E+00 | 0.578 | - |
| GST-alpha | ng/ml | 1.8E-02 | 0.000 | 0.00 |
| Haptoglobin | mg/mL | 0.0E+00 | 0.000 | - |
| IFN-gamma | pg/mL | 0.0E+00 | 3.080 | - |
| IgA | mg/mL | 0.0E+00 | 0.000 | - |
| IL-10 | pg/mL | 2.1E+00 | 0.000 | 0.00 |
| IL-12p70 | pg/mL | 9.2E+00 | 0.000 | 0.00 |
| IL-18 | pg/mL | 1.2E+01 | 0.000 | 0.00 |
| IL-1 alpha | ng/mL | 0.0E+00 | 0.000 | - |
| IL-1 beta | pg/mL | 1.3E-01 | 0.000 | 0.00 |
| IL-2 | pg/mL | 5.7E+00 | 0.000 | 0.00 |
| IL-3 | ng/mL | 6.8E-03 | 0.000 | 0.00 |
| IL-4 | pg/mL | 6.9E-01 | 10.600 | 1536.23 |
| IL-5 | pg/mL | 2.2E+00 | 0.000 | 0.00 |
| IL-6 | pg/mL | 2.5E-01 | 0.000 | 0.00 |
| IL-7 | pg/mL | 2.5E+01 | 7 | 27.56 |
| Lymphotactin | ng/mL | 0.0E+00 | 0.00147 | - |
| MCP-1 | pg/mL | 6.0E+01 | 0.000 | 0.00 |
| MDC | pg/ml | 3.2E+00 | 0.000 | 0.00 |
| MIP-1 alpha | pg/mL | 9.3E+00 | 0.000 | 0.00 |
| MIP-1 beta | pg/mL | 1.6E+01 | 0.600 | 3.66 |
| MMP-9 | ng/mL | 3.9E+00 | 0.000 | 0.00 |
| Myeloperoxidase | ng/mL | 7.0E-01 | 0.000 | 0.00 |
| Myoglobin | ng/mL | 7.3E-02 | 0.735 | 1006.85 |
| RANTES | ng/mL | 1.6E-03 | 0.000 | 0.00 |
| Serum Amyloid P | ug/mL | 0.0E+00 | 0.010 | - |
| Stem Cell Factor | pg/mL | 8.9E+01 | 0.000 | 0.00 |
| SGOT | ug/mL | 0.0E+00 | 0.000 | - |
| TIMP-1 | ng/ml | 0.0E+00 | 0.009 | 0.01 |
| Tissue Factor | ng/ml | 6.4E+01 | 0.000 | - |
| TNF-alpha | pg/mL | 4.7E-01 | 6 | 1276.60 |
| Thrombopoietin | ng/mL | 5.0E-01 | 0.000 | 0.00 |
| VCAM-1 | ng/mL | 2.0E-02 | 0.000 | 0.00 |
| VEGF | pg/mL | 1.7E+04 | 384.000 | 2.25 |
| von Willebrand Factor | ug/mL | 0.0E+00 | 0.000 | - |

| | | | | |
|---|---|---|---|---|
| * Cross reactivity = RodentMAP/HumanMAP* 100 | | | | |

### Example 12

### Reproducibility of Human Multi-Analyte Profile (MAP) assay

To demonstrate the reproducibility of the multi-analyte profile assays, protein expression profiles cells were assayed on multiple days. Supernatant was taken from cultured human breast carcinoma GI-101A cells on Day 0 (Test 1) and Day 13 (Test 2). Protein profiles of supernatants were determined using the HumanMAP® multi-analyte profile immunoassay carried out by Rules-Based Medicine, Inc. Table 23 shows the human protein concentration of proteins for Test 1 and Test 2 supernatants. Some antigens may have had a lower score due to the stability of the proteins during storage.

**Table 23**

| **Reproducibility of Protein Expression Profiles at Different Sample Times** | | | | | |
|---|---|---|---|---|---|
| **Antigen** | | **Test 1 (Day 0)** | **Test 2 (Day 13)** | **Average** | **STDEV** |
| Alpha-1 Antitrypsin | mg/mL | 1.3E-07 | 1.3E-07 | 1.32E-07 | 3.54E-09 |
| Adiponectin | ug/mL | 0.0016 | 0.0021 | 0.0018 | 0.00035 |
| Alpha-2 Macroglobulin | mg/mL | 7.7E-05 | 7.1E-05 | 7.41E-05 | 3.75E-06 |
| Alpha-Fetoprotein | ng/mL | 0.28 | 0.26 | 0.270 | 0.008 |
| Apolipoprotein A1 | mg/mL | <LOW> | 1.5E-07 | | |
| Apolipoprotein CIII | ug/mL | <LOW> | 3.4E-05 | | |
| Apolipoprotein H | ug/mL | 9.6E-06 | 1.7E-05 | 1.32E-05 | 5.01E-06 |
| Beta-2 Microglobulin | ug/mL | 0.044 | 0.036 | 0.040 | 0.006 |
| Brain-Derived Neurotrophic Factor | ng/mL | 0.027 | 0.017 | 0.022 | 0.007 |
| Complement 3 | mg/mL | 0.00023 | 0.00023 | 2.29E-04 | 7.07E-07 |
| Cancer Antigen 125 | U/mL | <LOW> | <LOW> | | |
| Cancer Antigen 19-9 | U/mL | 3.5 | 0.27 | 1.89 | 2.28 |
| Calcitonin | pg/mL | <LOW> | <LOW> | | |
| CD40 | ng/mL | <LOW> | <LOW> | | |
| CD40 Ligand | ng/mL | <LOW> | <LOW> | | |
| Carcinoembryonic Antigen | ng/mL | <LOW> | <LOW> | | |
| Creatine Kinase-MB | ng/mL | <LOW> | <LOW> | | |
| C Reactive Protein | ug/mL | <LOW> | <LOW> | | |
| EGF | pg/mL | 9.8 | 8.6 | 9.21 | 0.90 |
| ENA-78 | ng/mL | 0.029 | 0.018 | 0.024 | 0.008 |
| Endothelin-1 | pg/mL | 25 | 23 | 24 | 2 |
| EN-RAGE | ng/mL | 0.0024 | <LOW> | | |
| Eotaxin | pg/mL | 28 | 23 | 25 | 3 |
| Erythropoietin | pg/mL | 60 | 46 | 53 | 10 |
| Fatty Acid Binding Protein | ng/mL | <LOW> | 0.13 | | |
| Factor VII | ng/mL | <LOW> | 0.13 | | |
| Ferritin | ng/mL | 0.030 | <LOW> | | |
| FGF basic | pg/mL | 394 | 163 | 279 | 163 |
| Fibrinogen | mg/mL | <LOW> | <LOW> | | |
| G-CSF | pg/mL | <LOW> | <LOW> | | |
| Growth Hormone | ng/mL | <LOW> | <LOW> | | |
| GM-CSF | pg/mL | 1.0 | 1.6 | 1.31 | 0.44 |
| Glutathione S-Transferase | ng/mL | 0.18 | 0.17 | 0.18 | 0.01 |
| Haptoglobin | mg/mL | <LOW> | <LOW> | | |
| ICAM-1 | ng/mL | 0.28 | 0.17 | 0.23 | 0.08 |
| IFN-gamma | pg/mL | <LOW> | <LOW> | | |
| IgA | mg/mL | <LOW> | <LOW> | | |
| IgE | ng/mL | <LOW> | <LOW> | | |
| IGF-1 | ng/ml | <LOW> | <LOW> | | |
| IgM | mg/mL | 9.6E-08 | <LOW> | | |
| IL-10 | pg/mL | 2.1 | 2.1 | 2.06 | 0.01 |
| IL-12 p40 | ng/mL | 0.22 | 0.12 | 0.17 | 0.07 |
| IL-12 p70 | pg/mL | 22 | 15 | 18.05 | 4.88 |
| IL-13 | pg/mL | 9.0 | 8.2 | 8.64 | 0.57 |
| IL-15 | ng/mL | 0.13 | 0.12 | 0.13 | 0.00 |
| IL-16 | pg/mL | 11 | 3.9 | 7.44 | 5.03 |
| IL-18 | pg/mL | 12 | 3.7 | 7.84 | 5.88 |
| IL-1 alpha | ng/mL | <LOW> | <LOW> | | |
| IL-1 beta | pg/mL | 0.23 | 0.22 | 0.22 | 0.01 |
| IL-1 ra | pg/mL | <LOW> | <LOW> | | |
| IL-2 | pg/mL | 8.5 | 5.4 | 6.94 | 2.24 |
| IL-3 | ng/mL | 0.0068 | <LOW> | | |
| IL-4 | pg/mL | 5.3 | 8.7 | 7.02 | 2.38 |
| IL-5 | pg/mL | 2.2 | 1.5 | 1.82 | 0.49 |
| IL-6 | pg/mL | 0.55 | 0.35 | 0.45 | 0.14 |
| IL-7 | pg/mL | 38 | 45 | 41 | 5 |
| IL-8 | pg/mL | 5840 | 4440 | 5140 | 990 |
| Insulin | uIU/mL | 0.17 | <LOW> | | |
| Leptin | ng/mL | <LOW> | <LOW> | | |
| Lipoprotein (a) | ug/mL | 0.060 | <LOW> | | |
| Lymphotactin | ng/mL | 0.022 | <LOW> | | |
| MCP-1 | pg/mL | 60 | 57 | 58 | 2 |
| MDC | pg/mL | 3.2 | 1.6 | 2.40 | 1.18 |
| MIP-1 alpha | pg/mL | 15 | 6.7 | 10.69 | 5.67 |
| MIP-1 beta | pg/mL | 16 | 9.4 | 12.89 | 4.97 |
| MMP-2 | ng/mL | 142 | 21 | 81 | 86 |
| MMP-3 | ng/mL | 0.0061 | 0.0080 | 0.0071 | 0.0014 |
| MMP-9 | ng/mL | 3.9 | 3.4 | 3.61 | 0.36 |
| Myeloperoxidase | ng/mL | 0.70 | 0.74 | 0.72 | 0.03 |
| Myoglobin | ng/mL | 0.073 | 0.090 | 0.082 | 0.012 |
| PAI-1 | ng/mL | 0.37 | 0.51 | 0.442 | 0.095 |
| Prostatic Acid Phosphatase | ng/mL | <LOW> | <LOW> | | |
| PAPP-A | mIU/mL | 0.028 | 0.033 | 0.030 | 0.004 |
| Prostate Specific Antigen, Free | ng/mL | <LOW> | <LOW> | | |
| RANTES | ng/mL | 0.0023 | 0.0019 | 0.0021 | 0.0003 |
| Serum Amyloid P | ug/mL | <LOW> | <LOW> | | |
| Stem Cell Factor | pg/mL | 89 | 45 | 67 | 31 |
| SGOT | ug/mL | 1.4 | 0.93 | 1.16 | 0.32 |
| SHBG | nmol/L | <LOW> | <LOW> | | |
| Thyroxine Binding Globulin | ug/mL | <LOW> | <LOW> | | |
| Tissue Factor | ng/mL | <LOW> | 0.062 | | |
| TIMP-1 | ng/mL | 64 | 56 | 60 | 5 |
| TNF RII | ng/mL | 0.0014 | 0.0015 | 0.00147 | 0.00005 |
| TNF-alpha | pg/mL | 0.59 | 0.69 | 0.639 | 0.070 |
| TNF-beta | pg/mL | <LOW> | 1.5 | | |
| Thrombopoietin | ng/mL | 0.96 | 0.51 | 0.736 | 0.319 |
| Thyroid Stimulating Hormone | uIU/mL | <LOW> | <LOW> | | |
| VCAM-1 | ng/mL | 0.020 | <LOW> | | |
| VEGF | pg/mL | 17100 | 16100 | 16600 | 707 |
| von Willebrand Factor | ug/mL | 0.00035 | 0.00021 | 0.000278 | 0.000098 |

### Example 13

### Identification of differentially expressed host proteins in a host bearing xenograft tumors which respond favorably to viral therapy using A549 cells

To identify host proteins which are differentially expressed in hosts that respond favorably to viral therapy, A549 tumor cells (human lung carcinoma cells which are responsive to viral therapy) were implanted subcutaneously into nude mice (male or female). Once the tumors reached a desired size, the mice were injected intravenously with 5x10⁶ pfu/100 µl PBS/mouse of vaccinia virus GLV-1h68 or GLV-1h109. (GLV-1h109 is an LIVP vaccinia virus derived from GLV-1h68. The virus contains DNA encoding an anti-VEGF single chain antibody under the control of a vaccinia synthetic late promoter in place of the *LacZ*/*rTFr* expression cassette at the *TK* locus of GLV-1h68; U.S. Patent Application Serial No. 11/975,088). Mice were sacrificed at 21 days post-injection. Tumors were excised and prepared in Tris buffer. A blood sample was taken from the same mice, and serum was prepared. Protein expression profiles were determined by Rules-Based Medicine, Inc. using the RodentMAP^{™} multi-analyte profile immunoassay. Table 24 shows host protein profiles for tumors injected with GLV-1h68 or GLV-1h109 (treated), and non-injected control tumors (untreated), with the fold change in mouse protein levels between tumors injected with virus, and tumors non-injected with virus at 21 days post-injection (n=2). Table 25 shows host protein profiles for serum proteins from mice with tumors injected with GLV-1h68 or GLV-1h109 (treated), and non-injected control serum (untreated), with the fold change in mouse protein levels between serum from mice injected with virus, and serum from non-injected mice with virus at 21 days post-injection.

**Table 24**

| **Fold Change in Host Protein Expression Levels in GLV-1h68-treated, GLV-1h109-treated or Untreated A549 Human Lung Carcinoma Xenograft Tumors (tumor extract)** | | | | |
|---|---|---|---|---|
| | **Fold change in protein levels** | | | |
| **Mouse Protein from Tumor extracts** | **1h68-treated A549/untreated A549** | **1h109-treated A549/untreated A549** | **untreated A549/1h68-treated A549** | **untreated A549/1h109-treated A549** |
| Apo A1 (Apolipoprotein A1) | 1.23 | 1.87 | 0.81 | 0.53 |
| CD40 | 2.90 | 4.08 | 0.34 | 0.25 |
| CD40 Ligand | 0.77 | 2.97 | 1.31 | 0.34 |
| CRP (C Reactive Protein) | 1.06 | 0.30 | 0.94 | 3.30 |
| EGF (Epidermal Growth Factor) | 1.26 | 3.91 | 0.79 | 0.26 |
| Endothelin-1 | 0.76 | 3.37 | 1.32 | 0.30 |
| Eotaxin | 24.53 | 59.73 | 0.04 | 0.02 |
| Factor VII | 0.57 | 0.86 | 1.75 | 1.17 |
| FGF-9 (Fibroblast Growth Factor-9) | 1.17 | 3.42 | 0.86 | 0.29 |
| FGF-basic (Fibroblast Growth Factor-basic) | 0.70 | 0.16 | 1.43 | 6.43 |
| Fibrinogen | 1.81 | 1.25 | 0.55 | 0.80 |
| GCP-2 (Granulocyte Chemotactic Protein-2) | 4.04 | 5.41 | 0.25 | 0.18 |
| GM-CSF (Granulocyte Macrophage-Colony Stimulating Factor) | 6.53 | 14.78 | 0.15 | 0.07 |
| GST-alpha (Glutathione S-Transferase alpha) | 0.76 | 0.44 | 1.31 | 2.25 |
| Haptoglobin | 4.16 | 1.82 | 0.24 | 0.55 |
| IFN-gamma (Interferon-gamma) | 5.33 | 17.48 | 0.19 | 0.06 |
| IgA (Immunoglobulin A) | 1.11 | 0.68 | 0.90 | 1.48 |
| IL-10 (Interleukin-10) | 4.37 | 15.40 | 0.23 | 0.06 |
| IL-11 (Interleukin-11) | 1.86 | 2.40 | 0.54 | 0.42 |
| IL-12p70 (Interleukin-12p70) | 5.00 | 16.59 | 0.20 | 0.06 |
| IL-17 (Interleukin-17) | 3.40 | 10.32 | 0.29 | 0.10 |
| IL-18 (Interleukin-18) | 17.31 | 35.19 | 0.06 | 0.03 |
| IL-1 alpha (Interleukin-1 alpha) | 1.48 | 3.59 | 0.68 | 0.28 |
| IL-1 beta (Interleukin-1 beta) | 0.34 | 0.89 | 2.91 | 1.12 |
| IL-2 (Interleukin-2) | 1.66 | 5.37 | 0.60 | 0.19 |
| IL-3 (Interleukin-3) | 4.39 | 14.12 | 0.23 | 0.07 |
| IL-4 (Interleukin-4) | 6.70 | 21.04 | 0.15 | 0.05 |
| IL-5 (Interleukin-5) | 1.51 | 3.06 | 0.66 | 0.33 |
| IL-6 (Interleukin-6) | 22.91 | 16.32 | 0.04 | 0.06 |
| IL-7 (Interleukin-7) | 2.98 | 11.13 | 0.34 | 0.09 |
| IP-10 (Inducible Protein-10) | 79.98 | 296.58 | 0.01 | 0.003 |
| KC/GRO alpha (Melanoma Growth Stimulatory Activity Protein) | 5.53 | 2.59 | 0.18 | 0.39 |
| LIF (Leukemia Inhibitory Factor) | 0.76 | 1.22 | 1.31 | 0.82 |
| Lymphotactin | 4.87 | 17.54 | 0.21 | 0.06 |
| MCP-1 (Monocyte Chemoattractant Protein-1) | 214.83 | 433.58 | 0.005 | 0.002 |
| MCP-3 (Monocyte Chemoattractant Protein-3) | 32.96 | 85.93 | 0.03 | 0.01 |
| MCP-5 (Monocyte Chemoattractant Protein-5) | 311.16 | 307.64 | 0.003 | 0.003 |
| M-CSF (Macrophage-Colony Stimulating Factor) | 2.22 | 2.85 | 0.45 | 0.35 |
| MDC (Macrophage-Derived Chemokine) | 7.69 | 17.92 | 0.13 | 0.06 |
| MIP-1alpha (Macrophage Inflammatory Protein-1 alpha) | 0.74 | 1.69 | 1.35 | 0.59 |
| MIP-1 beta (Macrophage Inflammatory Protein-1 beta) | 11.79 | 38.80 | 0.08 | 0.03 |
| MIP-1 gamma (Macrophage Inflammatory Protein-1 gamma) | 8.88 | 2.09 | 0.11 | 0.48 |
| MIP-2 (Macrophage Inflammatory Protein-2) | 8.83 | 15.78 | 0.11 | 0.06 |
| MIP-3beta (Macrophage Inflammatory Protein-3beta) | 1.59 | 6.57 | 0.63 | 0.15 |
| MMP-9 (Matrix Metalloproteinase-9) | 2.80 | 1.91 | 0.36 | 0.52 |
| MPO (Myeloperoxidase) | 2.48 | 3.14 | 0.40 | 0.32 |
| Myoglobin | - | - | - | - |
| OSM (Oncostatin M) | 1.45 | 4.53 | 0.69 | 0.22 |
| RANTES (Regulation Upon Activation, Normal T-Cell Expressed and Secreted) | 11.11 | 106.67 | 0.09 | 0.01 |
| SAP (Serum Amyloid P) | 1.16 | 1.17 | 0.86 | 0.85 |
| SCF (Stem Cell Factor) | 2.58 | 7.42 | 0.39 | 0.13 |
| SGOT (Serum Glutamic-Oxaloacetic Transaminase) | 0.70 | 0.92 | 1.43 | 1.08 |
| TIMP-1 (Tissue Inhibitor of Metalloproteinase Type-1) | 66.90 | 90.11 | 0.01 | 0.01 |
| Tissue Factor | 0.73 | 0.32 | 1.37 | 3.14 |
| TNF-alpha (Tumor Necrosis Factor-alpha) | 5.20 | 22.86 | 0.19 | 0.04 |
| TPO (Thrombopoietin) | 1.67 | 3.32 | 0.60 | 0.30 |
| VCAM-1 (Vascular Cell Adhesion Molecule-1) | 1.48 | 1.24 | 0.67 | 0.81 |
| VEGF (Vascular Endothelial Cell Growth Factor) | 0.62 | 1.12 | 1.61 | 0.89 |
| vWF (von Willebrand Factor) | 2.85 | 3.80 | 0.35 | 0.26 |

**Table 25**

| **Fold Change in Host Protein Expression Levels in Serum from Mice Bearing GLV-1h68-treated, GLV-1h109-treated or Untreated A549 Human Lung Carcinoma Xenograft Tumors** | | | | |
|---|---|---|---|---|
| | **Fold change in protein levels** | | | |
| **Mouse Serum Protein** | **1h68-treated A549/untreated A549** | **1h109-treated A549/untreated A549** | **untreated A549/1h68-treated A549** | **untreated A549/1h109-treated A549** |
| Apo A1 (Apolipoprotein A1) | 1.51 | 1.34 | 0.66 | 0.74 |
| CD40 | 2.41 | 0.71 | 0.42 | 1.41 |
| CD40 Ligand | 1.07 | 1.03 | 0.94 | 0.97 |
| CRP (C Reactive Protein) | 0.95 | 0.56 | 1.05 | 1.78 |
| EGF (Epidermal Growth Factor) | 0.68 | 0.68 | 1.47 | 1.47 |
| Endothelin-1 | - | - | - | - |
| Eotaxin | 1.25 | 1.18 | 0.80 | 0.84 |
| Factor VII | 0.99 | 0.81 | 1.01 | 1.24 |
| FGF-9 (Fibroblast Growth Factor-9) | - | - | - | - |
| FGF-basic (Fibroblast Growth Factor-basic) | 0.60 | 0.83 | 1.66 | 1.21 |
| Fibrinogen | - | - | - | - |
| GCP-2 (Granulocyte Chemotactic Protein-2) | 0.92 | 0.76 | 1.09 | 1.32 |
| GM-CSF (Granulocyte Macrophage-Colony Stimulating Factor) | - | - | - | - |
| GST-alpha (Glutathione S-Transferase alpha) | - | - | - | - |
| Haptoglobin | 4.68 | 3.86 | 0.21 | 0.26 |
| IFN-gamma (Interferon-gamma) | 5.37 | - | 0.19 | - |
| IgA (Immunoglobulin A) | 0.66 | 0.53 | 1.51 | 1.90 |
| IL-10 (Interleukin-10) | 2.04 | 1.31 | 0.49 | 0.76 |
| IL-11 (Interleukin-11) | - | - | - | - |
| IL-12p70 (Interleukin-12p70) | 0.61 | - | 1.64 | - |
| IL-17 (Interleukin-17) | 2.12 | - | 0.47 | - |
| IL-18 (Interleukin-18) | 3.06 | 1.42 | 0.33 | 0.70 |
| IL-1alpha (Interleukin-1alpha) | 1.21 | 1.46 | 0.83 | 0.68 |
| IL-1 beta (Interleukin-1 beta) | 1.35 | 1.38 | 0.74 | 0.73 |
| IL-2 (Interleukin-2) | - | - | - | - |
| IL-3 (Interleukin-3) | - | - | - | - |
| IL-4 (Interleukin-4) | 4.76 | - | 0.21 | - |
| IL-5 (Interleukin-5) | 1.73 | 1.23 | 0.58 | 0.81 |
| IL-6 (Interleukin-6) | 10.43 | - | 0.10 | - |
| IL-7 (Interleukin-7) | 5.52 | 1.15 | 0.18 | 0.87 |
| IP-10 (Inducible Protein-10) | 10.07 | 3.71 | 0.10 | 0.27 |
| KC/GROalpha (Melanoma Growth Stimulatory Activity Protein) | 1.27 | 0.54 | 0.79 | 1.86 |
| LIF (Leukemia Inhibitory Factor) | 1.12 | 1.06 | 0.89 | 0.94 |
| Lymphotactin | 1.65 | 0.68 | 0.61 | 1.46 |
| MCP-1 (Monocyte Chemoattractant Protein-1) | 4.33 | 1.22 | 0.23 | 0.82 |
| MCP-3 (Monocyte Chemoattractant Protein-3) | 3.74 | 0.97 | 0.27 | 1.03 |
| MCP-5 (Monocyte Chemoattractant Protein-5) | 4.85 | 1.83 | 0.21 | 0.55 |
| M-CSF (Macrophage-Colony Stimulating Factor) | 1.05 | 0.71 | 0.95 | 1.40 |
| MDC (Macrophage-Derived Chemokine) | 0.85 | 0.81 | 1.18 | 1.24 |
| MIP-1alpha (Macrophage Inflammatory Protein-1alpha) | 1.25 | 0.74 | 0.80 | 1.34 |
| MIP-1 beta (Macrophage Inflammatory Protein-1 beta) | 7.01 | 3.71 | 0.14 | 0.27 |
| MIP-1gamma (Macrophage Inflammatory Protein-1 gamma) | 1.53 | 0.87 | 0.65 | 1.15 |
| MIP-2 (Macrophage Inflammatory Protein-2) | 2.82 | 1.67 | 0.35 | 0.60 |
| MIP-3beta (Macrophage Inflammatory Protein-3beta) | 1.28 | 1.13 | 0.78 | 0.89 |
| MMP-9 (Matrix Metalloproteinase-9) | 0.95 | 0.43 | 1.06 | 2.31 |
| MPO (Myeloperoxidase) | 0.99 | 0.69 | 1.01 | 1.45 |
| Myoglobin | 10.10 | 7.92 | 0.10 | 0.13 |
| OSM (Oncostatin M) | 2.29 | 1.45 | 0.44 | 0.69 |
| RANTES (Regulation Upon Activation, Normal T-Cell Expressed and Secreted) | 2.71 | 0.45 | 0.37 | 2.21 |
| SAP (Serum Amyloid P) | 0.78 | 0.55 | 1.28 | 1.82 |
| SCF (Stem Cell Factor) | 2.39 | 1.23 | 0.42 | 0.81 |
| SGOT (Serum Glutamic-Oxaloacetic Transaminase) | 0.67 | 0.64 | 1.48 | 1.56 |
| TIMP-1 (Tissue Inhibitor of Metalloproteinase Type-1) | 3.75 | 0.82 | 0.27 | 1.22 |
| Tissue Factor | 0.97 | 0.85 | 1.03 | 1.17 |
| TNF-alpha (Tumor Necrosis Factor-alpha) | 1.85 | - | 0.54 | - |
| TPO (Thrombopoietin) | 1.59 | 1.45 | 0.63 | 0.69 |
| VCAM-1 (Vascular Cell Adhesion Molecule-1) | 1.15 | 0.81 | 0.87 | 1.23 |
| VEGF (Vascular Endothelial Cell Growth Factor) | 2.34 | 4.60 | 0.43 | 0.22 |
| vWF (von Willebrand Factor) | 1.20 | 0.97 | 0.83 | 1.03 |

### Example 14

### Identification of tumor proteins which are differentially expressed in xenograft tumors which respond favorably to viral therapy using A549 cells.

To identify differentially expressed proteins in virus-infected tumors derived from cells known to respond favorably to viral therapy, A549 human tumor cells were implanted subcutaneously into nude mice (male or female). Once the tumors reached a desired size,(approximately 200-400 mm³), the mice were injected intravenously with 5x10⁶ pfu/100 µl PBS/mouse of vaccinia virus GLV-1h68 or GLV-1h109. Mice were sacrificed at 21 days after injection. Tumors were excised and prepared in Tris buffer. Human protein profiles were determined by Rules-Based Medicine, Inc. using the HumanMAP® multi-analyte profile immunoassay for tumors injected with GLV-1h68 or GLV-1h109 (treated), and non-injected control tumors (untreated). Table 26 shows fold change in protein expression levels of human proteins between treated and untreated tumors derived from A549 cells.

**Table 26**

| **Fold Change in Human Tumor Protein Expression Levels in GLV-1h68-treated, GLV-1h109-treated, or Untreated A549 human Lung Carcinoma Xenograft Tumors** | | | | |
|---|---|---|---|---|
| | **Fold change in protein level** | | | |
| **Human protein** | **1h68-treated A549/untreated A549** | **1h109-treated A549/untreated A549** | **untreated A549/ 1h68-treated A549** | **untreated A549/1h109-treated A549** |
| Alpha-1 Antitrypsin | 0.40 | - | 2.48 | - |
| Adiponectin | - | - | - | - |
| Alpha-2 Macroglobulin | 1.15 | - | 0.87 | - |
| Alpha-Fetoprotein | 0.67 | 1.91 | 1.49 | 0.52 |
| Apolipoprotein A1 | - | - | - | - |
| Apolipoprotein CIII | - | - | - | - |
| Apolipoprotein H | 0.86 | - | 1.17 | - |
| Beta-2 Microglobulin | 0.03 | - | 31.88 | - |
| Brain-Derived Neurotrophic Factor | 1.64 | 0.68 | 0.61 | 1.47 |
| Complement 3 | 0.60 | - | 1.68 | - |
| Cancer Antigen 125 | 0.62 | 0.13 | 1.61 | 7.53 |
| Cancer Antigen 19-9 | 0.82 | 0.73 | 1.21 | 1.38 |
| Calcitonin | 0.44 | 2.33 | 2.26 | 0.43 |
| CD40 | 1.38 | 0.79 | 0.73 | 1.27 |
| CD40 Ligand | 1.40 | 1.84 | 0.72 | 0.54 |
| Carcinoembryonic Antigen | - | - | - | - |
| Creatine Kinase-MB | 1.35 | 15.91 | 0.74 | 0.06 |
| C Reactive Protein | 1.01 | 0.85 | 0.99 | 1.18 |
| EGF | 0.75 | 0.71 | 1.33 | 1.41 |
| ENA-78 | 3.78 | 1.25 | 0.26 | 0.80 |
| Endothelin-1 | 0.84 | 2.43 | 1.18 | 0.41 |
| EN-RAGE | - | - | - | - |
| Eotaxin | 1.29 | 2.60 | 0.78 | 0.38 |
| Erythropoietin | - | - | - | - |
| Fatty Acid Binding Protein | 0.45 | 4.30 | 2.24 | 0.23 |
| Factor VII | 0.37 | 2.37 | 2.68 | 0.42 |
| Ferritin | 1.20 | - | 0.83 | - |
| FGF basic | 0.89 | 0.17 | 1.12 | 6.06 |
| Fibrinogen | 0.71 | - | 1.41 | - |
| G-CSF | 1.70 | 1.21 | 0.59 | 0.83 |
| Growth Hormone | 1.18 | 6.79 | 0.84 | 0.15 |
| GM-CSF | 1.48 | 1.95 | 0.68 | 0.51 |
| Glutathione S-Transferase | 1.17 | 5.46 | 0.85 | 0.18 |
| Haptoglobin | - | - | - | - |
| ICAM-1 | 0.68 | 1.15 | 1.47 | 0.87 |
| IFN-gamma | - | - | - | - |
| IgA | - | - | - | - |
| IgE | - | - | - | - |
| IGF-1 | - | - | - | - |
| IgM | 0.18 | - | 5.48 | - |
| IL-10 | 0.98 | 2.00 | 1.02 | 0.50 |
| IL-12p40 | - | 7.59 | - | 0.13 |
| IL-12p70 | 0.78 | 6.03 | 1.28 | 0.17 |
| IL-13 | 0.90 | 4.65 | 1.11 | 0.22 |
| IL-15 | 0.95 | 8.48 | 1.05 | 0.12 |
| IL-16 | 1.78 | 15.72 | 0.56 | 0.06 |
| IL-18 | 0.94 | 0.14 | 1.07 | 7.26 |
| IL-1alpha | 4.37 | 4.60 | 0.23 | 0.22 |
| IL-1beta | 0.60 | 0.13 | 1.67 | 7.44 |
| IL-1ra | 0.87 | 0.81 | 1.15 | 1.23 |
| IL-2 | - | 11.23 | - | 0.09 |
| IL-3 | 1.63 | 5.11 | 0.61 | 0.20 |
| IL-4 | - | - | - | - |
| IL-5 | 1.18 | 2.95 | 0.85 | 0.34 |
| IL-6 | 0.98 | 0.54 | 1.02 | 1.86 |
| IL-7 | 0.98 | 2.65 | 1.02 | 0.38 |
| IL-8 | 1.22 | 0.63 | 0.82 | 1.59 |
| Insulin | - | - | - | - |
| Leptin | 1.07 | 4.26 | 0.93 | 0.23 |
| Lipoprotein (a) | 0.34 | - | 2.91 | - |
| Lymphotactin | - | - | - | - |
| MCP-1 | 1.74 | 7.59 | 0.58 | 0.13 |
| MDC | - | - | - | - |
| MIP-1alpha | 0.92 | 2.43 | 1.09 | 0.41 |
| MIP-1 beta | 1.54 | 24.93 | 0.65 | 0.04 |
| MMP-2 | 0.53 | 1.62 | 1.90 | 0.62 |
| MMP-3 | - | - | - | - |
| MMP-9 | - | 6.83 | - | 0.15 |
| Myeloperoxidase | 4.49 | - | 0.22 | - |
| Myoglobin | - | - | - | - |
| PAI-1 | 0.72 | - | 1.38 | - |
| Prostatic Acid Phosphatase | 0.46 | 0.16 | 2.16 | 6.15 |
| PAPP-A | 1.91 | 3.11 | 0.52 | 0.32 |
| Prostate Specific Antigen, Free | - | 11.77 | - | 0.08 |
| RANTES | 19.03 | - | 0.05 | - |
| Serum Amyloid P | - | - | - | - |
| Stem Cell Factor | 0.92 | 1.13 | 1.08 | 0.88 |
| SGOT | 1.35 | 1.15 | 0.74 | 0.87 |
| SHBG | - | - | - | - |
| Thyroxine Binding Globulin | - | - | - | - |
| Tissue Factor | 0.79 | 0.14 | 1.27 | 7.18 |
| TIMP-1 | 1.27 | - | 0.79 | - |
| TNF RII | 0.57 | - | 1.76 | - |
| TNF-alpha | 1.07 | 6.12 | 0.93 | 0.16 |
| TNF-beta | 1.07 | 3.96 | 0.93 | 0.25 |
| Thrombopoietin | 1.07 | 7.16 | 0.93 | 0.14 |
| Thyroid Stimulating Hormone | - | - | - | - |
| VCAM-1 | 1.08 | | 0.93 | |
| VEGF | 0.70 | 0.07 | 1.43 | 13.56 |
| von Willebrand Factor | - | - | - | - |

### Example 15

### Specificity of HumanMAP multi-analyte profile immunoassay

To demonstrate the specificity of the human multi-analyte profile immunoassay, mouse mammary gland tumor 4T1 (ATCC Catalog No. CRL-2935) cells were seeded in a 60 mm dish at 5 x 10⁶ cells/dish. The following day, the medium was changed, and 24 hours later the supernatant was collected to determine antibody specificity using HumanMAP® and RodentMAP^{™} multi-analyte profile immunoassays carried out by Rules-Based Medicine, Inc. Table 27 shows the level of expression detected using RodentMAP^{™} or HumanMAP® multi-analyte profile immunoassays.

**Table 27**

| **Cross-reactivity of Expressed Mouse Tumor Proteins in Rodent and Human Multi-analyte Profile Assays** | | | | |
|---|---|---|---|---|
| **Antigen** | | **Rodent MAP** | **Human MAP** | **Cross Reactivity (%)*** |
| Apolipoprotein A1 | mg/mL | 0 | 0.0 | - |
| CD40 | ng/mL | 0.063 | 0 | 0.0 |
| CD40 Ligand | ng/mL | 0 | 0 | - |
| C Reactive Protein | ug/mL | 0 | 0 | - |
| EGF | pg/mL | 4.0 | 2.7400 | 68.7 |
| Endothelin-1 | pg/mL | 9.4 | 13.2700 | 140.9 |
| Eotaxin | pg/mL | 5.6 | 6.3300 | 113.2 |
| Factor VII | ng/mL | 1.4 | 0 | 0.0 |
| FGF basic | pg/mL | 1400 | 0.0000 | 0.0 |
| Fibrinogen | mg/mL | 0.0 | 0 | - |
| GM-CSF | pg/mL | 3739.4 | 0 | 0.0 |
| GST-alpha | ng/ml | 0 | 0.0000 | - |
| Haptoglobin | mg/mL | 0.0 | 0 | - |
| IFN-gamma | pg/mL | 15.4 | 0 | 0.0 |
| IgA | mg/mL | 0.0 | 0 | - |
| IL-10 | pg/mL | 101.1 | 0.0000 | 0.0 |
| IL-12p70 | pg/mL | 100 | 0.0000 | 0.0 |
| IL-18 | pg/mL | 200 | 0.0000 | 0.0 |
| IL-1alpha | ng/mL | 2.8247 | 0 | 0.0 |
| IL-1beta | pg/mL | 1100 | 0.0210 | 0.0 |
| IL-2 | pg/mL | 6.0 | 2.1300 | 35.3 |
| IL-3 | ng/mL | 0.0028 | 0 | 0.0 |
| IL-4 | pg/mL | 8.8 | 0.0000 | 0.0 |
| IL-5 | pg/mL | 300 | 0 | 0.0 |
| IL-6 | pg/mL | 11 | 0 | 0.0 |
| IL-7 | pg/mL | 100 | 0.0000 | 0.0 |
| Lymphotactin | ng/mL | 0.0125 | 0 | 0.0 |
| MCP-1 | pg/mL | 1458.3 | 0 | 0.0 |
| MDC | pg/ml | 133.6 | 0 | 0.0 |
| MIP-1 alpha | pg/mL | 100 | 6.1000 | 6.3 |
| MIP-1 beta | pg/mL | 75.0 | 3.5600 | 4.7 |
| MMP-9 | ng/mL | 529 | 2.5000 | 0.5 |
| Myeloperoxidase | ng/mL | 0 | 0.2500 | - |
| Myoglobin | ng/mL | 0.12 | 0 | 0.0 |
| RANTES | ng/mL | 0.1028 | 0.5200 | 505.6 |
| Serum Amyloid P | ug/mL | 0 | 0 | - |
| Stem Cell Factor | pg/mL | 261.7 | 3.3000 | 1.3 |
| SGOT | ug/mL | 0 | 0.0000 | - |
| TIMP-1 | ng/ml | 9.2 | 0.0000 | 0.0 |
| Tissue Factor | ng/ml | 2.2 | 0.0000 | 0.0 |
| TNF-alpha | pg/mL | 100 | 0.0000 | 0.0 |
| Thrombopoietin | ng/mL | 5.0 | 0.0590 | 1.2 |
| VCAM-1 | ng/mL | 29 | 0.1600 | 0.6 |
| VEGF | pg/mL | 8964.5 | 1300.6000 | 14.5 |
| von Willebrand Factor | ug/mL | 3100 | 0.0000 | 0.0 |

| | | | | |
|---|---|---|---|---|
| * Cross reactivity = HumanMAP/RodentMAP* 100 | | | | |

### Example 16

### Identification of differentially expressed tumor proteins in xenograft tumor cells which respond poorly to viral therapy using HT29 cells.

To identify tumor proteins which are differentially expressed in tumor cells that respond poorly to viral therapy, HT29 human colorectal adenocarcinoma cells were implanted subcutaneously into nude mice (male or female). Once the tumors reached a desired size, (approximately 200-400 mm³), the mice were injected intravenously with 5x10⁶ pfu/100 µl PBS/mouse of vaccinia virus GLV-1h68. Mice were sacrificed at 21 days and 42 days post-injection. Tumors were excised and prepared in Tris buffer. Protein expression profiles were determined by Rules-Based Medicine, Inc. using the HumanMAP® multi-analyte profile immunoassay. Table 28 shows human tumor cell protein profiles for mice injected with GLV-1h68 (treated), and non-injected control tumors (untreated) at either 21 days and 42 days post-infection (n=2). The data is expressed as either Untreated/GLV-1h68-Treated or GLV-1h68-Treated/Untreated for Day 21 or Day 42 post-infection.

**Table 28**

| **Fold Change in Human Tumor Protein Expression Levels in GLV-1h68-treated or Untreated HT29 Human Colorectal Adenocarcinoma Xenograft Tumors** | | | | |
|---|---|---|---|---|
| **Human protein** | **Fold change in protein level** | | | |
| | **Day 21** | | **Day 42** | |
| | **GLV-1h68-treated/untreated** | **Untreated/GLV-1h68-treated** | **GLV-1h68-treated/Untreated** | **Untreated/GLV-1h68-treated** |
| **Alpha-1 Antitrypsin** | **-** | **-** | **-** | **-** |
| Adiponectin | | - | - | - |
| Alpha-2 Macroglobulin | 0.89 | 1.12 | 0.76 | 1.31 |
| Alpha-Fetoprotein | 1.00 | 1.00 | 0.89 | 1.13 |
| Apolipoprotein A1 | - | - | - | - |
| Apolipoprotein CIII | - | - | - | - |
| Apolipoprotein H | - | - | - | - |
| Beta-2 Microglobulin | 1.05 | 0.95 | 0.96 | 1.04 |
| Brain-Derived Neurotrophic Factor | 2.37 | 0.42 | 0.49 | 2.04 |
| Complement 3 | - | - | - | - |
| Cancer Antigen 125 | 1.67 | 0.60 | 1.27 | 0.79 |
| Cancer Antigen 19-9 | | - | | - |
| Calcitonin | - | - | - | - |
| CD40 | 0.89 | 1.12 | 1.65 | 0.60 |
| CD40 Ligand | 0.63 | 1.59 | 1.55 | 0.65 |
| Carcinoembryonic Antigen | 1.09 | 0.92 | 0.62 | 1.63 |
| Creatine Kinase-MB | 0.95 | 1.05 | 0.80 | 1.26 |
| C Reactive Protein | - | - | 0.93 | 1.07 |
| EGF | 1.38 | 0.73 | 1.20 | 0.83 |
| ENA-78 | 0.98 | 1.02 | 1.16 | 0.86 |
| Endothelin-1 | 0.97 | 1.03 | 0.89 | 1.13 |
| EN-RAGE | - | - | - | - |
| Eotaxin | 0.80 | 1.26 | 0.86 | 1.16 |
| Erythropoietin | 0.90 | 1.11 | 1.15 | 0.87 |
| Fatty Acid Binding Protein | 1.20 | 0.83 | 0.74 | 1.35 |
| Factor VII | 1.35 | 0.74 | 0.67 | 1.49 |
| Ferritin | 1.29 | 0.78 | 0.71 | 1.40 |
| FGF basic | 1.93 | 0.52 | 1.24 | 0.81 |
| Fibrinogen | - | - | - | - |
| G-CSF | 1.17 | 0.86 | 1.10 | 0.91 |
| Growth Hormone | 0.85 | 1.18 | 1.11 | 0.90 |
| GM-CSF | 1.16 | 0.86 | 0.70 | 1.43 |
| Glutathione S-Transferase | 0.87 | 1.15 | 1.12 | 0.89 |
| Haptoglobin | - | - | - | - |
| ICAM-1 | 1.08 | 0.93 | 1.02 | 0.98 |
| IFN-gamma | 1.18 | 0.85 | 1.57 | 0.64 |
| IgA | - | - | - | - |
| IgE | - | - | - | - |
| IGF-1 | - | - | - | - |
| IgM | - | - | - | - |
| IL-10 | 0.87 | 1.14 | 1.25 | 0.80 |
| IL-12p40 | 1.14 | 0.88 | 1.24 | 0.81 |
| IL-12p70 | 1.08 | 0.93 | 1.10 | 0.91 |
| IL-13 | 0.92 | 1.09 | 1.33 | 0.75 |
| IL-15 | 0.91 | 1.10 | 0.79 | 1.26 |
| IL-16 | 1.20 | 0.83 | 2.40 | 0.42 |
| IL-18 | 1.24 | 0.80 | 1.68 | 0.60 |
| IL-1 alpha | 0.70 | 1.42 | 1.49 | 0.67 |
| IL-1 beta | 0.31 | 3.24 | 0.26 | 3.81 |
| IL-1 ra | - | - | - | - |
| IL-2 | 1.01 | 0.99 | - | - |
| IL-3 | 1.72 | 0.58 | 1.38 | 0.73 |
| IL-4 | - | - | - | - |
| IL-5 | 1.20 | 0.83 | 0.68 | 1.47 |
| IL-6 | 3.65 | 0.27 | 3.90 | 0.26 |
| IL-7 | 0.89 | 1.12 | 1.07 | 0.93 |
| IL-8 | 0.95 | 1.05 | 1.42 | 0.71 |
| Insulin | - | - | - | - |
| Leptin | 0.78 | 1.28 | 0.72 | 1.40 |
| Lipoprotein (a) | - | - | - | - |
| Lymphotactin | - | - | - | - |
| MCP-1 | 0.07 | 13.55 | 0.17 | 5.99 |
| MDC | 0.46 | 2.16 | 1.50 | 0.67 |
| MIP-1 alpha | 1.05 | 0.96 | 0.83 | 1.20 |
| MIP-1 beta | 1.10 | 0.91 | - | - |
| MMP-2 | 0.91 | 1.10 | 1.02 | 0.98 |
| MMP-3 | 0.39 | 2.59 | 0.53 | 1.89 |
| MMP-9 | 2.06 | 0.49 | - | - |
| Myeloperoxidase | 1.62 | 0.62 | 0.85 | 1.18 |
| Myoglobin | - | - | - | - |
| PAI-1 | 0.78 | 1.28 | 1.46 | 0.69 |
| Prostatic Acid Phosphatase | 1.85 | 0.54 | 0.61 | 1.64 |
| PAPP-A | - | - | - | - |
| Prostate Specific Antigen, Free | 1.14 | 0.88 | 0.30 | 3.38 |
| RANTES | 0.06 | 17.63 | 0.11 | 9.27 |
| Serum Amyloid P | - | - | - | - |
| Stem Cell Factor | 0.93 | 1.08 | 0.91 | 1.10 |
| SGOT | 0.90 | 1.11 | 0.89 | 1.13 |
| SHBG | 1.98 | 0.51 | 0.64 | 1.57 |
| Thyroxine Binding Globulin | - | - | - | - |
| Tissue Factor | 1.10 | 0.91 | 0.72 | 1.38 |
| TIMP-1 | 1.12 | 0.89 | 0.95 | 1.05 |
| TNF RII | 1.21 | 0.82 | 1.00 | 1.00 |
| TNF-alpha | 0.46 | 2.15 | 1.22 | 0.82 |
| TNF-beta | 0.98 | 1.02 | 1.44 | 0.70 |
| Thrombopoietin | 0.91 | 1.09 | 0.80 | 1.25 |
| Thyroid Stimulating Hormone | 0.40 | 2.47 | - | - |
| VCAM-1 | 0.50 | 2.02 | 1.02 | 0.98 |
| VEGF | 0.99 | 1.01 | 1.05 | 0.95 |
| von Willebrand Factor | 0.88 | 1.14 | 1.19 | 0.84 |

### Example 17

### Identification of differentially expressed tumor proteins in xenograft tumors which respond poorly to viral therapy using HT-29 cells.

To identify host proteins which are differentially expressed in hosts that respond poorly to viral therapy, HT-29 human colorectal adenocarcinoma cells were implanted subcutaneously into nude mice (male or female). Once the tumors reached a desired size, (approximately 200-400 mm³), the mice were injected intravenously with 5x10⁶ pfu/100 µl PBS/mouse of vaccinia virus GLV-1h68. Mice were sacrificed at 21 days and 42 days post-injection. Tumors were excised and prepared in Tris buffer. Protein expression profiles were determined by Rules-Based Medicine, Inc. using the RodentMAP^{™} multi-analyte profile immunoassay. Table 29 shows host protein profiles for tumors injected with GLV-1h68 (treated), and non-injected control tumors (untreated), with the fold change in mouse protein levels between tumors injected with virus, and tumors non-injected with virus at 21 days and 42 days post-injection (n=2).

**Table 29**

| **Fold Change in Mouse Tumor Protein Expression Levels in GLV-1h68-treated or Untreated HT29 Human Colorectal Adenocarcinoma Xenograft Tumors** | | | | |
|---|---|---|---|---|
| **Mouse protein** | **Fold change in protein levels** | | | |
| | **Day 21** | | **Day 42** | |
| | **GLV-1h68-treated/Untreated** | **Untreated/GLV-1h68-treated** | **GLV-1h68-treated /Untreated** | **Untreated/GLV-1h68-treated** |
| Apo A1 (Apolipoprotein A1) | 0.91 | 1.10 | 1.02 | 0.98 |
| CD40 | 0.92 | 1.09 | 1.67 | 0.60 |
| CD40 Ligand | 0.99 | 1.01 | 1.18 | 0.84 |
| CRP (C Reactive Protein) | 0.91 | 1.10 | 1.11 | 0.90 |
| EGF (Epidermal Growth Factor) | 2.07 | 0.48 | 1.24 | 0.81 |
| Endothelin-1 | 0.84 | 1.19 | 1.22 | 0.82 |
| Eotaxin | 2.45 | 0.41 | 7.78 | 0.13 |
| Factor VII | 1.36 | 0.74 | 0.88 | 1.14 |
| FGF-9 (Fibroblast Growth Factor-9) | 1.22 | 0.82 | 0.64 | 1.57 |
| FGF-basic (Fibroblast Growth Factor-basic) | 2.24 | 0.45 | 0.78 | 1.28 |
| Fibrinogen | 1.50 | 0.67 | 0.79 | 1.26 |
| GCP-2 (Granulocyte Chemotactic Protein-2) | 1.31 | 0.76 | 1.85 | 0.54 |
| GM-CSF (Granulocyte Macrophage-Colony Stimulating Factor) | 0.49 | 2.03 | 4.73 | 0.21 |
| GST-alpha (Glutathione S-Transferase alpha) | 0.75 | 1.34 | 0.50 | 2.00 |
| Haptoglobin | 0.93 | 1.08 | 1.36 | 0.74 |
| IFN-gamma (interferon-gamma) | 0.89 | 1.13 | 2.26 | 0.44 |
| IgA (Immunoglobulin A) | 0.75 | 1.34 | 0.82 | 1.21 |
| IL-10 (Interleukin-10) | 0.68 | 1.46 | 1.00 | 1.00 |
| IL-11 (Interleukin-11) | 0.46 | 2.18 | 2.28 | 0.44 |
| IL-12p70 (Interleukin-12p70) | 0.51 | 1.98 | 1.01 | 0.99 |
| IL-17 (Interleukin-17) | 0.71 | 1.41 | 1.91 | 0.52 |
| IL-18 (Interleukin-18) | 2.61 | 0.38 | 10.80 | 0.09 |
| IL-1 alpha (Interleukin-1 alpha) | 0.61 | 1.65 | 0.94 | 1.07 |
| IL-1 beta (Interleukin-1 beta) | 0.39 | 2.59 | 0.79 | 1.27 |
| IL-2 (Interleukin-2) | 1.17 | 0.85 | 1.57 | 0.64 |
| IL-3 (Interfeukin-3) | 0.48 | 2.08 | 1.08 | 0.93 |
| IL-4 (Interleukin-4) | 0.67 | 1.49 | 1.20 | 0.83 |
| IL-5 (Interleukin-5) | 0.45 | 2.22 | 0.74 | 1.35 |
| IL-6 (Interleukin-6) | 1.39 | 0.72 | 2.72 | 0.37 |
| IL-7 (Interleukin-7) | 0.34 | 2.92 | 1.04 | 0.96 |
| IP-10 (Inducible Protein-10) | 2.55 | 0.39 | 7.94 | 0.13 |
| KC/GRO alpha (Melanoma Growth Stimulatory Activity Protein) | 1.23 | 0.82 | 1.10 | 0.91 |
| LIF (Leukemia Inhibitory Factor) | 0.41 | 2.43 | 1.23 | 0.81 |
| Lymphotactin | 0.23 | 4.42 | 1.27 | 0.79 |
| MCP-1 (Monocyte Chemoattractant Protein-1) | 1.45 | 0.69 | 4.89 | 0.20 |
| MCP-3 (Monocyte Chemoattractant Protein-3) | 1.29 | 0.78 | 4.07 | 0.25 |
| MCP-5 (Monocyte Chemoattractant Protein-5) | 4.99 | 0.20 | 14.33 | 0.07 |
| M-CSF (Macrophage-Colony Stimulating Factor) | 1.04 | 0.96 | 1.46 | 0.68 |
| MDC (Macrophage-Derived Chemokine) | 1.27 | 0.78 | 3.67 | 0.27 |
| MIP-1 alpha (Macrophage Inflammatory Protein-1 alpha) | 0.64 | 1.57 | 0.72 | 1.39 |
| MIP-1 beta (Macrophage Inflammatory Protein-1 beta) | 0.66 | 1.52 | 2.25 | 0.45 |
| MIP-1 gamma (Macrophage Inflammatory Protein-1 gamma) | 0.00 | 275.32 | 0.01 | 122.46 |
| MIP-2 (Macrophage Inflammatory Protein-2) | 0.93 | 1.08 | 1.76 | 0.57 |
| MIP-3 beta (Macrophage Inflammatory Protein-3 beta) | 0.93 | 1.07 | 1.09 | 0.92 |
| MMP-9 (Matrix Metalloproteinase-9) | 0.80 | 1.25 | 1.22 | 0.82 |
| MPO (Myeloperoxidase) | 1.42 | 0.71 | 2.33 | 0.43 |
| Myoglobin | - | - | - | - |
| OSM (Oncostatin M) | 0.47 | 2.14 | 0.84 | 1.19 |
| RANTES (Regulation Upon Activation, Normal T-Cell Expressed and Secreted) | 0.64 | 1.56 | 1.47 | 0.68 |
| SAP (Serum Amyloid P) | 0.90 | 1.11 | 1.13 | 0.89 |
| SCF (Stem Cell Factor) | 0.65 | 1.54 | 1.15 | 0.87 |
| SGOT (Serum Glutamic-Oxaloacetic Transaminase) | 0.91 | 1.09 | 1.16 | 0.86 |
| TIMP-1 (Tissue Inhibitor of Metalloproteinase Type-1) | 0.81 | 1.24 | 1.89 | 0.53 |
| Tissue Factor | 1.17 | 0.86 | 0.89 | 1.12 |
| TNF-alpha (Tumor Necrosis Factor-alpha) | 0.37 | 2.72 | 0.80 | 1.25 |
| TPO (Thrombopoietin) | 0.68 | 1.48 | 0.83 | 1.21 |
| VCAM-1 (Vascular Cell Adhesion Molecule-1) | 0.96 | 1.04 | 1.40 | 0.72 |
| VEGF (Vascular Endothelial Cell Growth Factor) | 0.58 | 1.72 | 1.20 | 0.84 |
| vWF (von Willebrand Factor) | 0.99 | 1.01 | 1.09 | 0.92 |

### Example 18

### Comparison of Expression Levels in Different Tumors

The level of expression of one or more markers in a biological sample may be compared to the level of expression in biological samples known to respond favorably or poorly to viral therapy. For example, the level of expression of one or more markers in a tumor obtained from a subject can be compared to the level of expression in tumors known to respond favorably or poorly to viral therapy. An example of one method for comparing expression levels is described below.

| | |
|---|---|
| Weight of exemplary tumor sample A: | 500 mg |
| Weight of exemplary tumor sample B: | 600 mg |
| Weight of exemplary tumor sample C: | 550 mg |

All tumors are ground in a volume of Tris buffer (*e.g.*, 1000 ul Tris buffer, pH 7.4, with protease inhibitors added).

Assuming 1 mg = 1 ul, the tumor sample concentration for sample A is therefore = (500 mg) / (500 ul + 1000 ul) = 0.333 mg/ul

Similarly, for tumor sample B, the tumor sample concentration is (600 mg) / (600 ul + 1000 ul) = 0.375 mg/ul

Similarly, for tumor sample C, the tumor sample concentration is (550 mg) / (550 ul + 1000 ul) = 0.355 mg/ul

When the raw data of protein antigens (in concentration) is obtained, it may be adjusted to the tumor sample concentration. For example, if the raw data for protein K is, for example, 0.34 ng/ml, 0.55 ng/ml, and 0.39 ng/ml, for tumor samples A, B, and C, respectively, the relative concentration of protein K can be adjusted according the tumor sample concentration. Since tumor sample B is 0.375/0.333=1.072 fold higher than sample A, the concentration of antigen K for sample A is adjusted by calculating: 0.34 ng/ml x 1.072 = 0.36 ng/ml. The concentration of antigen K for sample C may be adjusted by calculating: 0.39 ng/ml x (0.375/0.355) = 0.41 ng/ml.

The adjusted data for the concentration of protein antigen K is 0.36 ng/ml, 0.55 ng/ml, 0.41 ng/ml for tumor sample A, B, C, respectively. After this concentration adjustment, the samples can be directly compared to identify those markers that are elevated and those markers that are decreased and the fold differences among them.

### Example 19

### Efficacy of GLV-1h68 Replication in Additional tumor cell lines

A panel of well-characterized human cancer cell lines of different histological derivation was employed in the studies described herein. All cell lines except noted were purchased from American Type Culture Collection (Manassas).

NCI-H460 lung large cell carcinoma (ATCC Cat No. HTB-177), MALME-3M malignant melanoma lung metastasis (ATCC Cat No. HTB-64), RXF-393 renal hypernephroma (National Cancer Institute Repository), NCI-H522 lung adenocarcinoma (ATCC Cat No. CRL-5810), NCI-H322M lung bronchi alveolar carcinoma (National Cancer Institute Repository), NCI-H226 lung squamous cell carcinoma (ATCC Cat No. CRL-5826), NCI-H23 lung adenocarcinoma (ATCC Cat No. CRL-5800), HOP-92 lung carcinoma (National Cancer Institute Repository), and EKVX lung adenocarcinoma (National Cancer Institute Repository) cells were cultured in Roswell Park Memorial Institute medium (RPMI) supplemented with 10% FBS. A-673 rhabdomyosarcoma (ATCC Cat No. CRL-1598) and D283/MED medulloblastoma (ATCC Cat No. HTB-185) cells were cultured in Eagle's minimal essential medium (EMEM) media supplemented with 10% FBS. MNNG/HOS osteosarcoma (ATCC Cat No. CRL-1547) cells were cultured in EMEM supplemented with 10% FBS, non-essential amino acids (NEAA) and sodium pyruvate. All cell cultures were carried out at 37°C under 5% CO₂.

Cells were seeded in 24-well plates and were infected individually with GLV-1h68, LIVP or WR at a MOI of 0.01 as described in Zhang, Q. et al. (2007) Cancer Res 67:10038-10046. Cells were harvested at various time points up to 72 hours post infection (h.p.i.). Viral titers in the cell samples were determined as pfu/ml of medium in duplicates by standard plaque assays in CV-1 cell cultures. Data is shown in Tables 30A-30L for viral titers as 1, 24, 48 and 72 hours post infection.

**Table 30A**

| **Viral titer values for NCI-H460** | | | | | | |
|---|---|---|---|---|---|---|
| **Hours Post Infection** | **LIVP Average Viral Titer (Log pful/ 10⁶ cells)** | **Standard Deviation (Log pfu/10⁶ cells)** | **WR Average Viral Titer (Log pfu/ 10⁶ cells)** | **Standard Deviation (Log pfu/ 10⁶ cells)** | **GLV-1h68 Average Viral Titer (Log pfu/ 10⁶ cells)** | **Standard Deviation (Log pfu/ 10⁶ cells)** |
| 1 | 4.25 | 0.04 | 4.13 | 0.06 | 3.33 | 0.17 |
| 24 | 6.52 | 0.08 | 6.30 | 0.03 | 5.67 | 0.04 |
| 48 | 7.29 | 0.07 | 7,06 | 0.03 | 6.31 | 0.32 |
| 72 | 7.56 | 0.22 | 7.48 | 0.17 | 6.74 | 0.10 |

**Table 30B**

| **Viral titer values for MALME-3M** | | | | | | |
|---|---|---|---|---|---|---|
| **Hours Post Infection** | **LIVP Average Viral Titer (Log pfu/ 10⁶ cells)** | **Standard Deviation (Log pfu/10⁶ cells)** | **WR Average Viral Titer (Log pfu/ 10⁶ cells** | **Standard Deviation (Log pfu/ 10⁶ cells)** | **GLV-1h68 Average Viral Titer (Log pfu/ 10⁶ cells)** | **Standard Deviation (Log pfu/ 10⁶ cells)** |
| 1 | 3.64 | 0.03 | 3.67 | 0.14 | 2.77 | 0.30 |
| 24 | 7.24 | 0.10 | 7.40 | 0.13 | 5.54 | 0.02 |
| 48 | 7.59 | 0.07 | 7.56 | 0.05 | 6.84 | 0.09 |
| 72 | 7.61 | 0.11 | 7.57 | 0.04 | 7.20 | 0.11 |

**Table 30C**

| **Viral titer values for RXF-393** | | | | | | |
|---|---|---|---|---|---|---|
| **Hours Post Infection** | **LIVP Average Viral Titer (Log pfu/ 10⁶ cells)** | **Standard Deviation (Log pfu/10⁶ cells)** | **WR Average Viral Titer (Log pfu/ 10⁶ cells** | **Standard Deviation (Log pfu/ 10⁶ cells)** | **GLV-1h68 Average Viral Titer (Log pfu/ 10⁶ cells)** | **Standard Deviation (Log pfu/ 10⁶ cells)** |
| 1 | 4.11 | 0.04 | 4.22 | 0.02 | 3.30 | 0.08 |
| 24 | 6.94 | 0.08 | 6.87 | 0.03 | 5.60 | 0.02 |
| 48 | 7.34 | 0.03 | 7.25 | 0.16 | 6.65 | 0.11 |
| 72 | 7.05 | 0.18 | 7.31 | 0.18 | 7.19 | 0.11 |

**Table 30D**

| **Viral titer values for NCI-H522** | | | | | | |
|---|---|---|---|---|---|---|
| **Hours Post Infection** | **LIVP Average Viral Titer (Log pfu/ 10⁶ cells)** | **Standard Deviation (Log pfu/10⁶ cells)** | **WR Average Viral Titer (Log pfu/ 10⁶ cells** | **Standard Deviation (Log pfu/ 10⁶ cells)** | **GLV-1h68 Average Viral Titer (Log pfu/ 10⁶ cells)** | **Standard Deviation (Log pfu/ 10⁶ cells)** |
| 1 | 4.25 | 0.17 | 4.13 | 0.21 | 3.07 | 0.32 |
| 24 | 6.83 | 0.10 | 6.47 | 0.16 | 5.58 | 0.09 |
| 48 | 7.72 | 0.06 | 7.21 | 0.29 | 7.34 | 0.14 |
| 72 | 7.76 | 0.05 | 7.46 | 0.11 | 7.53 | 0.05 |

**Table 30E**

| **Viral titer values for NCI-H322M** | | | | | | |
|---|---|---|---|---|---|---|
| **Hours Post Infection** | **LIVP Average Viral Titer (Log pfu/ 10⁶ cells)** | **Standard Deviation (Log pfu/10⁶ cells)** | **WR Average Viral Titer (Log pfu/ 10⁶ cells** | **Standard Deviation (Log pfu/ 10⁶ cells)** | **GLV-1h68 Average Viral Titer (Log pfu/ 10⁶ cells)** | **Standard Deviation (Log pfu/ 10⁶ cells)** |
| 1 | 3.81 | 0.14 | 3.81 | 0.05 | 3.11 | 0.14 |
| 24 | 6.00 | 0.04 | 5.47 | 0.10 | 4.30 | 0.05 |
| 48 | 6.61 | 0.06 | 5.74 | 0.07 | 4.47 | 0.07 |
| 72 | 6.71 | 0.09 | 5.88 | 0.11 | 4.49 | 0.05 |

**Table 30F**

| **Viral titer values for NCI-H226** | | | | | | |
|---|---|---|---|---|---|---|
| **Hours Post Infection** | **LIVP Average Viral Titer (Log pfu/ 10⁶ cells)** | **Standard Deviation (Log pfu/10⁶ cells)** | **WR Average Viral Titer (Log pfu/ 10⁶ cells** | **Standard Deviation (Log pfu/ 10⁶ cells)** | **GLV-1h68 Average Viral Titer (Log pfu/ 10⁶ cells)** | **Standard Deviation (Log pfu/ 10⁶ cells)** |
| 1 | 3.94 | 0.08 | 3.34 | 0.05 | 3.12 | 0.02 |
| 24 | 6.86 | 0.13 | 5.81 | 0.06 | 5.23 | 0.06 |
| 48 | 8.00 | 0.15 | 7.55 | 0.21 | 6.32 | 0.16 |
| 72 | 8.28 | 0.17 | 7.72 | 0.14 | 7.37 | 0.17 |

**Table 30G**

| **Viral titer values for NCI-H23** | | | | | | |
|---|---|---|---|---|---|---|
| **Hours Post Infection** | **LIVP Average Viral Titer (Log pfu/ 10⁶ cells)** | **Standard Deviation (Log pfu/10⁶ cells)** | **WR Average Viral Titer (Log pfu/ 10⁶ cells** | **Standard Deviation (Log pfu/ 10⁶ cells)** | **GLV-1h68 Average Viral Titer (Log pfu/ 10⁶ cells)** | **Standard Deviation (Log pfu/ 10⁶ cells)** |
| 1 | 3.99 | 0.13 | 3.82 | 0.17 | 3.14 | 0.12 |
| 24 | 6.97 | 0.19 | 7.06 | 0.07 | 6.24 | 0.09 |
| 48 | 7.24 | 0.16 | 7.11 | 0.11 | 6.98 | 0.20 |
| 72 | 7.18 | 0.22 | 7.09 | 0.21 | 7.14 | 0.12 |

**Table 30H**

| **Viral titer values for HOP-92** | | | | | | |
|---|---|---|---|---|---|---|
| **Hours Post Infection** | **LIVP Average Viral Titer (Log pfu/ 10⁶ cells)** | **Standard Deviation (Log pfu/10⁶ cells)** | **WR Average Viral Titer (Log pfu/ 10⁶ cells** | **Standard Deviation (Log pfu/ 10⁶ cells)** | **GLV-1h68 Average Viral Titer (Log pfu/ 10⁶ cells)** | **Standard Deviation (Log pfu/ 10⁶ cells)** |
| 1 | 4.85 | 0.04 | 4.63 | 0.09 | 3.75 | 0.12 |
| 24 | 6.88 | 0.20 | 7.07 | 0.11 | 4.93 | 0.12 |
| 48 | 7.70 | 0.14 | 7.49 | 0.07 | 6.58 | 0.04 |
| 72 | 7.90 | 0.07 | 7.65 | 0.17 | 7.17 | 0.06 |

**Table 30I**

| **Viral titer values for EKVX** | | | | | | |
|---|---|---|---|---|---|---|
| **Hours Post Infection** | **LIVP Average Viral Titer (Log pfu/ 10⁶ cells)** | **Standard Deviation (Log pfu/10⁶ cells)** | **WR Average Viral Titer (Log pfu/ 10⁶ cells** | **Standard Deviation (Log pfu/ 10⁶ cells)** | **GLV-1h68 Average Viral Titer (Log pfu/ 10⁶ cells)** | **Standard Deviation (Log pfu/ 10⁶ cells)** |
| 1 | 4.15 | 0.16 | 4.13 | 0.01 | 3.23 | 0.08 |
| 24 | 7.39 | 0.02 | 7.38 | 0.20 | 6.13 | 0.39 |
| 48 | 7.58 | 0.03 | 7.57 | 0.16 | 7.06 | 0.08 |
| 72 | 7.53 | 0.05 | 7.52 | 0.04 | 7.15 | 0.18 |

**Table 30J**

| **Viral titer values for A-673** | | |
|---|---|---|
| **Hours Post Infection** | **GLV-1h68 Average Viral Titer (Log pfu/ 10⁶ cells)** | **Standard Deviation (Log pfu/ 10⁶ cells)** |
| 1 | 3.26 | 0.07 |
| 24 | 4.38 | 0.36 |
| 48 | 5.66 | 0.14 |
| 72 | 6.20 | 0.01 |

**Table 30K**

| **Viral titer values for D283/MED** | | |
|---|---|---|
| **Hours Post Infection** | **GLV-1h68 Average Viral Titer (Log pfu/ 10⁶ cells)** | **Standard Deviation (Log pfu/ 10⁶ cells)** |
| 1 | 2.48 | 0.24 |
| 24 | 3.66 | 0.16 |
| 48 | 4.27 | 0.29 |
| 72 | 4.61 | 0.04 |

**Table 30L**

| **Viral titer values for MNNG/HOS** | | |
|---|---|---|
| **Hours Post Infection** | **GLV-1h68 Average Viral Titer (Log pfu/ 10⁶ cells)** | **Standard Deviation (Log pfu/ 10⁶ cells)** |
| 1 | 3.49 | 0.07 |
| 24 | 6.48 | 0.20 |
| 48 | 7.58 | 0.03 |
| 72 | 7.46 | 0.08 |

The cell lines were divided into groups of predicted responders and non-responders based on the ability of the virus to exhibit significant replication within the cells within 24 hours post infection. Cell types that exhibited an approximate 3-fold or greater increase in viral titer over input titer were designated as *in vitro* responders. Table 31 displays the fold increase in viral titer between consecutive time points.

**Table 31**

| **Predicted Responders and Non-responders based on *in vitro* replication assays** | | | | |
|---|---|---|---|---|
| | **Cell Line** | **Fold Increase** | | |
| | | **24 h.p.i. / input** | **48 h.p.i. / 24 h.p.i.** | **72 h.p.i. / 48 h.p.i.** |
| **In vitro responders** | A-673 | 3.01 | 15.77 | 3.36 |
| | EKVX | 167.06 | 6.94 | 1.30 |
| | HOP-92 | 8.82 | 43.29 | 3.92 |
| | MALME-3M | 34.46 | 20.20 | 2.33 |
| | MNNG/HOS | 327.27 | 11.57 | 0.77 |
| | NCI-H23 | 176.39 | 5.83 | 1.36 |
| | NCI-H226 | 16.94 | 12.79 | 11.41 |
| | NCI-H460 | 47.04 | 5.08 | 2.36 |
| | NCI-H522 | 38.89 | 58.57 | 1.51 |
| | RXF-393 | 40.24 | 11.26 | 3.45 |
| **In vitro non-responders** | D283/MED | 0.48 | 4.44 | 1.93 |
| | NCI-H322M | 2.01 | 1.48 | 1.05 |

| | | | | |
|---|---|---|---|---|
| h.p.i. = hours post viral infection | | | | |

### Example 20

### Comparison of Expression Levels in Between Untreated Tumors

In order to determine whether there were similarities in basal gene expression levels among tumors that respond favorably to viral therapy versus tumors that respond poorly to viral therapy, the expression profiles in untreated tumors were compared. The data from the experiments described in Examples 9 and 10 were used for the comparison. Table 32 shows the ratios of proteins that are expressed HT-29 tumor cells, which are poor responders, versus A549 or PANC-1 tumor cells, which respond favorably to tumor therapy.

**Table 32**

| **Fold Difference in Human Protein Expression Levels in HT29 (Poor Responder) versus A549 or PANC-1 (Responder) Tumor Cells** | | | | |
|---|---|---|---|---|
| | **Fold difference in protein levels** | | | |
| **Human Protein** | **HT-29/ A549** | **HT-29/ PANC-1** | **A549/ HT-29** | **PANC-1/ HT-29** |
| Alpha-2 Macroglobulin | 0.71 | 1.10 | 1.41 | 0.91 |
| Alpha-Fetoprotein | 0.77 | 0.68 | 1.30 | 1.47 |
| Beta-2 Microglobulin | 25.88 | 583.57 | 0.04 | 0.00 |
| Brain-Derived Neurotrophic Factor | 5.18 | 38.75 | 0.19 | 0.03 |
| Cancer Antigen 125 | 0.04 | 0.70 | 22.24 | 1.43 |
| Cancer Antigen 19-9 | 1050.50 | 1536.20 | 0.00 | 0.00 |
| Calcitonin | 0.98 | 1.36 | 1.02 | 0.73 |
| CD40 | 0.05 | 0.00 | 18.34 | 301.60 |
| CD40 Ligand | 1.27 | 0.14 | 0.79 | 7.33 |
| Creatine Kinase-MB | 5.80 | 0.87 | 0.17 | 1.15 |
| C Reactive Protein | 5.32 | 2.67 | 0.19 | 0.37 |
| EGF | 32.79 | 9.92 | 0.03 | 0.10 |
| ENA-78 | 0.00 | 0.11 | 591.03 | 8.96 |
| Endothelin-1 | 3.03 | 1.88 | 0.33 | 0.53 |
| Eotaxin | 0.83 | 0.81 | 1.20 | 1.23 |
| Fatty Acid Binding Protein | 66.05 | 30.41 | 0.02 | 0.03 |
| Factor VII | 2.03 | 3.66 | 0.49 | 0.27 |
| Ferritin | 0.34 | 1.01 | 2.96 | 0.99 |
| FGF basic | 0.27 | 1.54 | 3.64 | 0.65 |
| G-CSF | 0.80 | 0.83 | 1.25 | 1.21 |
| Growth Hormone | 4.94 | 2.77 | 0.20 | 0.36 |
| GM-CSF | 8.74 | 16.60 | 0.11 | 0.06 |
| Glutathione S-Transferase | 1.69 | 0.83 | 0.59 | 1.20 |
| ICAM-1 | 33.36 | 1.64 | 0.03 | 0.61 |
| IL-10 | 1.38 | 1.68 | 0.73 | 0.60 |
| IL-12p40 | 3.44 | 0.49 | 0.29 | 2.03 |
| IL-12p70 | 2.18 | 0.73 | 0.46 | 1.38 |
| IL-13 | 1.99 | 1.27 | 0.50 | 0.79 |
| IL-15 | 1.39 | 0.48 | 0.72 | 2.09 |
| IL-16 | 0.93 | 0.04 | 1.07 | 25.10 |
| IL-18 | 0.23 | 1.96 | 4.26 | 0.51 |
| IL-1alpha | 6.00 | 53.38 | 0.17 | 0.02 |
| IL-1beta | 0.73 | 12.38 | 1.38 | 0.08 |
| IL-1ra | 2361.95 | 107.55 | 0.00 | 0.01 |
| IL-2 | 1.13 | 0.10 | 0.88 | 9.57 |
| IL-5 | 3.30 | 1.19 | 0.30 | 0.84 |
| IL-6 | 0.03 | 0.41 | 31.11 | 2.43 |
| IL-7 | 5.12 | 6.29 | 0.20 | 0.16 |
| IL-8 | 1.32 | 20.56 | 0.76 | 0.05 |
| MCP-1 | 1.20 | 0.00 | 0.84 | 395.51 |
| MIP-1alpha | 3.65 | 1.86 | 0.27 | 0.54 |
| MIP-1 beta | 1.68 | 0.22 | 0.60 | 4.64 |
| MMP-2 | 0.52 | 0.20 | 1.93 | 4.92 |
| MMP-9 | 3.98 | 0.97 | 0.25 | 1.03 |
| PAI-1 | 0.53 | 0.60 | 1.88 | 1.66 |
| Prostatic Acid Phosphatase | 26.84 | 155.26 | 0.04 | 0.01 |
| PAPP-A | 0.00 | 0.13 | 555.34 | 7.94 |
| Prostate Specific Antigen, Free | 302.09 | 38.31 | 0.00 | 0.03 |
| RANTES | 37.28 | 0.21 | 0.03 | 4.85 |
| Stem Cell Factor | 3.64 | 10.61 | 0.27 | 0.09 |
| SGOT | 0.22 | 0.24 | 4.49 | 4.18 |
| Tissue Factor | 0.15 | 8.53 | 6.47 | 0.12 |
| TNF RII | 11.57 | 0.72 | 0.09 | 1.40 |
| TNF-alpha | 39.73 | 10.00 | 0.03 | 0.10 |
| TNF-beta | 0.42 | 0.24 | 2.41 | 4.11 |
| Thrombopoietin | 1.04 | 0.49 | 0.96 | 2.03 |
| VCAM-1 | 1.09 | 0.16 | 0.92 | 6.31 |
| VEGF | 18.76 | 142.28 | 0.05 | 0.01 |

### Example 21

### Generation of Modified Vaccinia Virus Strains

### A. Construction of modified vaccinia viruses

Modified vaccinia viruses were generated by replacing nucleic acid or inserting nucleic acid at the thymidine kinase (*TK*) gene locus (also referred to as *JR2* locus. The heterologous DNA inserted at this locus were expression cassettes containing protein-encoding DNA, operably linked in the correct or reverse orientation to a vaccinia virus promoter.

The starting strain used in generating the modified vaccinia viruses was vaccinia virus (VV) strain GLV-1h68 (also named RVGL21, SEQ ID NO: 2). This genetically engineered strain, which is described in U.S. Patent Publication No. 2005/0031643, contains DNA insertions in the *F14.5L* (also referred to as F3; see U.S. Patent Publication No. 2005/0031643), thymidine kinase (*TK*) and hemagglutinin (*HA*) genes. GLV-1h68 was prepared from the vaccinia virus strain designated LIVP (a vaccinia virus strain, originally derived by adapting the Lister strain (ATCC Catalog No. VR-1549) to calf skin (Research Institute of Viral Preparations, Moscow, Russia, Al'tshtein et al. (1985) Dokl. Akad. Nauk USSR 285:696-699). The LIVP strain (genome sequence set forth in SEQ ID NO: 1), from which GLV-1h68 was generated, contains a mutation in the coding sequence of the *TK* gene (see SEQ ID NO: I for the sequence of the LIVP strain) in which a substitution of a guanine nucleotide with a thymidine nucleotide (nucleotide position 80207 of SEQ ID NO: 1) introduces a premature STOP codon within the coding sequence.

As described in U.S. Patent Publication No. 2005/0031643 (see particularly Example 1, therein), GLV-1h68 was generated by inserting expression cassettes encoding detectable marker proteins into the *F14.5L* (also designated in LIVP as *F3)* gene, thymidine kinase (*TK*) gene, and hemagglutinin (*HA*) gene loci of the vaccinia virus LIVP strain. Specifically, an expression cassette containing a *Ruc-GFP* cDNA (a fusion of DNA encoding *Renilla* luciferase and DNA encoding GFP) under the control of a vaccinia synthetic early/late promoter P_{SEL} was inserted into the *F14.5L* gene; an expression cassette containing DNA encoding beta-galactosidase under the control of the vaccinia early/late promoter P_{7.5k} (denoted (P_{7.5k})*LacZ*) and DNA encoding a rat transferrin receptor positioned in the reverse orientation for transcription relative to the vaccinia synthetic early/late promoter P_{SEL} (denoted (P_{SEL})*rTrfR*) was inserted into the *TK* gene (the resulting virus does not express transferrin receptor protein since the DNA encoding the protein is positioned in the reverse orientation for transcription relative to the promoter in the cassette); and an expression cassette containing DNA encoding β-glucuronidase under the control of the vaccinia late promoter P₁₁ₖ (denoted (P₁₁ₖ)*gusA*) was inserted into the *HA* gene.

Insertion of the expression cassettes into the LIVP genome in the generation of GLV-1h68 resulted in disruption of the coding sequences for each of the *F14.5L*, *TK* and *HA* genes; accordingly, all three genes in the GLV-1h68 strain are nonfunctional in that they do not encode the corresponding full-length proteins. As described in U:S. Patent Publication No. 2005/0031643, disruption of these genes not only attenuates the virus but also enhances its tumor-specific accumulation. Previous data have shown that systemic delivery of the GLV-1h68 virus in a mouse model of breast cancer resulted in the complete eradication of large subcutaneous GI-101A human breast carcinoma xenograft tumors in nude mice (see U.S. Patent Publication No. 2005/0031643).

### 1. Modified Viral strains

Modified recombinant vaccinia viruses containing heterologous DNA inserted into the *TK* locus of the vaccinia virus genome were generated via homologous recombination between DNA sequences in the genome and a transfer vector using methods described herein and known to those of skill in the art (see, *e.g.*, Falkner and Moss (1990) J. Virol. 64:3108-2111; Chakrabarti et al. (1985) Mol. Cell Biol. 5:3403-3409; and U.S. Patent No. 4,722,848). With these methods, the existing target gene in the starting vaccinia virus (GLV-1h68) genome was replaced by an interrupted copy of the gene contained in each transfer vector through two crossover events: a first crossover event of homologous recombination between the vaccinia virus genome and the transfer vector and a second crossover event of homologous recombination between direct repeats within the target locus. The interrupted version of the target gene that was in the transfer vector contained the insertion DNA flanked on each side by DNA corresponding to the left portion of the target gene and right portion of the target gene, respectively. Each of the transfer vectors also contained a dominant selection marker, the *E.coli* guanine phosphoribosyltransferase (*gpt*) gene, under the control of a vaccinia virus early promoter (P_{7.5kE}). Including such a marker in the vector enabled a transient dominant selection process to identify recombinant virus grown under selective pressure that has incorporated the transfer vector within its genome. Because the marker gene was not stably integrated into the genome, it was deleted from the genome in a second crossover event that occurred when selection was removed. Thus, the final recombinant virus contained the interrupted version of the target gene as a disruption of the target loci, but did not retain the selectable marker from the transfer vector.

Homologous recombination between a transfer vector and the starting vaccinia virus genome (GLV-1h68) occurred upon introduction of the transfer vector into cells that had been infected with the starting vaccinia virus. A series of transfer vectors was constructed as described below and used in construction of the following modified vaccinia strains: GLV-1h103, GLV-1h119, GLV-1h120 and GLV-1h121. The construction of these strains is summarized in the following Table, which lists the modified vaccinia virus strains, including the previously described GLV-1h68 (see section A, above), their respective genotypes, and the transfer vectors used to engineer the viruses:

**Table 33: Generation of engineered vaccinia viruses**

| **Name of Virus** | **Parental Virus** | **VV Transfer Vector** | **Genotype** |
|---|---|---|---|
| GLV-1h68 | - | - | *F14.5L*: (P_{SEL})*Ruc-GFP* |
| | | | *TK:* (P_{SEL})*rTrfR*-(P_{7.5k})*LacZ* |
| | | | *HA*: (P₁₁)*gusA* |
| GLV-1h103 | GLV-1h68 | TK-SL-hMCP1 (SEQ ID NO: 395) | *F14.5L*: *(*P_{SEL})*Ruc-GFP* |
| | | | *TK:* (P_{SL})*hmcp-1* |
| | | | *HA*: (P₁₁ₖ)*gusA* |
| GLV-1h119 | GLV-1h68 | TK-SE-mIP10-1 (SEQ ID NO: 399) | *F14.5L*: (P_{SEL})*Ruc-GFP* |
| | | | *TK:* (P_{SE})*mIP-10* |
| | | | *HA*: (P₁₁ₖ)*gusA* |
| GLV-1h120 | GLV-1h68 | TK-SEL-mIP10-1 (SEQ ID NO: 401) | *F14.5L*: *(P_{SEL})Ruc-GFP* |
| | | | *TK:* (P_{SEL})*mIP-10* |
| | | | *HA*: (P₁₁ₖ)*gusA* |
| GLV-1h121 | GLV-1h68 | TK-SL-mIP10-1 (SEQ ID NO: 400) | *F14.5L*: *(*P_{SEL})*Ruc-GFP* |
| | | | *TK:* (P_{SL})*mIP-10* |
| | | | *HA*: (P₁₁ₖ)*gusA* |

Briefly, these strains were generated as follows (further details are provided below):
**GLV-1h103** was generated by insertion of an expression cassette containing DNA encoding human monocyte chemoattractant protein-1 (hMCP1; a chemoattractant that specifically attracts monocytes and memory T cells), under the control of the vaccinia synthetic late promoter (P_{SL}), into the *TK* locus of strain GLV-1h68 (parental virus), thereby deleting the *LacZ*/*rTFr* expression cassette at the *TK* locus of GLV-1h68. Strain GLV-1h103 retains the *Ruc-GFP* expression cassette at the *F14.5L* locus and the *gusA* expression cassette at the *HA* locus.
**GLV-1h119** was generated by insertion of an expression cassette containing DNA encoding mouse interferon-γ inducible protein 10 (mIP-10; a potent chemoattractant for activated T cells and NK cells), under the control of the vaccinia synthetic early promoter (P_{SE}), into the *TK* locus of strain GLV-1h68 (parental virus), thereby deleting the *LacZ*/*rTFr* expression cassette at the *TK* locus of GLV-1h68. Strain GLV-1h119 retains the *Ruc-GFP* expression cassette at the *F14.5L* locus and the *gusA* expression cassette at the *HA* locus.
**GLV-1h120** was generated by insertion of an expression cassette containing DNA encoding mouse interferon-γ inducible protein 10 (mIP-10; a potent chemoattractant for activated T cells and NK cells), under the control of the vaccinia synthetic early/late promoter (P_{SEL}), into the *TK* locus of strain GLV-1h68 (parental virus), thereby deleting the *LacZ*/*rTFr* expression cassette at the *TK* locus of GLV-1h68. Strain GLV-1h120 retains the *Ruc-GFP* expression cassette at the *F14.5L* locus and the *gusA* expression cassette at the *HA* locus.
**GLV-1h121** was generated by insertion of an expression cassette containing DNA encoding mouse interferon-γ inducible protein 10 (mIP-10; a potent chemoattractant for activated T cells and NK cells), under the control of the vaccinia synthetic late promoter (P_{SL}), into the *TK* locus of strain GLV-1h68 (parental virus), thereby deleting the *LacZ*/*rTFr* expression cassette at the *TK* locus of GLV-1h68. Strain GLV-1h121 retains the *Ruc-GFP* expression cassette at the *F14.5L* locus and the *gusA* expression cassette at the *HA* locus.

### 2. VV transfer vectors employed for the production of the modified vaccinia viruses

The following vectors were constructed and employed as described below to generate the recombinant vaccinia viral strains listed in Table 33, above.

### a. Construction of the TK-SL-hMCP1 transfer vector for insertion of human MPC-1 encoding DNA into the vaccinia virus TK locus

The TK-SL-hMCPI transfer vector (SEQ ID NO: 395) was used to produce the modified vaccinia virus strain GLV-1h103 (see Table 33, above), which had the genotype *F14.5L*: (PSEL)*Ruc*-GFP; *TK*: (P_{SL})*hmcp-1*; *HA*: (P₁₁ₖ)*gusA*. Strain GLV-1h103 was generated by inserting DNA (SEQ ID NO: 396) encoding a human monocyte chemoattractant protein-1 (hMCP-1) protein (SEQ ID NO: 135) into the *TK* locus of strain GLV-1h68, thereby deleting the *rTrfR-LacZ* expression cassette at the *TK* locus of strain GLV-1h68. The transfer vector TK-SL-hMCP1, which was used in this process, contained a DNA fragment encoding the hMCP-1 protein operably linked to the vaccinia synthetic late promoter (P_{SL}), sequences of the *TK* gene flanking the (P_{SL}) / protein-encoding DNA fragment. The vector was generated as follows:
hMCP-1 cDNA was amplified by PCR (Accu Prime Pfx Supermix) from a cDNA template (clone encoding Homo sapiens chemokine (C-C motif) ligand 2 (cDNA clone TC118317; Origene; SEQ ID NO: 391), using the following primers:
   HMCP1-5 (Sal I)
      5'-GTCGACGCCACCATGAAAGTCTCTGCCGCCCT-3' (SEQ ID NO: 392); and
   hMCPI-3 (Pac I)
      5'-TTAATTAATCAAGTCTTCGGAGTTTGGGTTTGC-3' (SEQ ID NO: 393).

These primers contained recognition sites for Sal I and Pac I restriction enzymes, respectively. The product from this PCR amplification was run on an agarose gel and purified and cloned into the pCR®- Blunt II-TOPO® vector (SEQ ID NO: 394) using a Zero Blunt® TOPO® PCR Cloning Kit (Invitrogen™, Carlsbad, CA). The nucleic acid sequence of the resulting construct, pCRII-hMCP1-2 was confirmed by sequencing. This pCRII-hMCP1-2 construct was digested with Sal I and Pac I to release the hMCP-1 cDNA, which then was subcloned into a TK-SL-CSF4 vector (which contains the cDNA for GM-CSF under the control of the vaccinia synthetic late promoter flanked by the *TK* gene regions) that also had been digested with Sal I and Pac I. Subcloning replaced the GM-CSF cDNA in the TK-SL-CSF4 vector with hMCP-1, thereby placing hMCP-1 under the control of vaccinia synthetic late promoter (P_{SL}). The nucleotide sequence of the resulting construct, TK-SL-hMCP1 (SEQ ID NO: 395), was confirmed by sequencing.

### b. Construction of the TK-SE-mIP10-1, TK-SL-mIP10-1 and TK-SEL-mIP10-1 transfer vectors for insertion of mouse IP-10 encoding DNA into the vaccinia virus TK locus

The TK-SE-mIP10-1 (SEQ ID NO: 399), TK-SL-mIP10-1 (SEQ ID NO: 400) and TK-SEL-mIP10-1 (SEQ ID NO: 401) transfer vectors were used to produce the modified vaccinia virus strains GLV-1h103GLV-1h119, GLV-1h121 and GLV-1h120, respectively (See Table 33, above). As listed in Table 33, these viruses had the following genotypes: *F14.5L:* (P_{SEL})*Ruc-GFP, TK:* (P_{SE})*mIP-10, HA*: (P₁₁ₖ)*gusA* (GLV-1h119); *F14.5L:* (P_{SEL})*Ruc-GFP, TK:* (P._{SL})*mIP-10 HA:* (P₁₁ₖ)*gusA* (GLV-1h121); and *F14.5L:* (P_{SEL})*Ruc-GFP, TK:* (P_{SEL})*mIP-10, HA:* (P₁₁ₖ)*gusA* (GLV-1h120). Each of the virus strains was generated by inserting DNA (SEQ ID NO: 398) encoding a mouse interferon-γ inducible protein 10 (mIP-10) (SEQ ID NO: 24) into the *TK* locus of strain GLV-1h68, thereby deleting the *rTrfR-LacZ* expression cassette at the *TK* locus of strain GLV-1h68.

The three transfer vectors used in this process, TK-SE-mIP10-1, TK-SL-mIP10-1 and TK-SEL-mIP10-1, container DNA fragments encoding the mIP-10 protein, operably linked to the vaccinia synthetic early promoter (P_{SE}), synthetic late promoter (P_{SL}), and synthetic early/late promoter (P_{SEL}), respectively. These transfer vectors further contained sequences of the *TK* gene flanking the promoter / protein-encoding DNA fragment. These transfer vectors were generated as follows:
Mouse IP-10 cDNA was amplified by PCR using a cDNA template (mouse GenePools cDNA from Biomedomics, Inc. (Research Triangle Park, NC; Catalog number BM2080-1)), and the following primers:
   mIP10-5 (Sal I)
      5'-GTCGACGCCACCATGAACCCAAGTGCTGCCGT-3' (SEQ ID NO: 402)
   mIP10-3 (Pac I)
      5'-TTAATTAATTAAGGAGCCCTTTTAGACCTTTTTTGG-3'(SEQ ID NO: 403).

As indicated, these primers contained the Sal I and Pac I restriction enzyme recognition sites, respectively. The product from this PCR amplification was run on an agarose gel, purified and cloned into the pCR®- Blunt II-TOPO® vector (SEQ ID NO: 394) using a Zero Blunt® TOPO® PCR Cloning Kit (Invitrogen™, Carlsbad, CA). The nucleotide sequence of the resulting construct, pCRII-mIP10, was confirmed by sequencing.

This pCRII-mIP10 construct was digested with Sal I and Pac I to release the mIP-10 cDNA, which then was subcloned into the TK-SE-CSF-2 (which contains the cDNA for GM-CSF under the control of the vaccinia synthetic early promoter flanked by the *TK* gene regions. SEQ ID NO: 404), TK-SL-CSF4 (which contains the cDNA for GM-CSF under the control of the vaccinia synthetic late promoter flanked by the *TK* gene regions) and TK-SEL-CSF-2 (which contains the cDNA for GM-CSF under the control of the vaccinia synthetic early/late promoter flanked by the *TK* gene regions; SEQ ID NO: 405) vectors (which had been digested with Sal I and Pac I), to generate the TK-SE-mIP10-1, TK-SL-mIP10-1, and TK-SEL-mIP10-1 transfer vectors, respectively. Subcloning into TK-SE-CSF-2, TK-SL-CSF4 and TK-SEL-CSF-2 replaced GM-CSF-encoding cDNA in these vectors with cDNA encoding mIP-10, thereby placing mIP-10 expression under the control of vaccinia synthetic early SE, late SL, and early/late promoter SEL, respectively. The nucleotide sequences of the resulting transfer vectors, TK-SE-mIP10-1, TK-SL-mIP10-1, and TK-SEL-mIP10-1, were confirmed by sequencing.

### 3. Preparation of recombinant vaccinia viruses

For preparation of the GVL-1h103, GVL-1h119, GVL-1h120 and GVL-1h121 modified viruses, CV-1 cells, grown in DMEM (Mediatech, Inc., Hemdon, VA) with 10% FBS, were infected with the indicated parental viruses (Table 33) at an m.o.i. of 0.1 for 1 hr, then transfected using Lipofectamine 2000 or Fugene (Roche, Indianapolis, IN) with 2 µg of the corresponding transfer vector (Table 33). Infected/transfected cells were harvested and the recombinant viruses were selected using a transient dominant selection system and plaque purified using methods known in the art (see, *e.g*., Falkner and Moss, J. Virol., 64, 3.108-3111 (1990)). Isolates were plaque purified five times with the first two rounds of plaque isolation conducted in the presence of mycophenolic acid, xanthine and hypoxanthine which permits growth only of recombinant virus that expressing the selectable marker protein, *i.e., E. coli* guanine phosphoribosyltransferase (*gpt*), under the control of the vaccinia P_{7.5kE} promoter. Each of the transfer vectors used in the generation of the GVL-1h103, GVL-1h119, GVL-1h120 and GVL-1h121 recombinant Vaccinia viruses contained a (P_{7.5kE})*gpt* expression cassette. Thus, growth of the virus in the presence of the selection agents enabled identification of virus in which the first crossover event of homologous recombination between the transfer vector and the parental strain genome had occurred. Subsequent growth of the isolates in the absence of selection agents and further plaque purification yielded isolates that had undergone a second crossover event resulting in deletion of the DNA encoding guanine phosphoribosyltransferase from the genome. This was confirmed by the inability of these isolates to grow in the presence of selection agents.

### 4. Verification of vaccinia virus strain genotypes

The genotypes of the GVL-1h103, GVL-1h119, GVL-1h120 and GVL-1h121 modified vaccinia virus strains were verified by PCR and restriction enzyme digestion and lack of expression of β-galactosidase in these viruses was confirmed by X-gal (5-bromo-4-chloro-3-indolyl-β-D-galactopyranoside) staining of the infected cells to confirm lack of ability to convert the X-gal substrate, as indicated by lack of development of blue color in the assays compared to a control strain (*e.g.* GLV-1h68). Viruses lacking *lacZ* expression are unable to convert the X-gal substrate. Standard techniques for X-GlcA and X-gal viral staining and fluorescence microscopy were employed and are well-known in the art.

### B. Vaccinia virus purification

Ten T225 flasks of confluent CV-1 cells (seeded at 2 x 10⁷ cells per flask the day before infection) were infected with each virus at m.o.i. of 0.1. The infected cells were harvested two days post infection and lysed using a glass Dounce homogenizer. The cell lysate was clarified by centrifugation at 1,800g for 5 min, and then layered on a cushion of 36% sucrose, and centrifuged at 13,000 rpm in a HB-6 rotor, Sorvall RC-5B Refrigerated Superspeed Centrifuge for 2 hours. The virus pellet was resuspended in 1 ml of 1 mM Tris, pH 9.0, loaded on a sterile 24% to 40% continuous sucrose gradient, and centrifuged at 26,000g for 50 min. The virus band was collected and diluted using 2 volumes of 1 mM Tris, pH 9.0, and then centrifuged at 13,000 rpm in a HB-6 rotor for 60 min. The final virus pellet was resuspended in 1 ml of 1 mM Tris, pH 9.0 and the titer was determined in CV-1 cells (ATCC No. CCL-70).

Since modifications will be apparent to those of skill in this art, it is intended that this invention be limited only by the scope of the appended claims.

## Claims

1. A method for predicting efficacy of viral therapy for a tumor in a subject, comprising:
introducing a therapeutic virus into a tumor cell obtained from a biopsy or body fluid of a subject or into a cell culture that comprises the tumor cells;
determining a replication indicator indicative of the level or amount of viral replication within a predetermined period of time or as a function of time after introduction of the therapeutic virus into tumor cells to thereby determine whether replication is delayed compared to a control or standard; and if replication is not delayed, selecting the virus as a candidate therapeutic virus for treatment of the tumor in the subject.

2. The method of claim 1, wherein delayed replication is assessed by:
infecting a cell culture with a therapeutic virus, wherein the cell culture comprises cells from a tumor;
after a predetermined time, determining a replication indicator of replication of the virus in the culture; and
based on the value of the replication indicator, predicting a therapeutic efficacy of the virus against the tumor.

3. The method of claim 1 or claim 2, wherein the control is a tumor cell or cell from a cell culture that comprises such tumor cells that respond to treatment with the virus.

4. The method of any of claims 1-3, wherein the virus is an oncolytic virus.

5. The method of any of claims 1-4, wherein the virus is a vaccinia virus.

6. The method of any of claims 1-5, wherein the virus is LIVP.

7. The methods of any of claims 1-6, wherein the virus is GLV-1h68.

8. The method of any of claims 1-7, wherein the replication indicator is compared to a standard indicative of delayed replication or non-delayed replication.

9. The method of claim 8, wherein the standard is pre-determined.

10. The method of claim 9, wherein the replication indicator is compared to a database of predetermined values for cell types to determine whether the replication indicator has a value indicative of non-delayed replication.

11. The method of any of claims 1-10, wherein the tumor cell is a biopsied cell or cell from a bodily fluid.

12. The method of any of claims 1-11, wherein the period of time for assessing replication delay comprises a range of zero to 10 days, zero to 5 days, zero to 2 days, zero to 1 day or less than 24 hours or 24 hours.

13. The method of any of claims 1-12, wherein the replication indicator by which a delay in replication is assessed is selected from among one or more of:
(i) an increase in expression of a viral gene or a heterologous gene encoded by the virus, wherein an increase in expression is indicative that the tumor cells are responsive to virus therapy;
(ii) a decrease in expression of a housekeeping gene expressed in the tumor upon viral expression, wherein a decrease in expression is indicative that the tumor cells are responsive to virus therapy;
(ii) an increase in expression of a gene in the tumor encoding immune effector proteins, wherein an increase in expression is indicative that the tumor cells are responsive to virus therapy or
(iv) a change in expression of a gene expressed by the tumor cells, wherein a change in expression is indicative that the tumor cells are responsive to virus therapy

14. The method of claim 13, wherein an increase in gene expression of one or more genes encoding a protein selected from among IL-18 (Interleukin-18), MCP-5 (Monocyte Chemoattractant Protein-5; CCL12), IL-11 (Interleukin-11), MCP-1 (Monocyte Chemoattractant Protein-1), MPO (Myeloperoxidase), Apo A1 (Apolipoprotein A1), TIMP-1 (Tissue Inhibitor of Metalloproteinase Type-1), CRP (C Reactive Protein), Fibrinogen, MMP-9 (Matrix Metalloproteinase-9), Eotaxin (CCL11), GCP-2 (Granulocyte Chemotactic Protein-2; CXCL6), IL-6 (Interleukin-6), Tissue Factor (TF), SAP (Serum Amyloid P), FGF-basic (Fibroblast Growth Factor-basic), MCP-3 (Monocyte Chemoattractant Protein-3; CCL7), IP-10 (CXCL 10), MIP-2, Thrombopoetin, Cancer antigen 125, CD40, CD40 ligand, ENA-78, Ferritin, IL-12p40, IL-12p70, IL-16, MMP-2, PAI-1, TNF RII, TNF-beta and VCAM-1 indicates that the tumor cells are responsive to virus therapy.

15. The method of claim 13, wherein an increase in gene expression of one or more genes encoding a protein selected from among MIP-1 beta (Macrophage Inflammatory Protein-1 beta), MDC (Macrophage-Derived Chemokine; CCL22), MIP-1 alpha (Macrophage Inflammatory Protein-1alpha; CCL3), KC/GROalpha (Melanoma Growth Stimulatory Activity Protein), VEGF (Vascular Endothelial Cell Growth Factor), Endothelin-1, MIP-3 beta (Macrophage Inflammatory Protein-3 beta; Exodus-3 or ELC), Beta-2 microglobulin, IL-5 (Interleukin-5), IL-1 alpha (Interleukin-1 alpha), EGF (Epidermal Growth Factor), Lymphotactin (XCL1), GM-CSF (Granulocyte Macrophage-Colony Stimulating Factor), MIP-1 gamma (Macrophage Inflammatory Protein-1 gamma; CCL4), IL-1 beta (Interleukin-1 beta), Brain-derived neutrophic factor, Cancer antigen 19-9, Carcinoembryonic antigen, C reactive protein, EGF, Fatty acid binding protein, Factor VII, Growth hormone, IL-1 alpha, IL-1 beta, IL-1 ra, IL-7, IL-8, MDC, Prostatic acid phosphatase, Prostate specific antigen, free, Stem cell factor, Tissue factor, TNF-alpha, VEGF and Von Willebrand factor, indicates that the tumor cells are not responsive to virus therapy.

16. The method of any of claims 13-15, wherein the expression of two or more genes is assessed.

17. The method of claim 16, wherein gene expression is assessed on an array.

18. The method of any of claims 1-17, further comprising,
modifying the virus to include a gene that encodes a protein whose expression is increased in responders compared to non-responders or encodes a gene product that reduces expression of a protein whose level of expression is increased in non-responders compared to responders, wherein a responder is a tumor that is susceptible to treatment with the virus and a non-responder is a tumor that is resistant to treatment with the virus.

## Patentansprüche

1. Verfahren zum Prognostizieren der Wirksamkeit einer viralen Therapie gegen einen Tumor bei einer Person, das umfasst:
Einbringen eines therapeutischen Virus in eine Tumorzelle, die aus einer Biopsie oder einer Körperflüssigkeit einer Person stammt, oder in eine Zellkultur, die die Tumorzellen enthält;
Bestimmen eines Replikationsindikators, der das Niveau oder die Menge der viralen Replikation innerhalb einer vorgegebenen Zeitspanne oder als Funktion der Zeit nach Einbringen des therapeutischen Virus in Tumorzellen anzeigt, um dadurch zu ermitteln, ob die Replikation im Vergleich mit einer Kontrolle oder einem Standard verzögert ist; und Auswählen des Virus als therapeutischen Viruskandidaten zur Behandlung des Tumors bei der Person, wenn die Replikation nicht verzögert ist.

2. Verfahren nach Anspruch 1, worin die verzögerte Replikation bewertet wird durch:
Infizieren einer Zellkultur mit einem therapeutischen Virus, wobei die Zellkultur Zellen eines Tumors enthält;
nach einer vorgegebenen Zeitspanne Ermitteln eines Replikationsindikators der Replikation des Virus in der Kultur; und
auf der Basis des Wertes des Replikationsindikators Prognostizieren der therapeutischen Wirksamkeit des Virus gegenüber dem Tumor.

3. Verfahren nach Anspruch 1 oder Anspruch 2, worin es sich bei der Kontrolle um eine Tumorzelle oder eine Zelle aus einer Zellkultur handelt, die Tumorzellen enthält, die auf eine Behandlung mit dem Virus ansprechen.

4. Verfahren nach einem der Ansprüche 1 bis 3, worin das Virus ein onkolytisches Virus ist.

5. Verfahren nach einem der Ansprüche 1 bis 4, worin das Virus ein Vaccinia-Virus ist.

6. Verfahren nach einem der Ansprüche 1 bis 5, worin das Virus LIVP ist.

7. Verfahren nach einem der Ansprüche 1 bis 6, worin das Virus GLV-1h68 ist.

8. Verfahren nach einem der Ansprüche 1 bis 7, worin der Replikationsindikator mit einem Standard verglichen wird, der eine verzögerte Replikation oder eine nicht verzögerte Replikation anzeigt.

9. Verfahren nach Anspruch 8, worin der Standard vorgegeben ist.

10. Verfahren nach Anspruch 9, worin der Replikationsindikator mit einer Datenbank von zuvor bestimmten Werten für Zelltypen verglichen wird, um zu ermitteln, ob der Replikationsindikator einen Wert aufweist, der eine nicht verzögerte Replikation anzeigt.

11. Verfahren nach einem der Ansprüche 1 bis 10, worin die Tumorzelle eine biopsierte Zelle oder eine Zelle aus einer Körperflüssigkeit ist.

12. Verfahren nach einem der Ansprüche 1 bis 11, worin die Zeitspanne zum Bewerten der Replikationsverzögerung einen Bereich von Null bis 10 Tagen, Null bis 5 Tagen, Null bis 2 Tagen, Null bis 1 Tag oder weniger als 24 Stunden oder 24 Stunden umfasst.

13. Verfahren nach einem der Ansprüche 1 bis 12, worin der Replikationsindikator, durch den eine Verzögerung in der Replikation bewertet wird, unter einem oder mehreren der folgenden Indikatoren ausgewählt ist:
(i) Zunahme der Expression eines viralen Gens oder eines heterologen Gens, das von dem Virus codiert wird, wobei die Zunahme der Expression anzeigt, dass die Tumorzellen auf die Virustherapie ansprechen;
(ii) Abnahme der Expression eines Housekeeping-Gens, das in dem Tumor bei viraler Expression exprimiert wird, wobei die Abnahme der Expression anzeigt, dass die Tumorzellen auf die Virustherapie ansprechen;
(iii) Zunahme der Expression eines Immuneffektorproteine codierenden Gens in dem Tumor, wobei die Zunahme der Expression anzeigt, dass die Tumorzellen auf die Virustherapie ansprechen; oder
(iv) Änderung der Expression eines von den Tumorzellen exprimierten Gens, wobei die Änderung der Expression anzeigt, dass die Tumorzellen auf die Virustherapie ansprechen.

14. Verfahren nach Anspruch 13, worin ein Anstieg der Genexpression eines oder mehrerer Gene, die ein Protein codieren, das unter IL-18 (Interleukin-18), MCP-5 (Monocyten-chemoattraktives Protein-5; CCL12), IL-11 (Interleukin-11), MCP-1 (Monocytenchemoattraktives Protein-1), MPO (Myeloperoxidase), ApoA1 (Apolipoprotein A1), TIMP-1 (Gewebsinhibitor der Metalloproteinasen Typ 1), CRP (C-reaktives Protein), Fibrinogen, MMP-9 (Matrix-Metalloproteinase-9), Eotaxin (CCL11), GCP-2 (Granulocyten-chemotaktisches Protein 2; CXCL6), IL-6 (Interleukin-6), Gewebe-Faktor (TF), SAP (Serum Amyloid P), basic-FGF (Fibroblasten Wachstumsfaktor-basic), MCP-3 (Monocyten-chemoattraktives Protein-3; CCL7), IP-10 (CXCL 10), MIP-2, Thrombopoetin, Cancer-Antigen 125, CD40, CD40-Ligand, ENA-78, Ferritin, IL-12p40, IL-12p70, IL-16, MMP-2, PAI-1, TNF-RII, TNF-beta und VCAM-1 ausgewählt ist, anzeigt, dass die Tumorzellen auf die Virustherapie ansprechen.

15. Verfahren nach Anspruch 13, worin ein Anstieg der Genexpression eines oder mehrerer Gene, die ein Protein codieren, das unter MIP-1beta (Makrophagen inflammatorisches Protein-1 beta), MDC (Makrophagen-abgeleitetes Chemokin; CCL22), MIP-lalpha (Makrophagen inflammatorisches Protein-1 alpha; CCL3), KC/GROalpha (Melanoma Growth Stimulatory Activity Protein), VEGF (vaskulär-endothelialer Wachstumsfaktor), Endothelin-1, MIP-3beta (Makrophagen inflammatorisches Protein-3 beta; Exodus-3 oder ELC), Beta2-Mikroglobulin, IL-5 (Interleukin-5), IL-1alpha (Interleukin-1 alpha), EGF (epidermaler Wachstumsfaktor), Lymphotactin (XCL1), GM-CSF (Granulocyten/Makrophagen-Kolonie-stimulierender Faktor), MIP-1gamma (Makrophagen inflammatorisches Protein-1 gamma; CCL4), IL-1beta (Interleukin-1 beta), vom Gehirn stammendem neurotrophen Faktor (Brain-derived Neutrophic Factor), Cancer-Antigen 19-9, carcinoembryonalem Antigen, C-reaktivem Protein, EGF, Fettsäurebindendem Protein, Faktor VII, Wachstumshormon, IL-1alpha, IL-1beta, IL-1RA, IL-7, IL-8, MDC, prostataspezifischer saurer Phosphatase, prostataspezifischem Antigen, frei, Stammzellfaktor, Gewebefaktor, TNF-alpha, VEGF und Von Willebrand-Faktor ausgewählt ist, anzeigt, dass die Tumorzellen nicht auf die Virustherapie ansprechen.

16. Verfahren nach einem der Ansprüche 13 bis 15, worin die Expression von zwei oder mehr Genen untersucht wird.

17. Verfahren nach Anspruch 16, worin die Genexpression an einem Array bewertet wird.

18. Verfahren nach einem der Ansprüche 1 bis 17, das ferner umfasst:
Modifizieren des Virus, sodass es ein Gen umfasst, das ein Protein codiert, dessen Expression in Respondern im Vergleich mit Nicht-Respondern erhöht ist, oder das ein Genprodukt codiert, das die Expression eines Proteins vermindert, dessen Expressionsniveau in Nicht-Respondern im Vergleich zu Respondern erhöht ist, wobei ein Responder ein Tumor ist, der mit dem Virus behandelt werden kann, und ein Nicht-Responder ein Tumor ist, der gegenüber der Behandlung mit dem Virus resistent ist.

## Revendications

1. Procédé pour prévoir l'efficacité d'une thérapie virale pour une tumeur chez un sujet, comprenant :
l'introduction d'un virus thérapeutique dans une cellule tumorale obtenue à partir d'une biopsie ou d'un fluide corporel d'un sujet ou dans une culture cellulaire qui comprend les cellules tumorales ;
la détermination d'un indicateur de réplication indiquant le degré ou la quantité de réplication virale en une période de temps prédéterminée ou en fonction du temps après introduction du virus thérapeutique dans des cellules tumorales pour déterminer ainsi si la réplication est retardée comparativement à un témoin ou un échantillon de référence ; et, si la réplication n'est pas retardée, la sélection du virus en tant que virus thérapeutique candidat pour le traitement de la tumeur chez le sujet.

2. Procédé suivant la revendication 1, dans lequel la réplication retardée est évaluée par :
infection d'une culture cellulaire avec un virus thérapeutique, ladite culture cellulaire comprenant des cellules provenant d'une tumeur ;
après un temps prédéterminé, détermination d'un indicateur de réplication indiquant la réplication du virus dans la culture, et
sur la base de la valeur de l'indicateur de réplication, la prévision d'une efficacité thérapeutique du virus contre la tumeur.

3. Procédé suivant la revendication 1 ou la revendication 2, dans lequel le témoin est une cellule tumorale ou une cellule provenant d'une culture cellulaire qui comprend de telles cellules tumorales qui répondent au traitement avec le virus.

4. Procédé suivant l'une quelconque des revendications 1 à 3, dans lequel le virus est un virus oncolytique.

5. Procédé suivant l'une quelconque des revendications 1 à 4, dans lequel le virus est un virus vaccinal.

6. Procédé suivant l'une quelconque des revendications 1 à 5, dans lequel le virus est le LIVP.

7. Procédé suivant l'une quelconque des revendications 1 à 6, dans lequel le virus est le GLV-1h68.

8. Procédé suivant l'une quelconque des revendications 1 à 7, dans lequel l'indicateur de réplication est comparé à une valeur de référence indicative d'une réplication retardée ou d'une réplication non retardée.

9. Procédé suivant la revendication 8, dans lequel la valeur de référence est prédéterminée.

10. Procédé suivant la revendication 9, dans lequel l'indicateur de réplication est comparé à une base de données de valeurs prédéterminées pour des types cellulaires pour déterminer si l'indicateur de réplication a une valeur indiquant une réplication non retardée.

11. Procédé suivant l'une quelconque des revendications 1 à 10, dans lequel la cellule tumorale est une cellule prélevée par biopsie ou une cellule provenant d'un fluide corporel.

12. Procédé suivant l'une quelconque des revendications 1 à 11, dans lequel la période de temps pour l'évaluation du retard de réplication comprend un intervalle de zéro à 10 jours, de zéro à 5 cinq jours, de zéro à 2 jours, zéro à 1 jour, ou moins de 24 heures ou 24 heures.

13. Procédé suivant l'une quelconque des revendications 1 à 12, dans lequel l'indicateur de réplication permettant d'évaluer un retard de réplication est choisi parmi une ou plusieurs :
(i) d'une augmentation de l'expression d'un gène viral ou d'un gène hétérologue codé par le virus, une augmentation de l'expression indiquant que les cellules tumorales sont aptes à répondre à la thérapie virale ;
(ii) d'une diminution de l'expression d'un gène d'entretien exprimé dans la tumeur par expression virale, une diminution de l'expression indiquant que les cellules tumorales sont aptes à répondre à la thérapie virale ;
(iii) d'une augmentation de l'expression d'un gène dans la tumeur codant pour des protéines effectrices immunitaires, une augmentation de l'expression indiquant que les cellules tumorales sont aptes à répondre à la thérapie virale ; ou
(iv) d'une modification de l'expression d'un gène exprimé par les cellules tumorales, une modification de l'expression indiquant que les cellules tumorales sont aptes à répondre à la thérapie virale.

14. Procédé suivant la revendication 13, dans lequel une augmentation de l'expression d'un ou plusieurs gènes codant pour une protéine choisie entre la IL-18 (interleukine-18), la MCP-5 (protéine chimio-attractive des monocytes 5 ; CCL12), la IL-11 (interleukine-11), la MCP-1 (protéine chimio-attractive des monocytes 1), la MPO (myéloperoxidase), la Apa A1 (apolipoprotéine A1, le TIMP-1 (inhibiteur tissulaire de métalloprotéinase de type 1), la CRP (protéine C-réactive), le fibrinogène, la MMP-9 (métalloprotéinase de matrice 9), l'éotaxine (CCL11), la GCP-2 (protéine chimiotactique des granulocytes 2 ; CXCL6), la I1-6 (interleukine-6), le facteur tissulaire (TF), la SAP (protéine amyloïde sérique P), le FGF basique (facteur de croissance des fibroblastes basique), la MCP-3 (protéine chimio-attractive des monocytes 3 ; CCL7), la IP-10 (CXCL 10), la MIP-2, la thrombopoïétine, l'antigène du cancer 125, la CD40, un ligand de CD40, la ENA-78, la ferritine, la I1-12p40, la IL-12p70, la IL-16, la MMP-2, la PAI-1, le TNF RII, le TNF-bêta et la VCAM-1 indique que les cellules tumorales sont aptes à répondre à la thérapie virale.

15. Procédé suivant la revendication 13, dans lequel une augmentation de l'expression d'un ou plusieurs gènes codant pour une protéine choisie entre la MIP-1 bêta (protéine inflammatoire des macrophages 1 bêta), la MDC (chimiokine dérivée des macrophages ; CCL22), la MIP-1 alpha (protéine inflammatoire des macrophages 1 alpha ; CCL3), la KC/GROalpha (protéine d'activité stimulatrice de croissance des mélanomes), le VEGF (facteur de croissance des cellules endothéliales vasculaires), l'endothéline-1, la MIP-3 bêta (protéine inflammatoire des macrophages 3 bêta ; Exodus-3 ou ELC), la microglobuline bêta-2, la IL-5 (interleukine-5), la IL-1 alpha (interleukine-1 alpha), le EGF (facteur de croissance épidermique), la lymphotactine (XCL1), le GM-CSF (facteur de stimulation des colonies de granulocytes-macrophages), la MIP-1 gamma (protéine inflammatoire des macrophages 1 gamma ; CCL4), la IL-1 bêta (interleukine-1 bêta), le facteur neurotrophe dérivé du cerveau, l'antigène du cancer 19-9, l'antigène carcinoembryonnaire, la protéine C-réactive, le EGF, la protéine de liaison aux acides gras, le facteur VII, une hormone de croissance, la IL-1 alpha, la IL-1 bêta, la IL-1 ra, la IL-7, la IL-8, la MDC, la phosphatase acide prostatique, l'antigène spécifique de la prostate, le facteur libre des cellules souches, le facteur tissulaire, le TNF-alpha, le VEGF et le facteur de Von Willebrand indique que les cellules tumorales ne sont pas aptes à répondre à la thérapie virale.

16. Procédé suivant l'une quelconque des revendications 13 à 15, dans lequel l'expression de deux ou plus de deux gènes est évaluée.

17. Procédé suivant la revendication 16, dans lequel l'expression de gènes est évaluée sur un réseau.

18. Procédé suivant l'une quelconque des revendications 1 à 17, comprenant en outre
la modification du virus de manière à comprendre un gène qui code pour une protéine dont l'expression est augmentée chez les répondeurs comparativement aux non-répondeurs ou qui code pour un produit de gène qui réduit l'expression d'une protéine dont le degré d'expression est augmenté chez les non-répondeurs comparativement aux répondeurs, un répondeur étant une tumeur qui est sensible au traitement avec le virus et un non-répondeur étant une tumeur qui est résistante au traitement avec le virus.
